(19) Europäisches Patentamt
European Patent Office
Office européen des brevets

(11) **EP 4 377 641 B1**

(12) **EUROPÄISCHE PATENTSCHRIFT**

(45) Veröffentlichungstag und Bekanntmachung des Hinweises auf die Patenterteilung:
**26.11.2025  Patentblatt 2025/48**

(21) Anmeldenummer: **22754419.4**

(22) Anmeldetag: **26.07.2022**

(51) Internationale Patentklassifikation (IPC):
**G01F 1/76** (2006.01)  **A61L 2/00** (2006.01)
**A61L 2/26** (2006.01)  **G01N 1/22** (2006.01)
**G01N 15/10** (2024.01)  **A61L 9/16** (2006.01)
**G01N 3/20** (2006.01)  **G01N 15/0205** (2024.01)
**G01N 15/06** (2024.01)  **G01N 15/14** (2024.01)
**G01N 15/00** (2024.01)

(52) Gemeinsame Patentklassifikation (CPC):
**A61L 2/0023; A61L 2/26; A61L 9/16; G01N 3/20; G01N 15/0205; G01N 15/06; G01N 15/1425; G01N 15/1459;** A61L 2202/122; A61L 2202/16; A61L 2202/21; A61L 2202/23; A61L 2209/14; G01N 1/22; G01N 15/075;          (Forts.)

(86) Internationale Anmeldenummer:
**PCT/EP2022/070899**

(87) Internationale Veröffentlichungsnummer:
**WO 2023/006719 (02.02.2023 Gazette 2023/05)**

(54) **PARTIKELZÄHLVORRICHTUNG UND VERFAHREN ZUM PARTIKELZÄHLEN IN EINEM STERILISATIONSTUNNEL EINER MEDIKAMENTENABFÜLLANLAGE**

PARTICLE COUNTER AND METHOD FOR COUNTING PARTICLES IN A STERILIZATION TUNNEL OF A MEDICAMENT FILLING SYSTEM

DISPOSITIF DE COMPTAGE DE PARTICULES ET PROCÉDÉ DE COMPTAGE DE PARTICULES DANS UN TUNNEL DE STÉRILISATION D'UNE INSTALLATION DE CONDITIONNEMENT DE MÉDICAMENTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.07.2021  PCT/EP2021/071122**

(43) Veröffentlichungstag der Anmeldung:
**05.06.2024  Patentblatt 2024/23**

(73) Patentinhaber: **F. Hoffmann-La Roche AG**
**4070 Basel (CH)**

(72) Erfinder:
• **ZEPERT, Mario**
  **68305 Mannheim (DE)**
• **JUELLY, Patrick**
  **68305 Mannheim (DE)**

(74) Vertreter: **Altmann Stößel Dick Patentanwälte PartG mbB**
**Theodor-Heuss-Anlage 2**
**68165 Mannheim (DE)**

(56) Entgegenhaltungen:
EP-A1- 0 273 976      CN-U- 210 198 911
CN-Y- 201 269 796     US-A1- 2009 051 908

• **THOMAS SCHROTH ET AL: "Testing HEPA/ULPA filters at the manufacturer's facility and in situ on the user's premises", CLEANROOMS MAGAZINE, 1 October 2001 (2001-10-01), pages 36 - 44, XP055184503**

(52) Gemeinsame Patentklassifikation (CPC): (Forts.)
G01N 2015/0038; G01N 2015/0046;
G01N 2015/1486; G01N 2015/1493;
G01N 2203/0023; G01N 2203/0066

# EP 4 377 641 B1

**Beschreibung**

Technisches Gebiet

Die Erfindung betrifft eine Partikelzählvorrichtung zum Partikelzählen in einem Sterilisationstunnel einer Medikamentenabfüllanlage, einen Sterilisationstunnel einer Medikamentenabfüllanlage, eine Verwendung der Partikelzählvorrichtung sowie ein Verfahren zum Partikelzählen in einem Sterilisationstunnel einer Medikamentenabfüllanlage. Die Vorrichtungen und das Verfahren gemäß der vorliegenden Erfindung können beispielsweise in medizinischen und/oder pharmazeutischen Anlagen verwendet werden, in welchen besondere Anfordernisse an die Luftreinheit gestellt werden, beispielsweise in Reinräumen, in denen sich Medikamentenabfüllanlagen befinden, und in welchen daher eine Überprüfung der zur Reinhaltung der Anlagen verwendeten Filteranlagen notwendig ist. Alternativ und/oder zusätzlich sind jedoch auch andere Einsatzmöglichkeiten denkbar, wie beispielweise in Reinräumen zur Halbleiterfertigung und/oder zur Nahrungsmittelproduktion.

Technischer Hintergrund

In pharmazeutischen Anlagen zur Herstellung und/oder Abfüllung von Medikamenten findet die Herstellung und/oder Abfüllung flüssiger Medikamente im Allgemeinen in steril gehaltene Räumen, insbesondere in Reinräumen, statt. Abfüllungsgefäße für flüssige Medikamente können beispielsweise gläserne Injektionsfläschchen, Vials und/oder Spritzen aus Glas umfassen. Bei der Abfüllung der Medikamente in die Gefäße müssen diese im Allgemeinen partikel- und keimfrei sein. Die Reinigungs- und Sterilisations-Prozesse können für die verschiedenen Gefäße identisch sein und unter anderem eine Sterilisation in Sterilisationstunneln beinhalten.

Beispielsweise können Heißluftsterilisiertunnel eingesetzt werden, um die Gefäße vor einer Abfüllung mit Medikamenten zu entpyrogenisieren. Das Entpyrogenisieren umfasst dabei insbesondere eine Sterilisation der Gefäße mit trockener Hitze bei 160 bis 400°C. Im Allgemeinen ist ein nahezu partikelfreier laminarer Luftstrom erforderlich. Die Partikelfreiheit des Luftstroms kann mit in Heißluftsterilisationstunneln verbauten Filtern, beispielsweise mit HEPA-Filtern, realisiert werden. Die Qualität der verbauten Filter kann dabei in regelmäßigen Abständen mittels sogenannten Leckpenetrationstests überprüft werden, um eventuelle Überschreitungen von Partikelmengen im Luftstrom erfassen zu können. Dabei kann beispielsweise eine Filterfläche mäandrierend mit einer Messsonde abgetastet werden.

Solche Leckpenetrationstests können beispielsweise Teil einer regelmäßigen Wartung der Sterilisationstunnel im Rahmen von Standardabläufen, englisch: "Standard Operating Procedures (SOP)", sein und sogenannte DEHS-Tests umfassen, wobei DEHS (Di Ethyl-Hexyl-Sebacat) das zum Testen verwendete Aerosol bezeichnet. Bei einem Leckpenetrationstest wird im Allgemeinen vor dem zu untersuchenden Filter ein Aerosol aufgegeben und eine Partikelkonzentration vor dem Filter, insbesondere auf einer Rohluftseite, bestimmt. Auf der gegenüberliegenden Filterseite, insbesondere auf einer Reinluftseite, kann dann mit einer Sonde die Filterfläche nach eventuell erhöhten Partikeldurchlässen gefahndet werden. Im Allgemeinen wird die Sonde beim Leckpenetrationstest händisch geführt. Die trichterförmige Sonde kann dabei an einer verlängerbaren Rohrleitung in den Sterilisationstunnel eingebracht werden. Mit Hilfe der Rohrleitung kann ein Prüfer die Sonde manuell auf einem Transportband, unter dem Luftaustritt der Filter in einer vorgegebenen Zeit, mit einem vorgegebenen Weg und einer vorgegebenen Geschwindigkeit entlangführen. Der Prüfvorgang und die damit einhergehenden Parameter sind im Allgemeinen in SOPs festgelegt.

Über die trichterförmige Sonde und die Rohrleitung kann die aus den Filtern strömende Luft von einem Partikelzähler zugeführt werden, welcher die Konzentration der in der Luft enthaltenen Partikel bestimmen kann. Wird eine Partikelkonzentration über einer in der SOP festgelegten Schwelle festgestellt, so kann von einer Undichtigkeit und/oder einer Leckage des Filters ausgegangen werden. Wird an dieser Position erneut die Partikelkonzentration bestimmt, so kann im Falle einer nicht erhöhten Partikelkonzentration der Filter als funktionsfähig eingestuft werden und im Falle einer erhöhten Partikelkonzentration kann der Filter als nicht funktionsfähig eingestuft werden. Nicht funktionsfähige Filter erfordern im Allgemeinen ein aufwendiges Austauschen der Filter.

Bekannte Vorrichtungen und Verfahren zur Durchführung der Leckpenetrationstests weisen jedoch zahlreiche technische Herausforderungen auf. Insbesondere weisen die derzeitigen, manuellen Messmethoden im Allgemeinen eine schlechte Reproduzierbarkeit auf, da die Ergebnisse der manuellen Methodik erheblich von dem durchführenden Prüfer abhängen. Beispielweise ergeben sich Schwierigkeiten im Hinblick auf die Einhaltung von vorgegebenen Prüfgeschwindigkeiten. Zusätzlich kann je nach Tiefe des Sterilisationstunnels eine größere Rohrlänge der Messsonde erforderlich sein. Beispielsweise sind in einigen Sterilisationstunneln Filterflächen in bis zu 5 m Entfernung zu untersuchen, was mit einer handgeführten Sonde eine besondere Herausforderung darstellt. Darüber hinaus kann bei einer Bestimmung einer punktuell erhöhten Partikelkonzentration eine Herausforderung darin bestehen, die exakte Position zu bestimmen und diese auch wieder zu finden. Dies kann insbesondere mit einem erhöhten Zeitaufwand verbunden sein. Zeitaufwendigere Untersuchungen der Filteranlage in den Abfüllanlagen haben im Allgemeinen auch eine geringere zeitliche Verfügbarkeit der Abfüllanlagen zum eigentlichen Abfüllen der Medikamente zur Folge.

In der Bachelorarbeit von Patrick Jülly "Konzeptentwicklung für einen teilautomatischen Scanroboter zur Partikelzählung im Sterilisationstunnel" im Fachbereich Wirtschaftswissenschaften und Technologiemanagement der Wilhelm-Büchner-Hochschule in Darmstadt wird ein Scanroboter zur Partikelzählung im Sterilisationstunnel beschrieben.

CN 210 198 911 U offenbart eine Partikelzählvorrichtung umfassend mindestens eine mit einem Partikelzähler verbindbare Sonde zum Aufnehmen von Partikeln und einen Scanner mit einem Sondenhalter zum Befestigen der Sonde.

Aufgabe der Erfindung

Es wäre daher wünschenswert, eine Partikelzählvorrichtung zum Partikelzählen in einem Sterilisationstunnel einer Medikamentenabfüllanlage, einen Sterilisationstunnel einer Medikamentenabfüllanlage, eine Verwendung der Partikelzählvorrichtung sowie ein Verfahren zum Partikelzählen in einem Sterilisationstunnel einer Medikamentenabfüllanlage bereitzustellen, welche die Nachteile bekannter Vorrichtungen, Verwendungen und Verfahren zumindest weitgehend vermeiden. Insbesondere soll eine reproduzierbare Untersuchung von Filtern in Sterilisationstunneln ermöglicht werden, welche auch im Hinblick auf Zeit und Kosten ökonomisch ist.

Allgemeine Beschreibung der Erfindung

Diese Aufgabe wird adressiert durch eine Partikelzählvorrichtung zum Partikelzählen in einem Sterilisationstunnel einer Medikamentenabfüllanlage, einen Sterilisationstunnel einer Medikamentenabfüllanlage, eine Verwendung der Partikelzählvorrichtung sowie ein Verfahren zum Partikelzählen in einem Sterilisationstunnel einer Medikamentenabfüllanlage mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

In einem ersten Aspekt der vorliegenden Erfindung wird eine Partikelzählvorrichtung zum Partikelzählen in einem Sterilisationstunnel einer Medikamentenabfüllanlage vorgeschlagen. Der Sterilisationstunnel umfasst mindestens ein Transportband. Die Partikelzählvorrichtung umfasst mindestens eine mit einem Partikelzähler verbindbare Sonde zum Aufnehmen von Partikeln in dem Sterilisationstunnel. Weiterhin umfasst die Partikelzählvorrichtung mindestens einen Scanner mit mindestens einem Sondenhalter zum Befestigen der Sonde. Der Scanner umfasst mindestens einen Querläufer mit mindestens einer Linearführung. Die Linearführung ist eingerichtet, den Sondenhalter quer, insbesondere im Wesentlichen senkrecht, zu einer Transportrichtung des Transportbands des Sterilisationstunnels zu führen. Weiterhin umfasst der Scanner mindestens ein Fahrgestell. Der Querläufer ist auf dem Fahrgestell befestigt. Das Fahrgestell ist eingerichtet, die Linearführung in der Transportrichtung des Transportbands zu bewegen. Weiterhin umfasst der Scanner mindestens eine Steuerung, insbesondere eine mit dem Fahrgestell verbundene Steuerung, wobei die Steuerung eingerichtet ist, um eine Bewegung des Scanners zu steuern.

Das Fahrgestell kann eingerichtet sein, sich und den Querläufer, insbesondere den Querläufer mit der Sonde, in einem zweidimensionalen Raum zu bewegen. Die Partikelzählvorrichtung kann insbesondere jeweils einen Antrieb für eine Führung des Sondenhalters quer, insbesondere im Wesentlichen senkrecht, zu der Transportrichtung des Transportbands des Sterilisationstunnels und für eine Bewegung des Fahrgestells entlang der Transportrichtung des Transportbandes umfassen. Beide Antriebe können jeweils mit Hilfe eines Motors bewegt werden, wie nachfolgend noch näher ausgeführt wird. Insbesondere kann die Partikelzählvorrichtung derart ausgestaltet sein, dass die Bewegung des Fahrgestells unabhängig ist von einer Führung des Sondenhalters.

Der Begriff "Medikamentenabfüllanlage", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine beliebige Anlage beziehen, welche eingerichtet ist, ein oder mehrere Medikamente in Gefäße zu füllen. Bei den Gefäßen kann es sich beispielsweise um Vials, insbesondere Injektionsfläschchen, oder um Spritzen, insbesondere Spritzen aus Glas, handeln. Die Medikamentenabfüllanlage kann insbesondere in einem Reinraum aufgestellt sein und betrieben werden. Die Medikamentenabfüllanlage kann insbesondere den Sterilisationstunnel umfassen, welcher nachfolgend noch näher beschrieben wird. Der Sterilisationstunnel kann eingerichtet sein, eine Partikel- und/oder Keimfreiheit von den Gefäßen zu erzielen. Weiterhin kann die Medikamentenabfüllanlage mindestens eine Spülmaschine, mindestens eine Abfüllanlage und/oder mindestens eine Inspektionsmaschine umfassen. Die Spülmaschine kann eingerichtet sein, die Gefäße mit Wasser für Injektionszwecke (WFI) zu reinigen. Die Medikamentenabfüllanlage kann eingerichtet sein, die Gefäße von der Spülmaschine auf das Transportband des Sterilisationstunnels zu transportieren. Optional kann die Medikamentenabfüllanlage mindestens eine Gefriertrocknungsanlage umfassen. Die Gefriertrocknungsanlage kann eingerichtet sein, abgefüllte Medikamente nach der Abfüllung zu gefriertrocknen, insbesondere um eine Haltbarkeit der abgefüllten Medikamente zu gewährleisten.

Der Begriff "Sterilisationstunnel", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche dazu eingerichtet ist, um ein oder mehrere Objekte, welche den Sterilisationstunnel durchlaufen, beispielweise Medikamentenbehälter, von mikroskopischen Verunreinigungen zu befreien oder derartige Verunreinigungen zumindest teilweise zu beseitigen. Der Sterilisationstunnel kann insbesondere zur Sterilisierung der Objekte eingerichtet sein. Die Sterilisierung kann sich dabei insbesondere auf einen Vorgang beziehen, bei welchem die Objekte vollständig oder teilweise von anhaftenden Keimen befreit werden und/oder bei welchem eine Keimreduktion an und/oder in den Objekten erfolgt. Diese Keimreduktion kann beispielsweise durch eine thermische Behandlung und/oder durch eine chemische Behandlung der Objekte erfolgen, beispielsweise durch eine Behandlung mit einem desinfizierenden Gas und/oder mit Heißdampf. Bevorzugt erfolgt die Keimreduktion durch eine thermische Behandlung in dem Sterilisationstunnel.

Der Begriff "Transportband", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um mindestens eine andere Vorrichtung oder mindestens ein anderes Element, beispielsweise ein oder mehrere zu befüllende Gefäße, zu transportieren und/oder eine Bewegung der anderen Vorrichtung oder des anderen Elements anzutreiben. Das Transportband kann insbesondere mindestens ein Antriebselement umfassen, beispielsweise mindestens ein im Kreislauf durch den Sterilisationstunnel laufendes Antriebselement. Insbesondere kann das Transportband zumindest teilweise luftdurchlässig sein, beispielsweise derart, dass zumindest ein Teil der dem Transportband zugeführten Zuluft durch das Transportband hindurchtreten kann. Beispielsweise umfasst das Transportband ein Drahtgittergeflecht. Das Drahtgittergeflecht kann dabei insbesondere eine notwendige Luftdurchlässigkeit, Flexibilität und Hitzebeständigkeit gewährleisten. Der Transport kann beispielsweise kontinuierlich oder auch diskontinuierlich oder getaktet erfolgen, so dass beispielsweise kontinuierlich arbeitende Sterilisationstunnel oder auch getaktete Sterilisationstunnel verwendet werden können. Die Transportrichtung kann beispielsweise eine Hauptrichtung einer Bewegung der zu befüllenden Gefäße innerhalb des Sterilisationstunnels sein. Die Transportrichtung kann fest vorgegeben sein oder kann sich auch ändern, beispielsweise lokal oder auch zeitlich. Beispielsweise kann die Transportrichtung von einem Einlauf des Sterilisationstunnels hin zu einem Auslauf gerichtet sein. Die Transportrichtung kann beispielsweise eine Hauptrichtung einer Bewegung der Gefäße innerhalb des Sterilisationstunnels sein. Die Transportrichtung kann fest vorgegeben sein oder kann sich auch ändern, beispielsweise lokal oder auch zeitlich. Beispielsweise kann die Transportrichtung von einem Eingang des Sterilisationstunnels hin zu einem Ausgang des Sterilisationstunnels gerichtet sein. Die Transportrichtung kann daher auch als Förderrichtung bezeichnet werden.

Der Begriff "Schwebstofffilter", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche dazu eingerichtet ist, Schwebstoffe, alternativ auch als "Partikel" bezeichnet, aus mindestens einem Medium, insbesondere aus mindestens einem gasförmigen Medium wie beispielsweise Luft, welches den Schwebstofffilter durchströmt, zumindest teilweise abzuscheiden. Das zumindest teilweise Abscheiden kann dabei insbesondere ein vollständiges Entfernen der Schwebstoffe aus dem durchströmenden Medium oder, alternativ, eine Reduktion einer Konzentration der Schwebstoffe in dem durchströmenden Medium, beispielsweise um mindestens 85 %, bevorzugt um mindestens 95 %, besonders bevorzugt um mindestens 99,95 %, einer Konzentration von Schwebstoffen mit Partikelgröße im Bereich von 0.1 $\mu$m bis 0.3 $\mu$m, umfassen. Der Schwebstofffilter kann insbesondere einen oder mehrere Schwebstoffe aus dem den Schwebstofffilter durchströmenden Medium abscheiden, beispielsweise Bakterien, Viren, Pollen, Stäube, Aerosole und/oder Rauchpartikel. Der Schwebstofffilter kann mindestens einen Filter umfassen ausgewählt aus der Gruppe bestehend aus: einem EPA-Filter (englisch: Efficient Particulate Air); einem HEPA-Filter (englisch: High Efficiency-Particulate Air); einem ULPA-Filter (englisch: Ultra Low Penetration Air). Besonders bevorzugt kann der Schwebstofffilter mindestens einen HEPA-Filter umfassen.

Der Begriff "Partikelzählvorrichtung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche das Partikelzählen in einem Sterilisationstunnel einer Medikamentenabfüllanlage ermöglicht. Die Partikelzählvorrichtung kann dabei insbesondere für einen Prozess des Partikelzählens verwendet werden. Die Partikelzählvorrichtung kann während dem Prozess des Partikelzählens insbesondere die Aufgabe der Führung einer Sonde in dem Sterilisationstunnel zukommen. Die Partikelzählvorrichtung kann zur automatisierten, insbesondere teil- oder vollautomatisierten, Führung der Sonde in dem Sterilisationstunnel eingerichtet sein. Alternativ und/oder zusätzlich kann die Partikelzählvorrichtung dazu eingerichtet sein, einem Partikelzähler ein gasförmiges Medium, beispielsweise Luft, mit den darin enthaltenen und zu zählenden Partikeln zuzuführen.

Die Partikelzählvorrichtung kann eingerichtet sein, mittels der Sonde auf einem vorgegebenen Weg mit einer vorgegebenen Geschwindigkeit verschieden große Flächen unterhalb eines Schwebstofffilters des Sterilisationstunnels abzufahren. Die Sonde kann eingerichtet sein, die durch den Schwebstofffilter strömende Luft aufzunehmen und zu dem, mit der Sonde verbundenen, Partikelzähler zu leiten. Der Partikelzähler kann, wie nachfolgend noch näher ausgeführt wird, eingerichtet sein, vorhandene Partikel in der gefilterten Luft zu zählen und/oder zu messen.

Die Partikelzählvorrichtung kann weiterhin den mindestens einen mit der Sonde verbindbaren Partikelzähler umfassen. Der Begriff "Partikelzähler", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche zur quantitativen und/oder qualitativen Erfassung von Partikeln in einem gasförmigen Medium, insbesondere in Luft, eingerichtet ist. Der Partikelzähler kann sich insbesondere auf eine Vorrichtung beziehen, welche zum Partikelzählen in dem gasförmigen Medium eingerichtet ist. Der Partikelzähler kann zur optischen Erfassung der Partikel in dem gasförmigen Medium eingerichtet sein. Beispielsweise kann der Partikelzähler mindestens eine Lichtquelle, mindestens eine Messzelle, welche zumindest einen Teil des gasförmigen Mediums enthält, sowie mindestens einen Photodetektor umfassen, welcher das von der Lichtquelle ausgesandte und an den im gasförmigen Medium enthaltenen Partikeln gestreute und/oder gebeugte Licht detektieren kann. Basierend auf dem vom Photodetektor detektierten Signal können die im gasförmigen Medium enthaltenen Partikel qualitativ und/oder quantitativ erfasst werden. Die Messzelle des Partikelzählers kann von dem gasförmigen Medium kontinuierlich durchströmt oder, alternativ, mit dem zu untersuchenden gasförmigen Medium diskontinuierlich befüllt werden. Somit kann der Partikelzähler kontinuierlich oder diskontinuierlich die Partikel in dem gasförmigen Medium erfassen. Der Partikelzähler kann insbesondere eine Anzahl, eine Größe und/oder eine Konzentration der Partikel in dem gasförmigen Medium erfassen. Der Partikelzähler kann Partikel mit einer Größe im Bereich von 10 nm bis 1000 $\mu$m, bevorzugt im Bereich von 100 nm bis 100 $\mu$m, besonders bevorzugt im Bereich von 0.3 $\mu$m bis 10 $\mu$m, erfassen. Entsprechend kann sich der Begriff "Partikelzählen", alternativ auch als "Partikelzählung" bezeichnet, grundsätzlich auf einen beliebigen Vorgang zur quantitativen und/oder qualitativen Erfassung von Partikeln in einem gasförmigen Medium, insbesondere in Luft, beziehen.

Insbesondere kann der Partikelzähler als stationärer Partikelzähler ausgestaltet sein und der Partikelzähler und die Sonde können mittels mindestens einer Rohrleitung, insbesondere einer flexiblen Rohrleitung, miteinander verbunden sein. Die Rohrleitung kann dabei Teil des Partikelzählers und/oder Teil der Partikelzählvorrichtung sein. Die Sonde und die Rohrleitung können eingerichtet sein, Luft anzusaugen und dem Partikelzähler zuzuführen. Der Partikelzähler kann insbesondere eingerichtet sein, Partikel zu zählen und/oder zu messen. Bei einer Überschreitung einer gemessenen Partikelzahl und/oder einer gemessenen Partikelkonzentration über einen definierten Wert kann auf eine Undichtigkeit, insbesondere auf eine Leckage, des Schwebstofffilters geschlossen werden. An der Position der Partikelzählvorrichtung auf dem Transportband mit der gemessenen erhöhten Partikelkonzentration oder Partikelzahl kann daraufhin nochmals genauer gemessen werden. Ergibt eine erneute Messung keine erhöhte Partikelkonzentration oder Partikelzahl, kann der Schwebstofffilter als funktionsfähig eingestuft werden. Wird jedoch wiederholt eine erhöhte Partikelkonzentration oder Partikelzahl gemessen, muss der Schwebstofffilter aufwendig ausgetauscht werden.

Der Begriff "Sonde", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche zur Informations- und/oder Objektübermittlung eingerichtet ist. Insbesondere kann die Sonde zur Objektvermittlung eingerichtet sein. Beispielsweise kann die Sonde eingerichtet sein, Partikel an einer ersten Position aufzunehmen und einer zweiten, von der ersten Position verschiedenen Position zuzuführen. Die Sonde kann so insbesondere das Partikelzählen an der ersten Position ermöglichen während die Erfassung der Partikel an der zweiten Position stattfindet. Alternativ und/oder zusätzlich kann auch eine direkte Erfassung der Partikel an der ersten Position und eine Informationsübermittlung eines Ergebnisses des Partikelzählens an die zweite Position möglich sein. Die Sonde kann insbesondere eine isokinetische Sonde sein. Der Begriff "isokinetische Sonde" bezeichnet grundsätzlich eine beliebige Sonde, welche eingerichtet ist, eine Probe, insbesondere Partikel, aus strömenden Fluiden aufzunehmen. Insbesondere kann das in die isokinetische Sonde fließende Fluid eine Geschwindigkeit aufweisen, welche einer Geschwindigkeit eines Fluids in einer unmittelbaren Umgebung der isokinetischen Sonde entspricht. Dadurch kann eine Verfälschung einer Partikelzahl des in die isokinetische Sonde fließenden Fluids während der Partikelaufnahme vermieden oder zumindest reduziert werden.

Der Begriff "Scanner", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine beliebige Vorrichtung beziehen, welche eingerichtet ist, einen Bereich, insbesondere einen zweidimensionalen Bereich, auf eine systematische und/oder regelmäßige Weise abzufahren. Insbesondere kann es sich bei dem Bereich um einen Bereich innerhalb des Sterilisationstunnels, insbesondere um einen Bereich unterhalb des mindestens einen Schwebstofffilters, insbesondere unterhalb mindestens einer Filterfläche des Schwebstofffilters, des Sterilisationstunnels handeln. Der Scanner kann vorzugsweise eingerichtet sein, den Bereich unterhalb des mindestens einen Schwebstofffilters in zumindest teilweise überlappenden Bahnen abzufahren, wie nachfolgend näher erläutert wird. Weiterhin kann der Scanner vorzugsweise eingerichtet sein, einen Fahrweg mit einem Mäandermuster durch abwechselnde Bewegungen der Sonde quer und parallel zu der Transportrichtung abzufahren, wie nachfolgend noch näher erläutert wird.

Wie oben ausgeführt, umfasst der Scanner den mindestens einen Sondenhalter. Der Sondenhalter kann zum Befestigen der Sonde eingerichtet sein. Der Begriff "Sondenhalter", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine beliebige Vorrichtung beziehen, welche eingerichtet ist, eine beliebig ausgestaltete Sonde auf einer Komponente des Scanners zu befestigen. Insbesondere kann der Sondenhalter eingerichtet sein, die Sonde auf einem Führungsschlitten der Linearführung zu befestigen. Der Sondenhalter selbst kann auf der Komponente des Scanners, insbesondere auf dem Führungsschlitten befestigt sein. Der Sondenhalter kann daher mindestens eine Aussparung aufweisen, in welche der Sondenhalter zumindest teilweise aufgenommen werden kann. Weiterhin kann die Aussparung eingerichtet sein, die Rohrleitung zumindest teilweise aufzunehmen. Insbesondere kann der Sondenhalter mindestens eine Nut für eine Aufnahme der Sonde aufweisen. Weiterhin kann der Sondenhalter mindestens eine Klemmplatte aufweisen, welche eingerichtet ist, die Sonde zu fixieren. Auch andere Ausgestaltungen sind grundsätzlich denkbar. Weiterhin kann der Sondenhalter zumindest teilweise aus Polyoxymethylene (POM) hergestellt sein. Dies kann zu einer Gewichtersparnis der Partikelzählvorrichtung führen. Auch andere Materialien sind grundsätzlich denkbar.

Wie oben ausgeführt, umfasst der Scanner mindestens einen Querläufer mit mindestens einer Linearführung. Der Begriff "Querläufer", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Komponente der Partikelzählvorrichtung beziehen, welche eingerichtet ist, mindestens eine Komponente der Partikelzählvorrichtung quer, insbesondere im Wesentlichen senkrecht, zu der Transportrichtung des Transportbands zu führen. Der Querläufer kann daher auch als Querachse bezeichnet werden. Der Querläufer weist die mindestens eine Linearführung auf, welche nachfolgend noch näher erläutert wird.

Der Begriff "Linearführung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine beliebige Vorrichtung beziehen, welche eingerichtet ist, eine geradlinige, geführte Bewegung eines Bauteils von einem Punkt zu einem anderen Punkt zu ermöglichen. Die Linearführung kann insbesondere eingerichtet sein, die Sonde quer zu der Transportrichtung des Sterilisationstunnels geradlinig und mit einer konstanten Geschwindigkeit zu bewegen. Die Linearführung kann eingerichtet sein, sechs Freiheitsgrade des Bauteils, insbesondere drei translatorische Freiheitsgrade und drei rotatorische Freiheitsgrade, auf einen, insbesondere einzigen, translatorischen Freiheitsgrad des Bauteils zu beschränken. Die Linearführung kann mindestens eine Führungsschiene, insbesondere eine profilierte Führungsschiene, insbesondere eine T-Führungsschiene, oder eine Rundwelle umfassen. Weiterhin kann die Linearführung mindestens einen Führungsschlitten umfassen, insbesondere mindestens einen auf der Führungsschiene oder der Rundwelle gelagerten Führungsschlitten. Die Sonde kann an dem Führungsschlitten befestigbar oder befestigt sein, insbesondere mittels des Sondenhalters. Die Linearführung kann daher eingerichtet sein, die Sonde zu führen. Die Linearführung kann insbesondere mindestens ein Gleitlager aufweisen, insbesondere für den Führungsschlitten. Das Gleitlager kann schmiermittelfrei ausgestaltet sein. Andere Lager wie Kugel- oder Rollenlager sind jedoch ebenfalls grundsätzlich denkbar.

Durch die T-Führungsschiene ist es grundsätzlich möglich, dass der Führungsschlitten mit Loslager in der Richtung quer, insbesondere senkrecht, zu der Transportrichtung des Transportbands und/oder in der Transportrichtung des Transportbands ausgeführt werden kann. Das Loslager ermöglicht, dass der Führungsschlitten in die gewählte Richtung etwas Spiel hat. So können Fertigungstoleranzen in der Konstruktion ausgeglichen werden. Ohne Loslager könnte man ein starres System erhalten, wodurch beispielsweise ein Verkanten des Führungsschlittens möglich wäre. Insbesondere kann die Führungsschiene als Loslager eingerichtet sein, insbesondere als Loslager in einer Richtung quer, insbesondere senkrecht, zu der Transportrichtung des Transportbands. Hierdurch kann der Führungsschlitten in der Lage sein, kleine Höhenunterschiede, insbesondere in einem Gesamtsystem, auszugleichen. Eine Länge des Führungsschlittens kann einer Flanschbreite eines Motors entsprechen.

Bei der Linearführung kann es sich insbesondere um eine drylin® T-Miniatur-Linearführung (Igus, Deutschland) aus hartanodisiertem Aluminium handeln. Die drylin® T-Miniatur-Linearführung kann eine Gesamthöhe von 16 mm, eine Länge des Führungsschlittens von 42 mm, eine Breite des Führungsschlittens von 32 mm und eine Schienenlänge aufweisen, welche individuell anpassbar ist. Die drylin® T-Miniatur-Linearführung kann daher eine geringe Bauhöhe sowie Gleitlager aufweisen. Eine Länge des Führungsschlittens kann kleiner oder gleich einer Flanschgröße eines Schrittmotors sein. Die drylin® T-Miniatur-Linearführung kann wartungs- und schmiermittelfrei ausgeführt sein. Die drylin® T-Miniatur-Linearführung kann polymere Hochleistungs-Gleitelemente mit guten Verschleiß- und Reibwerten aufweisen. Weiterhin kann die drylin® T-Miniatur-Linearführung eine T-Führungsschiene aufweisen. Dadurch, dass kein Schmiermittel nötig ist, wird grundsätzlich eine Verschmutzung durch Öle und Fette vermieden. Schmutz oder Staubpartikel können grundsätzlich nicht anhaften. Laut Hersteller ist dieses System unempfindlich gegen Wasser, Chemikalien, Hitze und Stöße. Anforderungen der Schmiermittelfreiheit und der Reinigbarkeit können somit erfüllt sein. Baulängen der Führungsschienen können individuell an verschiedene Tunnelbreiten des Sterilisationstunnels anpassbar sein.

Die Linearführung kann eingerichtet sein, die Sonde quer zu der Transportrichtung des Sterilisationstunnels zu führen. Der Führungsschlitten der Linearführung kann insbesondere für eine Befestigung aller Komponenten, welche für eine Erzeugung und Durchführung der linearen Bewegung notwendig sind, sowie des Sondenhalters, eingerichtet sein, wie nachfolgend nach näher ausgeführt wird. Insbesondere kann der Sondenhalter auf dem Führungsschlitten befestigt sein.

Die Linearführung, insbesondere die Führungsschiene, kann auf einer Grundplatte, insbesondere auf einer Grundplatte aus Aluminium, befestigt sein, insbesondere mittels mindestens einer Schraubverbindung. Die Grundplatte kann austauschbar ausgeführt sein. Sollte die Führungsschiene Verschleiß aufweisen, kann sie bei Bedarf jederzeit ausgetauscht werden. Die Linearführung kann mittels der Grundplatte auf dem Fahrgestell befestigt sein. Insbesondere kann die Grundplatte auf dem Fahrgestell mittels mindestens einer Verbindung befestigt sein ausgewählt aus der Gruppe bestehend aus: mindestens einer Schraubverbindung, mindestens einer Klick-Verbindung, mindestens einer Spannebelverbindung. Die Schraubverbindung kann insbesondere Rändelschrauben und/oder Zylinderkopfschrauben umfassen. Insbesondere kann die Grundplatte eine Vielzahl von Bohrungen umfassen, insbesondere um den Querläufer auf dem Fahrgestell zu befestigen. Aufgrund einer hohen Anzahl von jährlich notwendigen Wechseln des Querläufers, circa sechs Mal pro Jahr, kann eine Befestigung des Querläufers auf dem Fahrgestell mit Rändelschrauben vorteilhaft sein. Dadurch lässt sich grundsätzlich eine kompakte Konstruktion erstellen. So ist eine Möglichkeit eines werkzeugfreien Wechsels grundsätzlich gegeben und der Querläufer kann fest, aber lösbar, mit dem Fahrgestell verbunden sein. Die Grundplatte kann, insbesondere um Gewicht einzusparen, durch Ausklinken von nicht benötigtem Material angepasst werden. Auch weitere Ausführungsformen für eine Befestigung des Querläufers auf dem Fahrgestell sind grundsätzlich denkbar, wie Klick-Systeme oder Spannhebel.

Die Linearführung kann mindestens einen Antrieb, insbesondere einen linearen Antrieb, aufweisen. Der Antrieb kann eingerichtet sein, den Führungsschlitten auf der Führungsschiene zu bewegen. Der Antrieb kann eingerichtet sein, eine gesamte Transportbandbreite des Transportbands des Sterilisationstunnels abzufahren. Der Antrieb kann ausgewählt sein aus der Gruppe bestehend aus: einem Spindelantrieb; einem Zahnriemenantrieb, einem Zahnstangenantrieb. Auch andere Ausführungsformen sind grundsätzlich denkbar.

Der Zahnriemenantrieb kann insbesondere mindestens einen Zahnriemen und mindestens zwei Zahnriemenräder aufweisen. Eins der Zahnriemenräder kann eingerichtet sein, durch einen Motor angetrieben zu werden. Der Zahnriemen kann für eine Führung über die Zahnriemenräder eingerichtet sein. Ein auf dem Zahnriemen befestigtes Objekt, zum Beispiel ein Schlitten, kann dadurch bewegt werden. Der Zahnriemen kann eine Vielzahl von Zähnen aufweisen. Weiterhin können die Zahnriemenräder jeweils eine Vielzahl an Zähnen aufweisen. Eine Form der Zähne des Zahnriemens kann auf eine Form der Zähne des Zahnriemenrades angepasst sein. Hierdurch ergibt sich eine formschlüssige Kraftübertragung. Mit der passenden Zahnform kann sich grundsätzlich ein spielfreier Antrieb realisieren lassen. Durch ein Ändern einer Drehrichtung der Zahnriemenräder kann sich der Zahnriemen, und somit der Schlitten, linear in beide Richtungen bewegen. Ein Lauf eines Zahnriemenantriebs ist grundsätzlich geräuscharm. Eine Übertragung einer schlupffreien und synchronen Bewegung kann grundsätzlich stoßdämpfend und mit einer niedrigen Vorspannung erfolgen. Über eine Drehzahl eines Motors kann eine Verfahrgeschwindigkeit des Führungsschlittens geregelt werden. Ein Aufbau des Zahnriemenantriebs kann in der Höhe sehr klein gestaltet werden und eignet sich grundsätzlich für eine schnelle Positionierung kleinerer Lasten. Eine Schmierung ist grundsätzlich nicht notwendig. Zahnriemenantriebe sind auf dem Markt grundsätzlich bereits als vollständige Einheit erhältlich. Dies kann eine Konstruktion grundsätzlich vereinfachen.

Der Spindelantrieb kann mindestens eine Spindel aufweisen. Weiterhin können der Führungsschlitten und die Führungsschiene Komponenten des Spindelantriebs sein. Der Führungsschlitten kann insbesondere ein Innengewinde aufweisen, welches kompatibel zu einem Gewinde der Spindel ist. Die Spindel kann eingerichtet sein, mittels eines Motors angetrieben zu werden und eine Rotation der Spindel kann durch ein Ineinandergreifen der Gewinde in eine lineare Bewegung des Führungsschlittens umgewandelt werden. Durch die Führungsschiene kann der Führungsschlitten an einer Rotation um eine Achse der Spindel gehindert werden. Die Spindel kann insbesondere eine Kugelumlaufspindel oder eine Trapezspindel sein. Durch eine Drehrichtung des Motors kann eine Bewegungsrichtung des Führungsschlittens gesteuert werden. Eine Steigung des Gewindes kann einen Vorschub des Führungsschlittens pro Spindelumdrehung angeben. Eine größere Steigung kann eine größere Verfahrgeschwindigkeit je Umdrehung bewirken. Der Spindelantrieb kann grundsätzlich wesentlich lautere Laufgeräusche als der Zahnriemenantrieb erzeugen und kann grundsätzlich eine Schmierung benötigen.

Vorzugsweise kann die Linearführung den mindestens einen Zahnstangenantrieb aufweisen. Der Zahnstangenantrieb kann mindestens eine Zahnstange und mindestens ein Stirnzahnrad aufweisen. Beim Zahnstangenantrieb wird eine rotierende Bewegung des Stirnzahnrades in eine lineare Bewegung umgewandelt. Hierbei gibt es grundsätzlich zwei Möglichkeiten.

Zum einen kann das Stirnzahnrad mit einem Motor als Antriebseinheit feststehend ausgebildet sein und eingerichtet sein, eine linear gelagerte Zahnstange anzutreiben. Die linear gelagerte Zahnstange kann eingerichtet sein, sich je nach Drehrichtung des Zahnrades zu bewegen. Zum anderen kann die Zahnstange fixiert sein und eingerichtet sein, das Stirnzahnrad sowie eine Antriebseinheit linear, insbesondere entlang der Zahnstange, zu bewegen. Insbesondere kann die Antriebseinheit mit dem Stirnzahnrad auf dem Führungsschlitten der Linearführung befestigt sein. Diese Flexibilität ist grundsätzlich ein großer Vorteil von Zahnstangenantrieben. Zudem lässt sich grundsätzlich eine gesamte Länge der Zahnstange als Verfahrweg nutzen. Der Zahnstangenantrieb lässt sich grundsätzlich aufgrund der beschriebenen Funktionsweise kompakt aufbauen und bei einer geeigneten Materialauswahl grundsätzlich ohne Schmierung betreiben. Der Zahnstangenantrieb ist grundsätzlich formschlüssig, schlupffrei und kann bei genauer Fertigung einen hohen Wirkungsgrad erreichen. Ähnlich wie der Zahnriemenantrieb, ist der Zahnstangenantrieb grundsätzlich geräuscharm.

Die Zahnstange kann insbesondere einen runden Querschnitt aufweisen. Die Zahnstange kann insbesondere für eine zusätzliche Führung für den Sondenhalter eingerichtet sein. Der runde Querschnitt kann grundsätzlich eine Fertigung bei der zusätzlichen Führung vereinfachen. Die Zahnstange kann grundsätzlich auf benötigte Längen gekürzt werden. Die Zahnstange kann insbesondere aus einem austenitischen rostfreiem Stahl hergestellt sein. Insbesondere kann die Zahnstange aus einem austenitischen rostfreiem Stahl hergestellt sein mit einem Durchmesser von 10 mm, einer Lieferlänge von 1000 mm, einem Gewicht von 560 g und einem Elastizitätsmoduls $E$ von 200000 N/mm$^2$ und einem Modul $m$ von 1, wobei das Modul m einem Verzahnungsmaß für Zahnräder entspricht. Das Material weist die Werkstoffnummer 1.4305 gemäß EN 10027-2:1992-09 auf.

Wie oben ausgeführt, kann die Zahnstange, je nach Tunneltyp, auf die benötigte Länge gekürzt werden. Ausgehend von einer größten benötigten Länge des Querläufers kann eine Durchbiegung $f$ der Zahnstange berechnet werden. Hierbei kann überprüft werden, wie sich die Zahnstange durch ihr Eigengewicht durchbiegt. Die dadurch gewonnene Erkenntnis kann bei einer Konstruktion des Querläufers berücksichtigt werden. Für die Berechnung kann ein Tunneltyp des Sterilisationstunnels mit einer Breite des Transportbandes von 800 mm verwendet werden. Hier ist grundsätzlich eine größtmögliche Durchbiegung der Zahnstange zu erwarten. Zur Berechnung der Durchbiegung $f$ kann folgende Formel verwendet werden:

$$f = \frac{F * l^3}{384 * E * I} \tag{1}$$

Dabei entspricht $F$ einer Gewichtskraft der Zahnstange, $l$ einer Länge einer Streckenlast und $I$ einem Flächenmoment 2. Grades.

Die Zahnstange kann doppelseitig an zwei Zahnstangenhaltern mit je 10 mm Breite eingespannt sein. Daraus kann sich folgende Länge der Streckenlast *l* ergeben:

$$l = 800\ mm - 2 * 10\ mm = 780\ mm \tag{2}$$

In einer Nebenrechnung kann ein Gewicht *m* der gekürzten Zahnstange ermittelt werden:

$$m = \frac{8560\ g * 780\ mm}{1000\ mm} = 436.8\ g = 0{,}4368\ kg \tag{3}$$

Die Gewichtskraft *F* der Zahnstange kann wie folgt berechnet werden:

$$F = m * g = 0{,}4368\ kg * 9{,}81\ \frac{m}{s^2} = 4{,}287\ N \tag{4}$$

Das Flächenmoment *I* 2. Grades (TBB) kann folgendermaßen ermittelt werden:

$$I = \frac{\pi * d^4}{64}\ \frac{\pi * (10\ mm)^4}{64} = 490{,}87\ mm^4 \tag{5}$$

Die ermittelten Werte können in die Formel (1) eingesetzt werden, um die Durchbiegung *f* der Zahnstange zu ermitteln:

$$f = \frac{4{,}287\ N * (780\ mm)^3}{382 * 200000 \frac{N}{mm^2} * 490{,}87\ mm^4} = 0{,}054\ mm \tag{6}$$

Der ermittelte Wert der Durchbiegung *f* beträgt bei der größten Länge 0,054 mm. Der Führungsschlitten mit dem Loslager in der Richtung quer zu der Transportrichtung des Transportbands kann diesen Unterschied grundsätzlich problemlos ausgleichen. Die Durchbiegung der Zahnstange durch ihr Eigengewicht ist demnach grundsätzlich so gering, dass sie für die Konstruktion des Querläufers grundsätzlich nicht weiter beachtet werden muss. Bei kürzeren Baulängen des Querläufers ist grundsätzlich von einer geringeren Durchbiegung der Zahnstange auszugehen.

Der Querläufer kann weiterhin mindestens eine, vorzugsweise mindestens zwei, Zahnstangenhalterungen aufweisen. Die Zahnstangenhalterung kann eingerichtet sein, die Zahnstange zu fixieren, insbesondere auf der Grundplatte des Querläufers. Die Zahnstangenhalterung kann insbesondere aus Aluminium hergestellt sein. Auch andere Ausführungsformen sind grundsätzlich denkbar. Die Zahnstangenhalterung kann insbesondere ein Oberteil und ein Unterteil aufweisen. Das Unterteil kann eingerichtet sein, auf der Grundplatte fixiert zu sein, insbesondere mittels mindestens einer Schraubverbindung. Insbesondere kann der Querläufer, wie oben ausgeführt, zwei der Zahnstangenhalterungen aufweisen und die Unterteile können jeweils an Enden der Führungsschiene angeordnet sein. Das Oberteil kann eingerichtet sein, mit dem Unterteil verschraubt zu werden und kann weiterhin eingerichtet sein, die Zahnstange zu fixieren, insbesondere derart, dass eine Verdrehung und/oder Verschiebung der Zahnstange vermieden oder zumindest reduziert wird.

Wie oben ausgeführt, kann der Zahnstangenantrieb insbesondere eingerichtet sein, mittels einer Drehbewegung des Stirnzahnrads den Führungsschlitten über die Zahnstange, welche insbesondere auf der Grundplatte des Querläufers fixiert sein kann, zu bewegen. Das Stirnzahnrad kann insbesondere aus Polyoxymethylene (POM) hergestellt sein. Im Vergleich zu einem Stirnzahnrad aus Metall, können Laufgeräusche grundsätzlich minimiert werden und es kann grundsätzlich auf eine Schmierung verzichtet werden. Darüber hinaus kann das Stirnzahnrad aus Polyoxymethylene (POM) grundsätzlich ein vergleichsweise geringes Gewicht, sowie vergleichsweise niedrige Herstellungskosten aufweisen.

Das Stirnzahnrad kann insbesondere eine Zähnezahl von 19 aufweisen. Weiterhin kann das Stirnzahnrad ein Modul *m* von 1 aufweisen, wobei das Modul *m* einem Verzahnungsmaß für Zahnräder entspricht. Hierdurch ergeben sich folgende Werte für einen Teilkreisdurchmesser $d_z$ und einer zurückgelegten Strecke $l_z$ bei einer vollen Umdrehung des Zahnrads:

$$d_z = m * z \tag{7}$$

$$d_z = 1 * 19 = 19\ mm \tag{8}$$

$$l_z = \pi * d_z \tag{9}$$

$$l_z = \pi * 19\,mm = 56{,}69\,mm \tag{10}$$

Somit kann ein mit dem Zahnstangenantrieb verbundener Motor eine Umdrehung pro Sekunde durchführen, um eine geforderte Geschwindigkeit bei einem Partikelzählen von 5,9 cm/s einzuhalten. Dieser Wert kann für eine Programmierung des Motors eingesetzt werden.

Das Stirnzahnrad kann als kraftschlüssige Verbindung mit einem Gewindestift mit Schneidring auf einer Motorwelle des Motors des Zahnstangenantriebs verspannt sein. Die Motorwelle kann eine Abflachung aufweisen. Die Abflachung auf der Motorwelle kann dem Gewindestift eine vergleichsweise größere Fläche bieten, um einen vergleichsweise höheren Anpressdruck zu erzeugen. Beim Anziehen des Gewindestiftes kann ein Grat auf einer Oberfläche der Motorwelle entstehen. Durch die Abflachung der Motorwelle wird eine Demontage des Stirnzahnrades aufgrund des entstandenen Grates grundsätzlich nicht behindert. Ein Durchdrehen der Motorwelle wird grundsätzlich ebenfalls erschwert.

Durch eine genaue Positionierung des Stirnzahnrades zur Zahnstange können die Zähne des Stirnzahnrades grundsätzlich bestmöglich in die Zähne der Zahnstange greifen. Hierdurch wird grundsätzlich ein Spiel zwischen ineinandergreifenden Zähnen zumindest weitestgehend reduziert. Ein optimaler Achsabstand $a$ von Zahnstange und Zahnrad wird folgend berechnet, wobei $d_0$ einer Teilkreislinie und $d$ einem Durchmesser der Zahnstange entspricht:

$$d_0 = \frac{d}{2} * m \tag{11}$$

$$d_0 = \frac{10\,mm}{2} * 1 = 5\,mm \tag{12}$$

$$a = d_0 + \frac{d_z}{2} \tag{13}$$

$$a = 5\,mm + \frac{19\,mm}{2} = 14{,}5\,mm \tag{14}$$

Ein Befestigungswinkel, insbesondere aus Aluminium, kann an Gewindebohrungen des Führungsschlittens befestigt, insbesondere verschraubt, sein. Der Schrittmotor kann ebenfalls am Befestigungswinkel befestigt, insbesondere verschraubt, sein und kann eingerichtet sein, das Stirnzahnrad mit dem Achsabstand $a$ zur Zahnstange zu positionieren. Die Funktion des Zahnstangenantriebes ist somit grundsätzlich gegeben. Durch das Drehen der Motorwelle und damit des Stirnzahnrades kann sich der Führungsschlitten auf der Führungsschiene bewegen. Für eine Montage des Sondenhalters können Bohrungen am Befestigungswinkel vorgesehen sein.

Durch die Verwendung eines Zahnstangenantriebs mit den oben dargestellten Eigenschaften kann mit einer geschickten Auswahl und Anordnung der Bauteile der Führungsschlitten mit der Sonde grundsätzlich fast über eine gesamte Zahnstangenlänge, und somit einer gesamten Transportbandbreite verfahren werden. Der Zahnstangenantrieb ist hier grundsätzlich besonders geeignet, weil unabhängig von den meisten Bauteilen, die Zahnstangenlänge variabel festgelegt werden kann. Mit wenig Aufwand und durch Anpassung von wenigen Bauteilen in der Baulänge, wie zum Beispiel der Zahnstange, lassen sich grundsätzlich passende Querläufer für jeden Tunneltyp entwerfen.

Der Antrieb kann mindestens einen ersten Motor umfassen, wobei der Antrieb mindestens einen ersten Motor umfasst ausgewählt aus der Gruppe bestehend aus: einem Servomotor; einem Schrittmotor. Auch weitere Arten von Motoren sind grundsätzlich denkbar. Die Begriffe "erster Motor" und "zweiter Motor" sind als reine Beschreibungen anzusehen, ohne eine Reihenfolge oder Rangfolge anzugeben und beispielsweise ohne die Möglichkeit auszuschließen, dass mehrere Arten von ersten Motoren beziehungsweise zweiten Motoren oder jeweils genau eine Art vorgesehen sein können. Weiterhin können zusätzliche Motoren, beispielsweise ein oder mehrere dritte Motoren, vorhanden sein.

Der Begriff "Motor", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine beliebige Kraftmaschine beziehen, welche eingerichtet ist, mechanische Arbeit zu verrichten, insbesondere indem eine Energieform, wie thermische, chemische, hydraulische, pneumatische oder elektrische Energie, in Bewegungsenergie umgewandelt wird.

Der Servomotor kann insbesondere ein Synchron-Servomotor sein. Der Servomotor kann zusammen mit einem Servoregler einen Servoantrieb bilden. Der Synchron-Servomotor kann insbesondere einen Stator mit Kupferdrahtwicklungen und einen Rotor mit Permanentmagneten umfassen. Die Permanentmagneten können eingerichtet sein, um den Rotor ein konstantes Magnetfeld zu bilden. Der Servoregler kann eingerichtet sein, den Stator mit Wechselstrom zu bestromen, wodurch ein zweites magnetisches Feld, insbesondere ein Drehfeld, entsteht. Das Drehfeld kann eine Kraftwirkung auf das Magnetfeld des Rotors ausüben, welcher sich synchron zum Drehfeld dreht. Durch eine Änderung einer Stromfrequenz kann sich die Drehzahl des Drehfeldes und damit des Rotors ändern. Durch eine Höhe des Stroms kann eine Höhe der elektromagnetischen Kraft und damit ein Rotordrehmoment bestimmt werden. Durch eine übergeordnete Steuerungseinheit kann eine Drehzahl und eine Zielposition des Motors über den Servoregler an den Servomotor weitergegeben werden. Der Motor kann eingerichtet sein, aktuelle Ist-Werte, Drehzahlen und Positionen, an den Servoregler zurückzugeben. Bei Abweichungen können die Drehzahl und der Strom über die Steuerungseinheit nachgeregelt werden. Servomotoren haben grundsätzlich eine hohe Dynamik, eine hohe Positioniergenauigkeit und eine hohe Überlastfähigkeit innerhalb eines großen Drehzahlbereichs. Weitere Merkmale des Servomotors sind eine hohe Drehzahlgenauigkeit, eine kurze Hochlaufzeit, eine kurze Drehmoment-Anregelzeit, ein hohes Stillstandsmoment und ein kleines Massenträgheitsmoment. Außerdem haben sie grundsätzlich eine kompakte Bauweise und weisen im Verhältnis zur Leistung grundsätzlich ein niedriges Gewicht auf.

Der Schrittmotor kann insbesondere ausgewählt sein aus der Gruppe bestehend aus: einem permanenterregten Schrittmotor, einem Reluktanzschrittmotor; einem Hybridschrittmotor. Vorzugsweise kann der Schrittmotor ein Hybridschrittmotor sein. Der Hybridschrittmotor kann grundsätzlich Vorteile des permanenterregten Schrittmotors und des Reluktanzschrittmotors vereinen. Der Hybridschrittmotor kann insbesondere eingerichtet sein, sehr kleine Schrittwinkel zu erzielen. Der Schrittmotor, insbesondere der Hybridschrittmotor, kann mindestens einen Rotor, insbesondere mindestens einen Permanentmagneten, insbesondere mindestens einen zylinderförmigen Permanentmagneten mit axialer Polausrichtung, umfassen. Weiterhin kann der Schrittmotor, insbesondere der Hybridschrittmotor, mindestens ein Statorfeld, insbesondere ein feststehendes Statorfeld, mit einer Vielzahl an Statorspulen, beispielsweise acht Statorspulen, aufweisen. Der Schrittmotor, insbesondere der Hybridschrittmotor, kann eingerichtet sein, den Rotor um einen bestimmten Winkel bzw. um einen bestimmten Schritt durch abwechselnd angesteuerte Statorspulen zu drehen.

Um den Permanentmagneten können mindestens zwei, hintereinander geschobene, mit dem Rotor festverbundene Rotorschalen mit einer Vielzahl an Zähnen, beispielsweise jeweils 50 Zähnen, welche um eine Zahnbreite gegeneinander verdreht sind, angeordnet sein. Die Rotorschalen können eingerichtet sein, eine Magnetisierung des Permanentmagneten anzunehmen. Das Statorfeld kann ebenfalls eine Vielzahl an Zähnen aufweisen, beispielsweise 48 Zähne. Die Statorspulen des Statorfeldes können pro Schritt nacheinander um jeweils 45 Grad versetzt bestromt werden, wodurch ein wechselndes elektromagnetisches Feld erzeugt wird. Die magnetisierten Rotorschalen können sich mit ihren Zähnen zum elektromagnetischen Feld der Statorspulen ausrichten. Durch die unterschiedliche Zähnezahl des Statorfeldes und der Rotorschalen dreht sich der Rotor, beispielsweise um 1,8° pro Schritt.

Der erste Motor kann insbesondere einen ersten Motortreiber umfassen. Die Begriffe "erster Motortreiber" und "zweiter Motortreiber" sind als reine Beschreibungen anzusehen, ohne eine Reihenfolge oder Rangfolge anzugeben und beispielsweise ohne die Möglichkeit auszuschließen, dass mehrere Arten von ersten Motortreibern beziehungsweise zweiten Motortreibern oder jeweils genau eine Art vorgesehen sein können. Weiterhin können zusätzliche Motortreiber, beispielsweise ein oder mehrere dritte Motortreiber, vorhanden sein. Der erste Motortreiber kann eingerichtet sein, Signale, insbesondere Signale der Steuerung, an den ersten Motor zu übermitteln und/oder den ersten Motor mit einer Spannung zu versorgen. Hierzu kann der erste Motortreiber beispielsweise an ein Netzteil angeschlossen sein. Das Netzteil kann eingerichtet sein, eine Spannung auf eine vom ersten Motor benötigte Spannung umzuwandeln.

Insbesondere kann der Antrieb der Linearführung mindestens einen Schrittmotor NEMA 17 (Stepperonline, China) und einen zugehörigen Schrittmotortreiber umfassen. Der Schrittmotor NEMA 17 kann eine Hersteller-Teilnummer 17HS24-2104S, ein Haltemoment M1 von 0,65 Nm, einen Schrittwinkel von 1,8°, ein Flanschmaß von 42 mm, eine Baulänge von 60 mm und ein Gewicht von 500 g aufweisen. Der Schrittmotor NEMA 17 hat grundsätzlich für seine Baugröße ein geringes Gewicht und eine große Auswahl an verschiedenen Haltemomenten. Sollte der gewählte Haltemoment nicht ausreichen, um den Führungsschlitten mit dem Sondenhalter und der Sonde zu bewegen, besteht grundsätzlich die Möglichkeit, den Schrittmotor durch einen stärkeren Schrittmotor auszutauschen, ohne an der Konstruktion des Querläufers etwas ändern zu müssen. Auch kann der Schrittmotor Nema17 trotz seiner geringen Baugröße geeignet sein, eine erforderliche Leistung zum Antrieb des Linearführung des Querläufers aufzubringen. Auch kann grundsätzlich ein Schrittmotor mit einem kleineren Haltemoment eingesetzt werden. Dies kann grundsätzlich eine Gewichtsersparnis für den Querläufer bedeuten. Mit einem Flanschmaß von 42 mm sind NEMA 17-Schrittmotoren grundsätzlich nur unwesentlich breiter als ein Durchmesser der Sonde, welcher beispielweise 36 mm betragen kann. Dies bedeutet, dass die Sonde grundsätzlich beim Erreichen von äußeren Positionen nur minimal, beispielsweise um jeweils 3 mm vom Motor eingeschränkt wird. Der Motor kann außerdem grundsätzlich durch sein Flanschmaß eine maximale Länge des Führungsschlittens vorgeben. Die Antriebswelle des Motors kann eine gefräste Abflachung aufweisen. Die Abflachung kann grundsätzlich eine Montage des Stirnzahnrades an der Motorwelle durch eine kraftschlüssige Welle-Nabe-Verbindung erleichtern. Weiterhin kann der zweite Motor ein Schrittmotor vom Typ OMC Stepperonline 17HS24-2104S mit einem Schrittgeber vom Typ OMC Stepperonline DM556N sein.

Hybridschrittmotoren weisen grundsätzlich selbst im Stillstand ein hohes Haltemoment ohne dabei zu überhitzen auf und weisen grundsätzlich eine kleine Baugröße auf. Für einfache Positionieraufgaben mit einem Hybridschrittmotor wird grundsätzlich kein Wegmesssystem benötigt, da die Schritte zählbar sind. Jedoch ist grundsätzlich keine Überwachung der Schritte vorgesehen und somit gibt es grundsätzlich auch keine Positionsrückmeldung. Dieses System eines offenen Regelkreises wird auch Open- Loop-System genannt. Dies hat grundsätzlich den Nachteil, dass bspw. bei äußeren Störeinflüssen oder Überlast Schritte übersprungen werden können. Dies würde grundsätzlich für den Verfahrweg der Sonde bedeuten, dass eine Genauigkeit des Verfahrweges abnimmt. Eine Möglichkeit für eine Positionsrückmeldung ist, einen Schrittmotor mit Encoder einzusetzen. Der Encoder kann eingerichtet sein, die Anzahl der Schritte zu zählen und zu überwachen. Bei einem solchen geschlossenen Regelkreis, auch Closed-Loop-System genannt, wird grundsätzlich zusätzlich eine passende Endstufe zwischen Steuerung und Encoder geschaltet, welche die vom Encoder kommenden Informationen verarbeitet und an die Steuerung übermittelt. So kann die Steuerung grundsätzlich die aktuelle Position bestimmen, speichern und bei Bedarf nachregeln. Allerdings erhöht sich durch einen Encoder grundsätzlich die Baulänge und das Gewicht des Schrittmotors. Grundsätzlich können Hybridschrittmotoren nur bis zu einer bestimmten Drehzahl ein volles Drehmoment aufbringen. Wird die Drehzahl erhöht, nimmt grundsätzlich das Drehmoment ab einer bestimmten Drehzahl ab. Der Drehmomentabfall kann aus einer Kennlinie des Motors entnommen werden. Wird das Drehmoment überschritten, bleibt der Motor grundsätzlich stehen.

Der Schrittmotor kann für eine Beschleunigung des Antriebs, insbesondere des Linearantriebs, einige Vorteile aufweisen. Die maximale Drehzahl ist bei Schrittmotoren zwar grundsätzlich geringer als bei Servomotoren, und auch das übertragbare Drehmoment nimmt grundsätzlich bei Schrittmotoren mit zunehmender Drehzahl ab, allerdings kann eine erwartete, benötigte Drehzahl voraussichtlich kleiner 100 Umdrehungen pro Minute betragen. Die kürzere Baulänge und das niedrigere Gewicht des Schrittmotors sind grundsätzlich Vorteile, die bei einer Konstruktion helfen, den Querläufer, insbesondere die Querachse, kleiner und leichter zu konstruieren. Eine Programmierung und Steuerung eines Schrittmotors lässt sich grundsätzlich einfacher als die Programmierung und Steuerung eines Servomotors durchführen.

Durch die Schritte von 1,8° können sich 200 Schritte für eine vollständige Umdrehung des Motors ergeben. Eine Strecke kann abgemessen und so sehr gut programmiert werden, ohne dass grundsätzlich eine zusätzliche Überwachung durch bspw. Endtaster oder Encoder notwendig ist. Auch in Bezug auf die Kosten, weist der Schrittmotor grundsätzlich gute Konditionen auf. Dies hilft die Gesamtkosten der Partikelzählvorrichtung gering zu halten, da die Kosten eines Schrittmotors grundsätzlich weitaus geringer sind, als die eines Servomotors.

Ein weiterer Vorteil des Schrittmotors ist grundsätzlich eine genormte Flanschgröße. Das Anschlussmaß bei Schrittmotoren ist grundsätzlich gemäß National Electrical Manufacturers Association (NEMA)-Norm geregelt und somit je nach Baugröße gleich. Anhand der Bezeichnung der Schrittmotoren lassen sich grundsätzlich deren Flanschgröße bestimmen, beispielsweise hat ein Schrittmotor mit der Bezeichnung NEMA 17 eine Flanschgröße von 42 mm oder ein NEMA 23-Schrittmotor eine Flanschgröße von 57 mm. Diese Normung hat grundsätzlich den Vorteil, dass sich Motoren unterschiedlicher Hersteller meist ohne konstruktive Änderungen austauschen lassen. Auch können Schrittmotoren mit gleicher Flanschgröße, jedoch unterschiedlichem Drehmoment ausgetauscht werden.

Eine Aufgabe des Querläufers kann eine Führung der Sonde für die Partikelmessung quer, insbesondere orthogonal, zur Transportrichtung des Transportbandes sein. Die Sonde kann auf dem Führungsschlitten beziehungsweise auf dem Befestigungswinkel befestigt sein. Der Sondenhalter kann eingerichtet sein, zusätzlich durch die Zahnstange geführt zu werden. Der Sondenhalter kann insbesondere aus POM hergestellt sein. Das Stirnzahnrad kann, wie oben ausgeführt, ebenfalls aus POM hergestellt sein. Dadurch kann eine Gleitreibung zwischen der Zahnstange und dem Sondenhalter minimiert werden. Weiterhin kann eine Gewichtsersparnis erzielt werden. Der Sondenhalter und der Befestigungswinkel können insbesondere mittels einer Schraubverbindung miteinander verbunden sein. Der Sondenhalter kann die Nut aufweisen, in welche die Sonde eingesetzt und mit der Klemmplatte verspannt werden kann. Der Sondenhalter kann, insbesondere aus Sicherheitsgründen, derart eingerichtet sein, dass der Sondenhalter das Stirnzahnrad umgibt, insbesondere vollständig. Dadurch kann bei der Bewegung des Stirnzahnrades ein Einklemmen von weiteren Elementen zwischen dem Stirnzahnrad und der Zahnstange vermieden werden.

Für eine Konstruktion des Querläufers können Tunneltypen des Sterilisationstunnels analysiert werden und für den Scanner, insbesondere für den Querläufer, wichtige Maße aufgenommen werden. Die wichtigsten Maße sind grundsätzlich eine maximale Höhe des Sterilisationstunnels und eine Breite des Transportbands. Der Sterilisationstunnel kann insbesondere eine Transportbandbreite von 600 mm bis 800 mm aufweisen. Weiterhin kann der Sterilisationstunnel eine maximale Höhe von 160 mm bis 230 mm aufweisen. Weiterhin können sich in dem Sterilisationstunnel zwischen 1 und 5 Schwebstofffilter befinden. Der Schwebstofffilter kann eine Filterlänge von 250 mm bis 580 mm und die Filterbreite von 600 mm bis 720 mm aufweisen. Die aufgeführten Maße können insbesondere von vorhandenen technischen Zeichnungen der Sterilisationstunnel entnommen werden und/oder der Sterilisationstunnel kann vor Ort vermessen werden. Aus diesen Maßen lassen sich grundsätzlich die maximale Höhe und die maximale Breite des Querläufers des Scanners ableiten. Die maximale Höhe, insbesondere Durchfahrtshöhe, von 160 mm gibt grundsätzlich eine Begrenzung in einer Bauhöhe vor. Zudem kann die maximale Bauhöhe durch im Sterilisationstunnel befindliche Trennschotts zwischen unterschiedlichen Zonen begrenzt sein. Dies kann ebenfalls eine möglichst flache Bauform der Partikelzählvorrichtung erfordern. Es ist jedoch grundsätzlich ein Ziel, eine Höhe der Partikelzählvorrichtung so niedrig wie möglich zu gestalten, um eine Handhabung am Sterilisationstunnel und die Einbringung der Partikelzählvorrichtung auf das Transportband zu erleichtern. Der Querläufer kann grundsätzlich auf die Breite des Transportbands angepasst werden. Aufgrund unterschiedlicher Breiten der Schwebstofffilter kann der Scanner modular aufgebaut sein. Der Querläufer kann derart konstruiert sein, dass der Querläufer für eine Tunnelbreite des Sterilisationstunnels anpassbar ist. Insbesondere können eine Länge der Zahnstange, eine Länge der Führungsschiene und eine Länge der Grundplatte angepasst werden. Die restlichen Bauteile der Partikelzählvorrichtung können unabhängig von dem Tunneltyp des Sterilisationstunnels sein. Beispielsweise kann eine Höhe des Querläufers 80 mm und ein Gesamtgewicht des Querläufers 1870 g betragen. Zudem können Anschlüsse von Komponenten des Querläufers Phoenix-Stecckklemmen aufweisen. Dies kann zu einer weiteren Reduzierung einer Gesamthöhe der Partikelzählvorrichtung, insbesondere um bis zu 10 cm, beitragen.

Wie oben ausgeführt, umfasst der Scanner das Fahrgestell. Der Begriff "Fahrgestell", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen beliebigen Teil einer Vorrichtung beziehen, welcher eingerichtet ist, weitere Komponenten der Vorrichtung zu tragen. Das Fahrgestell kann daher als tragendes Teil der Vorrichtung eingerichtet sein. Das Fahrgestell kann insbesondere Elemente aufweisen, welche eine Bewegung des Fahrgestells auf einer Oberfläche ermöglichen. Die Elemente können insbesondere ein oder mehrere Räder, ein oder mehrere Radaufhängungen, mindestens einen Antrieb und/oder mindestens einen Motor umfassen. Das Fahrgestell kann daher auch als Chassis bezeichnet werden.

Das Fahrgestell kann einen Rahmen aufweisen. Der Rahmen kann auch als Grundgestell bezeichnet werden. Der Rahmen kann insbesondere aus einem Blech, insbesondere aus einem Blech aus einem austenitischen nichtrostendem Stahl, hergestellt sein. Das Blech kann insbesondere eine Stärke von 1 mm bis 5 mm, vorzugsweise von 1,5 mm bis 2,5 mm und besonders bevorzugt von 2 mm aufweisen. Der austenitische nichtrostende Stahl lässt sich grundsätzlich aufgrund seines Gefüges sehr gut kaltumformen, so dass das Blech gebogen werden kann. Durch einen Chromgehalt von >13,5% kann der austenitische nichtrostende Stahl außerdem eine gute Korrosionsbeständigkeit aufweisen und sich deshalb gut für einen Einsatz im Pharmaumfeld eignen. Durch gut gewählte Abkantungen kann eine hohe Steifigkeit des Bleches erreicht werden. Durch Verschweißen von Stoßkanten, kann eine Steifigkeit des Blechs zusätzlich erhöht werden. So kann ein verwindungssteifer und leichter Rahmen hergestellt werden, an welchem weitere Komponenten befestigt werden können. Alternativ oder zusätzlich kann der Rahmen zumindest teilweise aus Aluminium hergestellt sein. Dies kann zu einer Gewichtsreduzierung der Partikelzählvorrichtung beitragen. Wie oben ausgeführt, ist der Querläufer auf dem Fahrgestell befestigt. Insbesondere kann der Querläufer, insbesondere die Grundplatte des Querläufers, auf dem Rahmen befestigt sein. Insbesondere kann der Querläufer mittig auf dem Fahrgestell, insbesondere auf dem Rahmen, befestigt sein. Weiterhin kann der Querläufer bündig auf dem Fahrgestell, insbesondere auf dem Rahmen, befestigt sein. Insbesondere kann der Querläufer bündig an einer Hinterseite des Fahrgestells, insbesondere des Rahmens, befestigt sein. Aufgrund von geringen Auslaufflächen vor und nach dem Sterilisationstunnel, kann eine Baugröße somit minimiert werden und ein Partikelzählen kann selbst ohne Auslaufzonen realisiert werden.

Die Steuerung kann insbesondere mittig auf dem Fahrgestell angebracht, insbesondere installiert sein. Die Steuerung kann insbesondere auf einer Freifläche des Fahrgestells angebracht sein. Eine Länge der Freifläche kann insbesondere derart gewählt sein, dass die Steuerung und darüber hinaus Kabelkanäle, insbesondere zur Verdrahtung, angebracht sein können.

Wie oben ausgeführt, ist das Fahrgestell eingerichtet, die Linearführung in der Transportrichtung des Transportbands zu bewegen. Beispielsweise kann das Fahrgestell ein Raupenfahrwerk aufweisen, welches eingerichtet ist, die Linearführung in der Transportrichtung des Transportbands zu bewegen. Vorzugsweise kann das Fahrgestell jedoch mindestens zwei Räder, insbesondere mindestens zwei Antriebsräder, aufweisen, welche eingerichtet sind, die Linearführung in der Transportrichtung des Transportbands zu bewegen. Bei einem Antriebsrad kann es sich insbesondere um ein beliebiges Rad handeln, welches für eine selbstständige Fortbewegung einer Vorrichtung, an welcher die Antriebsräder angebracht sind, eingerichtet ist. Die Antriebsräder können insbesondere mittels eines Motors angetrieben werden, wie nachfolgend noch näher erläutert wird.

Wie oben ausgeführt, kann das Transportband des Sterilisationstunnels ein Drahtgittergeflecht aufweisen. Die Räder können, wie nachfolgend näher ausgeführt wird, insbesondere derart konstruiert sein, dass eine Kontaktfläche zwischen den Rädern und dem Drahtgittergeflecht des Transportbands erhöht wird. Insbesondere kann durch eine geeignete Materialauswahl und durch ein Eigengewicht der Partikelzählvorrichtung ausreichend Rollreibung aufgebracht werden, um eine schlupffreie Bewegung zu erreichen.

Da das Drahtgittergeflecht üblicherweise aus mindestens einem Metall hergestellt ist, kann bei einer Konstruktion und einer Materialauswahl der Räder grundsätzlich ein Metall-auf-Metall-Kontakt vermieden werden, da sonst, aufgrund einer grundsätzlich kleinen Kontaktfläche auf dem Drahtgittergeflecht, grundsätzlich nicht genügend Rollreibung zwischen den Rädern und dem Transportband entstehen kann und eine schlupffreie Bewegung grundsätzlich nicht gewährleistet werden kann. Ein ähnliches Verhalten wird angenommen, wenn die Räder aus einem Thermoplast oder einem Duroplast hergestellt sind. Auch hier kann grundsätzlich zwischen dem Material der Räder und dem Transportband nicht genügend Rollreibung entstehen, um eine zuverlässige, schlupffreie Bewegung zu realisieren.

Insbesondere können die Räder zumindest teilweise aus einem Elastomer hergestellt sein. Das Elastomer kann ausgewählt sein aus einer Gruppe bestehend aus: Silikon, Ethylen-Propylen- Dien-(Monomer)-Kautschuk (EPDM). Diese Materialien sind für einen Einsatz im Pharmaumfeld zugelassen Auch andere Elastomere sind jedoch grundsätzlich denkbar. Insbesondere können die Räder jeweils ein oder mehrere O-Ringe aufweisen, welche aus dem Elastomer hergestellt sind. Das Elastomer kann eingerichtet sein, eine Rollreibung zwischen den Rädern und dem Transportband zu erzeugen. Durch eine elastische Verformbarkeit und einen hohen Reibwert der Elastomere kann eine Kontaktfläche zwischen den O-Ringen und dem Transportband erhöht sein. Somit kann ein schlupffreier Betrieb möglich sein.

Die Räder können insbesondere derart ausgestaltet sein, dass die O-Ringe aus dem Elastomer daran befestigt werden können. Die Räder, insbesondere die Antriebsräder, können insbesondere aus Polyoxymethylene (POM) hergestellt sein. Insbesondere können die Räder ein oder mehrere Nuten aufweisen, welche für eine Aufnahme der O-Ringe eingerichtet sind. Ein Durchmesser der Räder kann so gewählt werden, dass eine geringe Bodenfreiheit zwischen dem Rahmen und dem Transportband erreicht wird. So kann eine spätere Gesamthöhe des Scanners klein gehalten werden. Die Räder, insbesondere die Antriebsräder, können jeweils mehrere O-Ringe aufweisen, insbesondere mindestens zwei, vorzugsweise mindestens drei, vorzugsweise mindestens vier O-Ringe, insbesondere um die Kontaktfläche zwischen den Rädern und dem Transportband zu erhöhen. Die O-Ringe können voneinander beabstandet auf mindestens einer Umfangsfläche der Antriebsräder angeordnet sein. Die O-Ringe können jeweils in Nuten der Umfangsfläche der Antriebsräder aufgenommen sein.

Die Antriebsräder können jeweils auf einer Radwelle befestigt sein. Eine Drehbewegung einer Radwelle auf die Antriebsräder kann formschlüssig mit Passfedern übertragen werden. So kann ein Durchdrehen der Räder verhindert werden. Zusätzlich können die Antriebsräder mit einem Gewindestift gesichert werden, insbesondere gegen ein Verschieben auf der Radwelle.

Die Partikelzählvorrichtung kann weiterhin mindestens einen zweiten Motor umfassen ausgewählt aus der Gruppe bestehend aus: einem Schrittmotor, einem Servomotor. Auch andere Arten von Motoren sind jedoch grundsätzlich denkbar. Der zweite Motor kann eingerichtet sein, mindestens eines der Antriebsräder anzutreiben. Hinsichtlich weiterer Details zu der Ausgestaltung des Schrittmotors und des Servomotors kann auf obige Beschreibung verwiesen werden.

Vorzugsweise kann der zweite Motor ein Schrittmotor sein. Insbesondere kann es sich bei dem zweiten Motor um einen Motor mit einer Baugröße handeln, welche einer Baugröße des ersten Motors entspricht. Insbesondere kann es sich bei dem zweiten Motor um einen Motor handeln, welcher baugleich zu dem ersten Motor ist. Dadurch kann eine Anzahl an verschiedenen Komponenten der Partikelzählvorrichtung minimiert werden. Weiterhin kann nur eine Bauart von Schrittmotor als Ersatzteil auf Lager gelegt werden und eine Austauschbarkeit ist grundsätzlich gegeben.

Der zweite Motor kann insbesondere einen zweiten Motortreiber umfassen. Der zweite Motortreiber kann eingerichtet sein, Signale an den zweiten Motor zu übermitteln und/oder den zweiten Motor mit einer Spannung zu versorgen. Hierzu kann der zweite Motortreiber beispielsweise an ein Netzteil angeschlossen sein. Das Netzteil kann eingerichtet sein, eine Spannung auf eine vom zweiten Motor benötigte Spannung umzuwandeln, beispielswese kann das Netzteil eingerichtet sein, eine Spannung von 230 V auf 24 V umzuwandeln. Insbesondere kann die Partikelzählvorrichtung ein Netzteil umfassen, welches eingerichtet ist, den ersten Motor, den zweiten Motor, den ersten Motortreiber und den zweiten Motortreiber zu versorgen. Alternativ können auch zwei Netzteile vorgesehen sein, welche jeweils den ersten Motor und den ersten Motortreiber bzw. den zweiten Motor und den zweiten Motortreiber versorgen.

Insbesondere kann der zweite Motor ein Schrittmotor NEMA 17 (Stepperonline, China) mit einem zugehörigen Schrittmotortreiber sein. Der Schrittmotor Nema17 kann trotz seiner geringen Baugröße geeignet sein, eine erforderliche Leistung zum Antrieb der Bewegung des Fahrgestells aufzubringen. Für weitere Details wird auf obige Beschreibung verwiesen. Weiterhin kann der zweite Motor ein Schrittmotor vom Typ OMC Stepperonline 17HS24-2104S mit einem Schrittgeber vom Typ OMC Stepperonline DM556N sein. Das Fahrgestell kann insbesondere eine Antriebsachse aufweisen. Der Schrittmotor kann quer, insbesondere um 90°, versetzt zu der Antriebsachse am Grundgestell befestigt sein. Das Fahrgestell kann weiterhin mehrere Kegelräder aufweisen. Um eine Drehbewegung des Schrittmotors auf die Antriebsachse zu übertragen, können Kegelräder mit einer Übersetzung 2:1 gewählt werden. Ein erstes Kegelrad kann beispielsweise eine Zähnezahl z von 15 und ein zweites Kegelrad kann eine Zähnezahl z von 30 aufweisen. Das erste Kegelrad und das zweite Kegelrad können jeweils aus POM hergestellt sein und ein Modul $m$ von 1 aufweisen.

Das erste Kegelrad kann eingerichtet sein, von dem zweiten Motor angetrieben zu werden und eine Drehbewegung über das zweite Kegelrad auf eine Antriebsachse zu übertragen. Durch das Übersetzungsverhältnis kann eine Drehzahl $n_1$ des zweiten Motors an der Antriebsachse halbiert werden und das übertragene Drehmoment $M_1$ verdoppelt werden. Daraus resultierend kann grundsätzlich ein Schrittmotor mit kleinem Haltemoment verwendet werden. $z_1$ entspricht einer Zähnezahl des ersten Kegelrads und $z_2$ einer Zähnezahl des zweiten Kegelrads.

$$n_2 = \frac{n_1 * z_1}{z_2} \tag{15}$$

$$n_2 = \frac{n_1 * 15}{30} = n_1 * 0,5 \tag{16}$$

$$M_2 = M_1 * \frac{z_2}{z_1} \tag{17}$$

$$M_2 = 0,65\ Nm * \frac{30}{15} = 1,3\ Nm \tag{18}$$

Zur Übertragung des Drehmoments und der Drehzahl vom zweiten Motor auf die Welle des ersten Kegelrads kann eine drehstarre, aber winkel- und quer-nachgiebige Ausgleichskupplung verwendet werden. Hierdurch können grundsätzlich Toleranzen oder Fluchtungsfehler in der Konstruktion ausgeglichen werden. Eine verwendete Ausgleichskupplung kann grundsätzlich spielfrei und drehsteif sein und sowohl radialen als auch axialen Winkelversatz ausgleichen. Wellen der Zahnräder können durch einen Halter in einer passenden Position gehalten werden. In dem Halter können Rillenkugellager eingepresst sein, welche die Welle des ersten Kegelrads und die Radwelle lagern und einen leichtgängigen Lauf gewährleisten. Rillenkugellager haben grundsätzlich den Vorteil, dass sie beim Anlaufen kein erhöhtes Reibmoment haben. Auch weisen sie bei niedrigen Drehzahlen grundsätzlich einen geringen Verschleiß auf und sind wartungsfrei. Das erste Kegelrad kann kraftschlüssig mit einem Gewindestift auf der Welle befestigt werden. Das zweite Kegelrad kann ebenfalls mit einem Gewindestift auf der Radwelle befestigt werden kann und kann zusätzlich mit einem Stellring gegen unabsichtliches Verschieben auf der Welle gesichert werden.

Weiterhin können die Räder ein oder mehrere, insbesondere zwei, Hinterräder umfassen. Die Hinterräder können antriebsfrei ausgestaltet sein. Die Hinterräder können insbesondere einen leichtgängigen Lauf aufweisen. Für weitere Details der Ausgestaltung der Hinterräder wird auf obige Beschreibung verwiesen. Die Hinterräder können aus POM hergestellt sein. Weiterhin können die Hinterräder Nuten aufweisen, welche eingerichtet sein, O-Ringe aufzunehmen. Die O-Ringe können aus mindestens einem Elastomer hergestellt sein. Ein Durchmesser der Hinterräder kann so gewählt sein, dass die Hinterräder keine Behinderung für die Bewegung des Querläufers darstellen. In die Hinterräder können, insbesondere für einen leichtgängigen Lauf, Kugellager eingepresst sein. Die Hinterräder können einen Sicherungsring aufweisen, welches eingerichtet ist, das Kugellager gegen ein unbeabsichtigtes Lösen zu sichern. Die Hinterräder können jeweils auf einer Welle geschoben und mit einer selbstsichernden Mutter befestigt sein. Die selbstsichernde Mutter kann eingerichtet sein, ein Lösen der selbstsichernden Mutter bei einer Drehbewegung des Hinterrads zu vermeiden. Die Wellen der Hinterräder können weiterhin jeweils mit selbstsichernden Muttern am Fahrgestell befestigt, insbesondere verschraubt, sein.

Das Fahrgestell kann so konstruiert sein, dass es in sämtlichen Typen von Sterilisationstunneln eingesetzt werden kann. Durch eine kompakte Baugröße lässt sich das Fahrgestell einfach handhaben. Der Rahmen kann Schweißbolzen, insbesondere an einem Heck angeordnete Schweißbolzen, aufweisen, welche insbesondere für eine Montage des Querläufers eingerichtet sein können. Mit Rändelmuttern kann der Querläufer lösbar mit dem Fahrgestell verbunden sein. Ein leichtes Austauschen des Querläufers kann möglich sein. Eine Bauhöhe des Fahrgestells kann insbesondere so gewählt sein, dass diese eine Gesamthöhe der Partikelzählvorrichtung von 160 mm nicht überschreitet. Weiterhin kann die Bauhöhe des Fahrgestells so gering wie möglich gehalten sein. Das Fahrgestell mit den oben ausgeführten Komponenten kann beispielsweise eine Gesamthöhe von 79 mm und ein Gesamtgewicht von 3000 g aufweisen. Das Fahrgestell kann beispielsweise eine Breite von 300 mm aufweisen. Dies kann eine Handhabung und ein Einbringen der Partikelzählvorrichtung in den Sterilisationstunnel erleichtern.

Wie oben ausgeführt, umfasst der Scanner weiterhin die mindestens eine Steuerung, welche eingerichtet ist, die Bewegung des Scanners zu steuern. Bei der Bewegung kann es sich insbesondere um eine Bewegung des Fahrgestells in die Transportrichtung des Transportbands handeln. Weiterhin kann es sich bei der Bewegung um die Führung des Sondenhalters quer zu der Transportrichtung des Transportbands handeln. Der Begriff "Steuerung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine einteilige oder mehrteilige Vorrichtung der Partikelzählvorrichtung beziehen, welche eingerichtet ist, um einen Betrieb der Partikelzählvorrichtung vollständig oder teilweise zu steuern und/oder zu regeln. Insbesondere kann die Steuerung eine speicherprogrammierbare Steuerung (SPS) umfassen. Unter einer "speicherprogrammierbaren Steuerung (SPS)" ist dabei im Rahmen der vorliegenden Erfindung grundsätzlich eine beliebige Vorrichtung zu verstehen, welche für die Steuerung oder Regelung einer Maschine oder Anlage eingesetzt und auf digitaler Basis programmiert ist. Insbesondere kann die Steuerung eingerichtet sein, um den Motor des Antriebs für die Führung des Sondenhalters quer, insbesondere im Wesentlichen senkrecht, zu der Transportrichtung des Transportbands und/oder den Motor des Antriebs für eine Bewegung des Fahrgestells entlang der Transportrichtung des Transportbandes zu steuern und/oder zu regeln. Die Steuerung kann insbesondere mindestens eine Datenverarbeitungsvorrichtung umfassen, beispielsweise mindestens einen Prozessor. Dementsprechend kann die Steuerung teilweise durch Hardware realisiert werden oder/auch, alternativ oder zusätzlich, vollständig oder teilweise durch Software. Weiterhin kann die Steuerung mindestens einen flüchtigen und/oder nicht flüchtigen Datenspeicher umfassen. Die Steuerung kann insbesondere eingerichtet sein, um mindestens einen der Antriebe anzusteuern. Unter einer "Ansteuerung eines Antriebs" ist dabei im Rahmen der vorliegenden Erfindung grundsätzlich eine Art des Betriebs des Antriebs zu verstehen, insbesondere ein Starten und/oder ein Stoppen einer Bewegung oder von Schritten sowie insbesondere eine Änderung einer Geschwindigkeit der Bewegung. Die Steuerung kann insbesondere eingerichtet sein, um über einen Betrieb der Partikelzählvorrichtung hinweg die Antriebe derart anzusteuern, dass wiederholt vorgebbare Bewegungen des Fahrgestells bzw. Führungen des Sondenhalters mit der Linearführung durchgeführt werden. Die Steuerung kann insbesondere eingerichtet sein, Signale an den ersten Motortreiber des ersten Motors oder Signale an den zweiten Motortreiber des zweiten Motors zu übertragen. Die Steuerung kann insbesondere programmtechnisch eingerichtet sein, beispielsweise um das Verfahren zur Partikelzählung, welches nachfolgend noch näher ausgeführt wird, zu steuern. Die Steuerung kann weiterhin eingerichtet sein, Daten der Antriebe zu protokollieren. Das Protokollieren kann insbesondere ein Speichern oder ein Aufzeichnen der Daten umfassen. Insbesondere kann die Steuerung eine TIA-SPS S7-1200 umfassen. Die TIA-SPS S7-1200 kann trotz einer geringen Baugröße und einer begrenzten Kanalzahl eine ausreichend hohe Leistung für die Steuerung aufbringen. Weiterhin kann die Steuerung einen Siemens SPS S7-1211C DC/DC/DC, und insbesondere ein 8" Touchpanel für eine Bedienung, umfassen. Die Siemens SPS S7-1211C DC/DC/DC kann insbesondere eine kompakte Bauform aufweisen.

Weiterhin kann die Steuerung ein Board mit Mikrokontroller (Arduino) umfassen, welches insbesondere über eine Schnittstelle, insbesondere über eine USB-Schnittstelle mit einem Computer verbunden sein kann. Ein Programm zur Steuerung der Bewegung des Fahrgestells und/oder der Führung des Sondenhalters kann auf dem Board geladen sein. Das Programm kann insbesondere Schritte, eine Geschwindigkeit und/oder eine Drehrichtung der Motoren, insbesondere der Schrittmotoren, vorgeben.

Weiterhin kann die Partikelzählvorrichtung mindestens einen y-Positionssensor zur Bestimmung einer Position der Sonde in einer Dimension in Transportrichtung auf dem Transportband aufweisen. Weiterhin kann die Partikelzählvorrichtung mindestens einen x-Positionssensor zur Bestimmung einer Position der Sonde in einer Dimension quer zur Transportrichtung, insbesondere im Wesentlichen senkrecht zur Transportrichtung, auf dem Transportband aufweisen. Der Begriff "Positionssensor" bezeichnet grundsätzlich einen beliebigen Sensor, welcher eingerichtet ist, einen Abstand zwischen einem Objekt und einem Bezugspunkt und/oder Längenänderungen zu messen. Der Positionssensor kann insbesondere eingerichtet sein, eine Änderung eines Weges in ein Einheitssignal umzuwandeln oder an ein Steuergerät zu übermitteln. Der y-Positionssensor kann mit dem Fahrgestell, insbesondere dem Antrieb des Fahrgestells, verbunden sein. Das Fahrgestell kann, wie oben ausgeführt, den Schrittmotor aufweisen und der y-Positionssensor kann einen Inkrementalgeber des Schrittmotors umfassen. Der x-Positionssensor kann mit der Linearführung, insbesondere dem Antrieb der Linearführung, verbunden sein. Die Linearführung kann, wie oben ausgeführt, den Schrittmotor aufweisen und der x-Positionssensor kann einen Inkrementalgeber des Schrittmotors umfassen. Die Partikelzählvorrichtung, insbesondere die Steuerung, kann eingerichtet sein, Impulse des Schrittmotors, insbesondere des Schrittmotortreibers, zu zählen, insbesondere mittels eines Vorwärts-Rückwärts-Zählers. Der Vorwärts-Rückwärts-Zähler kann insbesondere Teil der Steuerung sein. Damit können eventuell messwertauffällige Positionen während der Messung kartesisch zwischengespeichert und bei einer nachfolgenden genaueren Überprüfung im Handbetrieb angefahren werden, wie nachfolgend näher ausgeführt wird.

Insbesondere kann die Partikelzählvorrichtung, insbesondere die Steuerung, eingerichtet sein, mittels mindestens eines ersten Vorwärts-Rückwärts-Zählers erste Impulse des ersten Motors, insbesondere des ersten Schrittmotortreibers, zu zählen. Weiterhin kann die Partikelzählvorrichtung, insbesondere die Steuerung, eingerichtet sein, mittels mindestens eines zweiten Vorwärts-Rückwärts-Zählers zweite Impulse des zweiten Motors, insbesondere des zweiten Schrittmotortreibers, zu zählen. Insbesondere kann die Partikelzählvorrichtung, insbesondere die Steuerung, eingerichtet sein, mittels der ersten Impulse des ersten Motors eine Position der Sonde auf der Linearführung und mittels der zweiten Impulse des zweiten Motors eine Position des Fahrgestells auf dem Transportband zu ermitteln. Darüber hinaus kann die Partikelzählvorrichtung, insbesondere die Steuerung, mindestens einen weiteren Vorwärts-Rückwärts-Zähler, welcher für eine Zählung von Impulsen des zweiten Motors, insbesondere des zweiten Schrittmotortreibers, eingerichtet ist, umfassen. Der weitere Vorwärts-Rückwärts-Zähler kann daher insbesondere für eine Ermittlung eines Bahnabstandes bei einer Bewegung in die Transportrichtung eingerichtet sein. Die Partikelzählvorrichtung, insbesondere die Steuerung, kann eingerichtet sein, nach einer schrittweisen Bewegung des Fahrgestells, insbesondere nach einem Erreichen einer nächsten Messbahn, den weiteren Vorwärts-Rückwärts-Zähler auf Null zurückzusetzen. Weiterhin kann die Linearführung einen ersten Endanschlag und einen zweiten Endanschlag aufweisen und die Steuerung kann eingerichtet sein, den ersten Vorwärts-Rückwärts-Zähler auf Null zurückzusetzen, wenn sich die Sonde am ersten Endanschlag befindet. Insbesondere kann es sich bei dem ersten Endanschlag um einen linken Endanschlag handeln.

Insbesondere da der erste Vorwärts-Rückwärts-Zähler, der zweite weitere Vorwärts-Rückwärts-Zähler und der weitere Vorwärts-Rückwärts-Zähler jeweils eingerichtet sind, Impulse des ersten Schrittmotortreibers bzw. des zweiten Schrittmotortreibers zu zählen, kann die Steuerung eingerichtet sein, mittels der Impulse des ersten Schrittmotortreibers bzw. des zweiten Schrittmotortreibers metrische Angaben für eine Darstellung von Koordinaten zu berechnen.

Für eine Bestimmung einer Position des Sondenhalters quer zu der Transportrichtung ergibt sich grundsätzlich:

$$Impuls\ pro\ Umdrehung = \pi * Durchmesser_{Zahnrad} \tag{19}$$

Somit kann ein Verhältnis zwischen Impulsen und kartesischen Positionen angegeben werden:

$$\frac{x_{Position;karthesisch}}{x_{Position;Impuls}} = \frac{\pi * Durchmesser_{Zahnrad}}{Impuls\ pro\ Umdrehung} \tag{20}$$

Durch Umstellen kann nun eine kartesische Position ermittelt werden:

$$x_{Position;karthesisch} = \frac{\pi * Durchmesser_{Zahnrad}}{Impuls\ pro\ Umdrehung} * x_{Position;Impuls} \tag{21}$$

Der Impuls pro Umdrehung kann abhängig sein von einer Geschwindigkeit als auch von einer kleinstmöglich einstellbaren Zeitverzögerung der Impulsprogrammierung. Wenn man eine Mindestzeitverzögerung von 1 ms heranzieht und eine Geschwindigkeit von 5 cm/s, ergibt sich aus der Geometrie des antreibenden Zahnrads:

$$Impuls\ pro\ Umdrehung = \frac{\pi * Durchmesser_{Zahnrad}}{2 * Geschwindigkeit * Impulslänge} \tag{22}$$

$$Impuls\ pro\ Umdrehung = \frac{\pi * 19\ mm}{2 * 50\frac{mm}{s} * 0,001\ s} = 596,90 \tag{23}$$

Für die kartesische Position ergibt sich nun:

$$x_{Position;karthesisch} = \frac{x_{Position;Impuls}}{6.70126\frac{1}{mm}} \tag{24}$$

Da für einen Vortrieb, wie oben ausgeführt, ein Kegelradgetriebe, insbesondere ein einstufiges Kegelradgetriebe, verbaut werden kann, kann eine Übersetzung berücksichtigt werden:

$$n = \frac{Durchmesser_{Zahnrad}}{Durchmesser_{Ritzel}} \tag{25}$$

Bei einem Ritzel-Durchmesser von 11,96 mm kann sich ergeben:

$$Impuls\ pro\ Umdrehung_{Rad} = Impuls\ pro\ Umdrehung_{Motor} * n \tag{26}$$

$$Impuls\ pro\ Umdrehung_{Rad} = Impuls\ pro\ Umdrehung_{Motor} * 1,58824 = 635,294 \tag{27}$$

Für eine Bestimmung einer Position des Scanners in der Transportrichtung ergibt sich:

$$y_{Position;karthesisch} = \frac{\pi * Durchmesser_{Zahnrad}}{Impuls\ pro\ Umdrehung * n} * y_{Position;Impuls} \tag{28}$$

$$y_{Position;karthesisch} = \frac{y_{Position;Impuls}}{2,55975\frac{1}{mm}} \tag{29}$$

Weiterhin kann der Scanner einen oder mehrere Endlagenschalter aufweisen. Der Begriff "Endlagenschalter" bezeichnet grundsätzlich eine beliebige Vorrichtung, welche eingerichtet ist, zu erkennen, wenn ein bewegter Gegenstand eine bestimmte Position erreicht hat.

Insbesondere kann der Querläufer mindestens zwei Endlagenschalter, insbesondere mindestens zwei Rollenendschalter, aufweisen, welche für eine Bestimmung der Positionen der Endlagen der Sonde quer zu der Transportrichtung des Transportbands eingerichtet sind. Insbesondere kann der Querläufer mindestens zwei Schnappschalter, beispielsweise mindestens zwei Marquardt 1006.1501 Schnappschalter, aufweisen. Weiterhin kann das Fahrgestell mindestens einen Endlagenschalter, insbesondere mindestens einen Federstabendschalter, für eine Bestimmung mindestens einer Position mindestens einer Endlage der Partikelzählvorrichtung in der Transportrichtung aufweisen.

Weiterhin kann die Partikelzählvorrichtung mindestens eine stationäre Benutzerschnittstelle umfassen. Die Benutzerschnittstelle kann mit dem Scanner verbunden sein. Mittels der Benutzerschnittstelle kann eine Bewegung des Scanners steuerbar sein. Der Begriff "Schnittstelle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine grundsätzlich beliebig ausgestaltete Vorrichtung beziehen, welche eingerichtet ist, um mindestens eine Information zu empfangen und dann entsprechend optional vollständig oder teilweise zu verarbeiten und/oder weiterzuleiten, beispielsweise an mindestens eine Steuerung. Der Begriff "Benutzerschnittstelle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine beliebige Schnittstelle zur Eingabe von Befehlen und/oder zur Ausgabe von Informationen, und/oder auf eine drahtlose oder drahtgebundene Schnittstelle zum unidirektionalen oder bidirektionalen Austausch von Daten und/oder Befehlen zwischen einer Vorrichtung und mindestens einem Bediener der Vorrichtung beziehen. Die Benutzerschnittstelle kann eine Kommunikationsschnittstelle, insbesondere eine Datenschnittstelle sein, welche eingerichtet ist, Daten von einer anderen Vorrichtung und/oder von einem Benutzer zu empfangen und/oder Daten von der Benutzerschnittstelle an externe Vorrichtungen zu übermitteln. Die Benutzerschnittstelle kann mindestens eine elektronische Schnittstelle und/oder eine Mensch-Maschine-Schnittstelle aufweisen, wie beispielsweise eine Eingabe-/Ausgabe-Vorrichtung wie ein Display, insbesondere ein Touchdisplay, insbesondere ein 8''-Touchdisplay, und/oder eine Tastatur. Die Schnittstelle kann mindestens eine Datenverbindung aufweisen, beispielweise eine Bluetooth-Verbindung, eine NFC-Verbindung oder eine andere Verbindung. Die Benutzerschnittstelle kann mindestens ein Netzwerk aufweisen oder Teil eines Netzwerkes sein. Die Benutzerschnittstelle kann mindestens einen Internet-Port, mindestens einen USB-Port, mindestens ein Laufwerk oder ein Webinterface aufweisen.

Insbesondere kann die Benutzerschnittstelle eine grafische Benutzeroberfläche aufweisen, insbesondere eine grafische Benutzeroberfläche mit mindestens einem Touch Screen. Der Begriff "grafische Benutzeroberfläche", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Form einer Benutzerschnittstelle eines Computers beziehen, welche die Aufgabe hat, Anwendungssoftware auf einem Rechner mittels grafischer Symbole oder Steuerelemente, bedienbar zu machen. Dies kann beispielsweise mittels einer Maus als Steuergerät durchgeführt werden, mit der die grafischen Elemente bedient oder ausgewählt werden. Alternativ oder zusätzlich kann die Bedienung durch Berührung eines Sensorbildschirms, insbesondere eines Touch Screens, durchgeführt werden.

Insbesondere kann mittels der Benutzerschnittstelle mindestens ein Fahrweg und/oder mindestens eine Messposition für die Partikelzählung vorgebbar sein. Weiterhin kann der mindestens eine Fahrweg und/oder die mindestens eine Messposition in einem Handbetrieb oder in einem automatischen Betrieb vorgebbar sein. Weiterhin kann mindestens eine Geschwindigkeit für den mindestens einen Fahrweg vorgebbar sein, insbesondere in dem Handbetrieb, wie nachfolgend näher ausgeführt wird. Weiterhin kann die Benutzerschnittstelle mit dem Partikelzähler verbunden sein.

Die graphische Benutzeroberfläche kann insbesondere mindestens drei Bedienoberflächen, insbesondere mindestens drei einzelne Bedienoberflächen umfassen. Die Bedienoberflächen können jeweils durch ein Hauptmenü erreichbar sein. Das Hauptmenü kann auch als Grundbildschirm bezeichnet werden. Die graphische Benutzeroberfläche kann derart eingerichtet sein, dass von dem Hauptmenü aus die drei Bedienoberflächen, welche auch als untergeordnet Menüpunkte bezeichnet werden können, erreicht werden, insbesondere durch eine TIA-interne Funktion, welche als "AktiviereBild" bezeichnet werden kann. Eine erste Bedienoberfläche kann einem automatischen, insbesondere einem teil- oder vollautomatischen Betrieb entsprechen. Die erste Bedienoberfläche kann insbesondere ein Starten und ein Stoppen einer definierten, automatischen Fahrt des Fahrgestells und/oder der Sonde umfassen. Weiterhin kann die erste Bedienoberfläche eine Koordinatenanzeige und/oder eine Koordinatenspeicherung umfassen. Der teil- oder vollautomatische Betrieb kann auch als Parmessbetrieb bezeichnet werden. Eine zweite Bedienoberfläche kann einer manuellen Bedienung, insbesondere einem Handbetrieb entsprechen. Die zweite Bedienoberfläche kann insbesondere eine separate Ansteuerungsmöglichkeit von Bewegungsrichtungen, insbesondere Bewegungsrichtungen des Fahrgestells und/oder des Sondenhalters, bereitstellen. Weiterhin kann die zweite Benutzeroberfläche eine Fahrt in einer Ausgangsposition bereitstellen. Darüber hinaus kann die zweite Bedienoberfläche eine oder mehrere Servicefunktionen bereitstellen, welche beispielsweise einen Reset von Inkrementen oder eine Anzeige von digitalen Ausgängen umfassen können. Eine dritte Bedienoberfläche kann einer Oberfläche für Systemeinstellungen entsprechen. Die mindestens drei Bedienoberflächen können jeweils eine Darstellung eines Datums sowie einer Uhrzeit umfassen, welches insbesondere von einer steuerungsinternen Systemzeit abgegriffen werden kann. Weiterhin können die mindestens drei Bedienoberflächen jeweils eingerichtet sein, über eine Meldetextzeile auftretende Störungen anzuzeigen. Insbesondere kann die dritte Bedienoberfläche eingerichtet sein, eine Runtime zu beenden und insbesondere anschließend in einen Einstellungsbereich der Benutzerschnittstelle zu gelangen. Weiterhin kann die dritte Bedienoberfläche eine Benutzerverwaltung, insbesondere eine Verwaltung von Benutzerkonten umfassen. Die Benutzerschnittstelle kann insbesondere für eine Anlegung von Gruppen für Bediener sowie Administratoren, insbesondere mit jeweiligen Usern und deren Initialpasswörtern, eingerichtet sein, insbesondere über die TIA-interne Funktion, insbesondere im Vorfeld der Partikelzählung. Eine oder mehrere Funktionen, welche nur von einem eingeschränkten Benutzerkreis gesteuert oder aktiviert werden dürfen, können mit einer Sicherungsfunktion, insbesondere einer Security-Funktion, mit einer entsprechenden Berechtigungsbeschränkung auf eine vorab eingestellte Gruppe versehen sein.

Wie oben ausgeführt, kann die zweite Bedienoberfläche insbesondere eine separate Ansteuerungsmöglichkeit von Bewegungsrichtungen, insbesondere Bewegungsrichtungen des Fahrgestells und/oder des Sondenhalters, bereitstellen. Die zweite Bedienoberfläche kann eingerichtet sein für ein manuelles Fahren des Scanners, insbesondere für ein manuelles Führen des Sondenhalters quer zu der Transportrichtung des Transportbands und/oder für ein manuelles Fahren des Fahrgestells in die Transportrichtung des Transportbands. Die zweite Bedienoberfläche kann insbesondere mehrere Buttons, insbesondere mehrere einzelne Buttons, aufweisen, welche eingerichtet sind, den ersten Motor der Linearführung und/oder den zweiten Motor des Fahrgestells anzusteuern, insbesondere in einer Fahrtrichtung. Insbesondere können die Buttons mindestens einen ersten Button umfassen für eine Steuerung einer Vorwärtsbewegung des Fahrgestells in die Transportrichtung. Weiterhin können die Buttons mindestens einen zweiten Button umfassen für eine Steuerung einer Rückwärtsbewegung des Fahrgestells gegen die Transportrichtung. Weiterhin können die Buttons mindestens einen dritten Button umfassen für eine Steuerung des Sondenhalters quer zu der Transportrichtung, insbesondere von dem ersten Ende der Führungsschiene zu dem zweiten Ende der Führungsschiene. Weiterhin können die Buttons mindestens einen vierten Button umfassen für eine Steuerung des Sondenhalters quer zu der Transportrichtung, insbesondere von dem zweiten Ende der Führungsschiene zu dem ersten Ende der Führungsschiene. Die Führung des Sondenhalters von dem ersten Ende der Führungsschiene zu dem zweiten Ende der Führungsschiene kann auch als Bewegung oder Führung des Sondenhalters nach links bezeichnet werden. Weiterhin kann die Führung des Sondenhalters von dem zweiten Ende der Führungsschiene zu dem ersten Ende der Führungsschiene auch als Bewegung oder Führung des Sondenhalters nach rechts bezeichnet werden, oder umgekehrt. Der erste Button, der zweite Button und der vierte Button können insbesondere als Steuerkreuz angeordnet sein. Weiterhin kann die zweite Bedienoberfläche einen Homebutton umfassen, welcher insbesondere zentriert in dem Steuerkreuz angeordnet sein kann. Über den Homebutton kann eine Führung des Sondenhalters an das erste Ende der Führungsschiene oder an das zweite Ende der Führungsschiene erfolgen.

Die zweite Bedienoberfläche kann insbesondere eingerichtet sein für ein Zurückfahren des Scanners nach Durchführen des Verfahrens zum Partikelzählen in eine Ausgangsposition, insbesondere für das Zurückfahren des Fahrgestells in die Ausgangsposition. Insbesondere kann das Zurückfahren des Fahrgestells in die Ausgangsposition eine Rückwärtsbewegung des Fahrgestells in die Ausgangsposition umfassen. Insbesondere aus diesem Grund kann die zweite Bedienoberfläche mindestens ein, insbesondere mindestens zwei Eingabefelder für eine Geschwindigkeit umfassen. Insbesondere kann die zweite Bedienoberfläche ein erstes Eingabefeld für eine Geschwindigkeit des Fahrgestells in Transportrichtung und ein zweites Eingabefeld für eine Geschwindigkeit des Sondenhalters quer zu der Transportrichtung umfassen. Dadurch kann der Scanner gegebenenfalls schneller wieder in die Ausgangsposition gefahren werden, als der Scanner das Verfahren zum Partikelzählen durchgeführt hat. Eingegebene Werte der Geschwindigkeit können eingerichtet sein, Haltezeiten von Einschalterverzögerungen beziehungsweise Ausschalterverzögerungen, welche für Pulsgeber verwendet werden können, zu manipulieren, was später noch näher erläutert wird. Weiterhin kann die zweite Bedienoberfläche mindestens einen "zurück"-Button aufweisen, über welchem die zweite Bedienoberfläche, insbesondere der Handbetrieb, mit einem Bildwechsel in das Hauptmenü, wechselt und insbesondere den Handbetrieb beendet. Weiterhin kann die zweite Bedienoberfläche ein oder mehrere Informationsfenster aufweisen, insbesondere da über die zweite Bedienoberfläche ein oder mehrere Testfahrten, beispielsweise für eine Störungssuche, durchgeführt werden können. Das Informationsfenster kann insbesondere ein oder mehrere Texte, Anzeigen und Buttons aufweisen. Die zweite Bedienoberfläche kann insbesondere einen Service-Button aufweisen, über welchen das Informationsfenster aktiviert wird. Die Aktivierung kann insbesondere durch eine Funktion einer Sichtbarkeit von Bildelementen in der Programmierung umgesetzt werden. Insbesondere kann der Service-Button eingerichtet sein, ein Bit zu aktivieren, welches für eine Sichtbarkeitsabfrage von Elementen verwendet wird. Das Informationsfenster kann insbesondere einen Schließ-Button aufweisen, welcher insbesondere eingerichtet sein kann, den Bit zu deaktivieren, wodurch die Elemente ihre Sichtbarkeit verlieren. Das Informationsfenster kann darüber hinaus ein oder mehrere Buttons für ein Zurücksetzen von Inkrementen für die Bewegung des Fahrgestells und/oder für die Führung des Sondenhalters umfassen. Darüber hinaus kann das Informationsfenster ein oder mehrere Buttons für eine Simulierung von Endanschlägen aufweisen. Zusätzlich kann das Informationsfenster ein oder mehrere Anzeigen für binäre Ausgänge der Schrittgebersteuerung aufweisen. Auch weitere Ausgestaltungen sind grundsätzlich denkbar.

Wie oben ausgeführt, kann die erste Bedienoberfläche insbesondere ein Starten und ein Stoppen einer definierten, automatischen Fahrt des Fahrgestells und/oder der Sonde umfassen. Insbesondere kann die erste Bedienoberfläche ein Verfahren einer vordefinierten mäandrischen Bahn des Scanners umfassen. Insbesondere kann der Fahrweg ein Mäandermuster mit abwechselnden Bewegungen der Sonde quer und parallel zu der Transportrichtung umfassen. Insbesondere kann die Benutzerschnittstelle eingerichtet sein, die Linearführung in die Transportrichtung des Transportbands mittels des Fahrgestells schrittweise zu bewegen. Weiterhin kann die Benutzerschnittstelle eingerichtet sein, den Sondenhalter quer, insbesondere im Wesentlichen senkrecht, zu der Transportrichtung mittels des Querläufers zu führen. Die schrittweise Bewegung der Linearführung in der Transportrichtung und die Führung des Sondenhalters quer zu der Transportrichtung, insbesondere von dem ersten Ende der Führungsschiene zu dem zweiten Ende der Führungsschiene oder von dem zweiten Ende der Führungsschiene zu dem ersten Ende der Führungsschiene können abwechselnd erfolgen, sodass sich das Mäandermuster ergibt. Die erste Bedienoberfläche kann insbesondere einen Start-Button umfassen, über welchen eine einprogrammierte Schrittkette gestartet wird. Weiterhin kann die erste Bedienoberfläche eingerichtet sein, einen Stopp-Button sichtbar zu machen, welcher insbesondere an einer Position der ersten Bedienoberfläche erscheint, welche einer Position des Start-Buttons entspricht. Weiterhin kann die erste Bedienoberfläche eingerichtet sein, über den Stopp-Button die einprogrammierte Schrittkette anzuhalten und insbesondere die Sichtbarkeit des Stopp-Buttons zu deaktivieren. Weiterhin kann die erste Bedienoberfläche zwei Ausgabefelder aufweisen, welche eine aktuelle kartesische Position der Sonde anzeigen. Weiterhin kann die erste Bedienoberfläche einen Button "Position speichern" aufweisen. Die erste Bedienoberfläche eingerichtet sein, durch Drücken des Buttons "Position speichern" erfasste Koordinaten, insbesondere zu dem Zeitpunkt des Drückens erfasste Koordinaten, zwischenzuspeichern, insbesondere in einem Datenbaustein. Dadurch kann ein Zähler, insbesondere ein Leckage-Zähler, eine Anzahl möglicher Lecks erhöhen. Eine Darstellung von Positionen möglicher Lecks kann mehrere, insbesondere fünf, Variablen, jeweils für eine Position in der Transportrichtung und für eine Position quer zu der Transportrichtung, aufweisen. Die Positionen können auch als x- und y-Positionen bezeichnet werden. Werte dieser Variablen können default-mäßig Null betragen, insbesondere wenn der Leckagezähler den Wert Null aufweist. Sobald der Leckagezähler auf den Wert Eins erhöht wird, werden in einem ersten Variablenpaar, insbesondere einem ersten x-y-Variablenpaar, aktuelle Positionswerte angezeigt werden. Dadurch hätte man nun jedoch grundsätzlich das Problem, dass Positionswerte einer ersten Leckage simultan zu einer aktuellen Messposition weiterlaufen, anstatt eine Momentaufnahme der Position anzufertigen. Gelöst werden kann dieses Problem durch eine Verwendung von Flanken. Hierbei kann, sobald der Leckagezähler sich auf den Wert Eins erhöht, ein Impuls, insbesondere ein Impuls mit einer Zeitspanne von 100ms, gestartet werden, welcher eingerichtet ist, dass nur während der Zeitspanne des Impulses die aktuellen Positionswerte geschrieben werden. Die Darstellung von Positionen möglicher Lecks kann insbesondere einen clear"-Button aufweisen, welcher eingerichtet ist, die Variablen auf den Wert Null zurückzusetzen, insbesondere über eine Zurücksetzung des Leckagezählers. Wie bereits ausgeführt, kann insbesondere ein Speichern von bis zu 5 Leckagen vorgesehen sein, da bereits diese Anzahl an Auffälligkeiten auf einen kritischen Zustand des überprüften Filterelementes hindeutet. Die Benutzerschnittstelle kann eingerichtet sein, die Sonde gezielt zu mindestens einer vorgebbaren Sondenposition, insbesondere zu einer gespeicherten Position, zu bewegen und dort eine Partikelzählung durchzuführen. So können die gespeicherten Positionen möglicher Lecks nochmals überprüft werden.

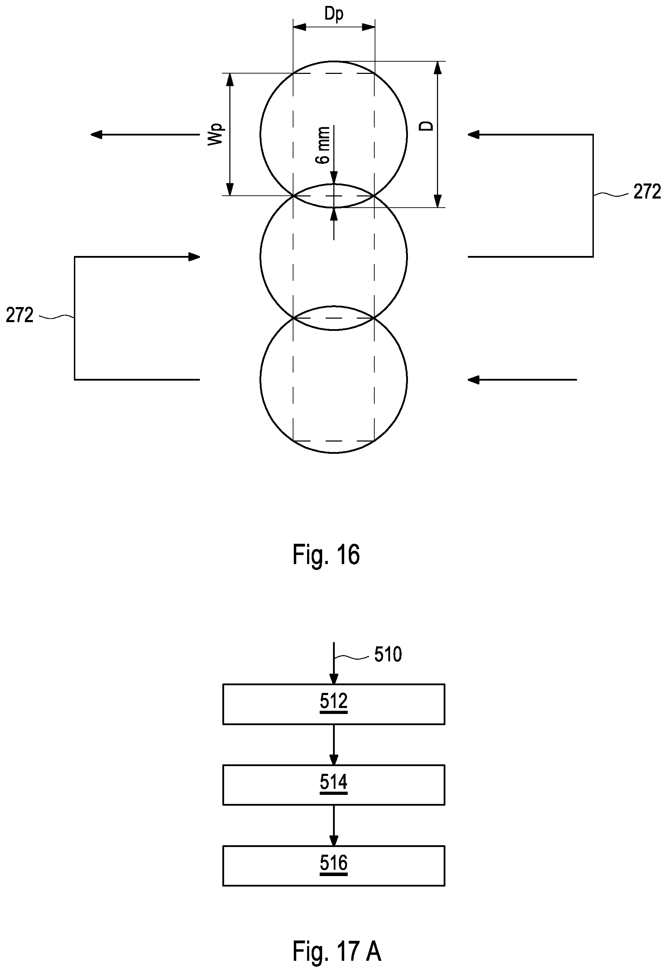

Die Sonde kann, wie oben ausgeführt, die Sondenöffnung, insbesondere den Sondentrichter, umfassen. Die Benutzerschnittstelle kann, insbesondere über die erste Bedienoberfläche, eingerichtet sein, die schrittweise Bewegung der Linearführung in die Transportrichtung des Transportbands mittels des Fahrgestells derart durchzuführen, dass eine Schrittweite der Partikelzählvorrichtung in die Transportrichtung des Transportbands mittels des Fahrgestells kleiner ist als der äußere Durchmesser der Sondenöffnung. Weitere Details hierzu finden sich in Figur 16 und der dazugehörigen, nachfolgend aufgeführten, Beschreibung. Weiterhin kann die Benutzerschnittstelle eingerichtet sein, um Partikelzahlen in Abhängigkeit von einer Sondenposition zu erfassen und insbesondere darzustellen.

Weiterhin kann die Partikelzählvorrichtung mindestens einen Temperatursensor umfassen. Der Temperatursensor kann eingerichtet sein, eine Temperatur in dem Sterilisationstunnel zu erfassen. Insbesondere kann der Temperatursensor eingerichtet sein, eine Temperatur von Luft in dem Sterilisationstunnel zu erfassen. Der Temperatursensor kann insbesondere ein elektrisches oder elektronisches Bauteil sein, welches eingerichtet sein kann, ein elektrisches Signal als Maß für die Temperatur bereitzustellen.

Der Temperatursensor kann insbesondere auf dem Fahrgestell angebracht sein. Die Partikelzählvorrichtung, insbesondere die Benutzerschnittstelle, kann eingerichtet sein, um bei Überschreiten mindestens einer Temperaturschwelle, insbesondere einer definierten Temperaturschwelle, eine Warnung auszugeben, insbesondere in der ersten Bedienoberfläche und/oder in der zweiten Bedienoberfläche. Weiterhin kann die Partikelzählvorrichtung, insbesondere die Benutzerschnittstelle, eingerichtet sein, bei Überschreiten der mindestens einen Temperaturschwelle eine Bewegung der Linearführung in der Transportrichtung des Transportbands durch das Fahrgestell abzubrechen.

In einem weiteren Aspekt der vorliegenden Erfindung wird ein Sterilisationstunnel einer Medikamentenabfüllanlage vorgeschlagen. Der Sterilisationstunnel umfasst mindestens ein Transportband. Das Transportband ist eingerichtet, mindestens ein Gefäß entlang einer Transportrichtung des Transportbands zu führen. Weiterhin umfasst der Sterilisationstunnel mindestens einen Schwebstofffilter. Weiterhin umfasst der Sterilisationstunnel mindestens eine zwischen dem Schwebstofffilter und dem Transportband angeordnete Partikelzählvorrichtung wie sie bereits beschrieben wurde oder im Folgenden noch beschrieben wird. Insbesondere kann die Partikelzählvorrichtung zumindest teilweise auf dem Transportband angeordnet sein. Die Sonde der Partikelzählvorrichtung weist mit einer Sondenöffnung, insbesondere einem Sondentrichter, dem Schwebstofffilter zu.

Der Sterilisationstunnel kann weiterhin mindestens einen Zuluftkanal umfassen. Der Begriff "Zuluftkanal", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, Zuluft umfassend ein gasförmiges Medium, insbesondere Luft, derart gerichtet zu leiten, dass die Zuluft einer anderen Vorrichtung zuführbar ist. Der Zuluftkanal kann dabei eingerichtet sein, die Zuluft dem Sterilisationstunnel, insbesondere dem Schwebstofffilter, zuzuführen. Der Zuluftkanal kann insbesondere die die Medikamentenabfüllanlage umgebende Luft dem Sterilisationstunnel zuführen. Der Zuluftkanal kann eingerichtet sein, die Zuluft der anderen Vorrichtung in einer laminaren Strömung zuzuführen.

Der Zuluftkanal kann mindestens einen Ventilator zum Ansaugen von Umgebungsluft umfassen. Der Begriff "Ventilator", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, ein gasförmiges Medium durch Druckerhöhung in dem gasförmiges Medium zu fördern. Der Ventilator kann beispielsweise ein axial oder radial rotierendes Laufrad umfassen, welches aufgrund durch eben jene Rotation den Druck in dem gasförmigen Medium erhöht. Der Ventilator kann eine Ansaugseite und eine Druckseite aufweisen, wobei der Druck in dem gasförmigen Medium auf der Druckseite größer als auf der Ansaugseite sein kann. Das gasförmige Medium kann dabei insbesondere von der Ansaugseite zu der Druckseite des Ventilators befördert werden. Das vom Ventilator geförderte gasförmige Medium kann Luft, insbesondere die die Medikamentenabfüllanlage umgebende Luft, umfassen. Der Ventilator kann ausgewählt sein aus der Gruppe bestehend aus: ein Axialventilator; ein Diagonalventilator; ein Radialventilator; ein Zentrifugalventilator; ein Tangentialventilator; ein Querstromventilator.

Der Sterilisationstunnel kann weiterhin mindestens eine Absaugvorrichtung umfassen. Die Absaugvorrichtung kann eingerichtet sein, Luft unterhalb des Transportbands abzusaugen. Der Begriff "Absaugvorrichtung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, ein gasförmiges Medium, insbesondere Luft, aus einem Entnahmebereich teilweise zu entnehmen und einem Ausgabebereich zuzuführen. Die teilweise Entnahme kann insbesondere eine Entnahme nur eines Teilvolumens des gasförmigen Mediums in dem Entnahmebereich umfassen, wobei das entnommene Teilvolumen abhängig von einer Förderleistung der Absaugvorrichtung ist. Der Entnahmebereich kann zumindest teilweise von einer anderen Vorrichtung umfasst sein oder mit dieser anderen Vorrichtung zumindest teilweise zusammenfallen. So kann die Absaugvorrichtung das gasförmige Medium, beispielsweise Luft, aus dieser anderen Vorrichtung hinausbefördern, insbesondere indem das gasförmige Medium aus dem Entnahmebereich entnommen wird und dem Ausgabebereich zugeführt wird. Beispielsweise kann der Entnahmebereich der Absaugvorrichtung zumindest teilweise mit dem Sterilisationstunnel zusammenfallen, wobei sich der Ausgabebereich außerhalb des Sterilisationstunnels befinden kann, sodass die Absaugvorrichtung Luft aus dem Sterilisationstunnel hinausbefördern kann.

Der Sterilisationstunnel kann insbesondere mindestens drei Zonen umfassen. Bei einer ersten Zone kann es sich um eine Anwärmzone handeln. Die Anwärmzone kann auch als Einlauf bezeichnet werden. Bei der zweiten Zone kann es sich um eine Sterilisationszone handeln. Die Sterilisationszone kann auch als Heißteil bezeichnet werden. Bei einer dritten Zone kann es sich um eine Abkühlzone handeln. Die dritte Zone kann auch als Abkühlzone bezeichnet werden. Das Transportband kann eingerichtet sein, sich, insbesondere mit einer konstanten Geschwindigkeit, durch den Sterilisationstunnel zu bewegen.

Die Anwärmzone kann eingerichtet sein, die Gefäße langsam an eine Temperatur der Sterilisationszone heranzuführen. Insbesondere kann die Anwärmzone eingerichtet sein, Glas der Gefäße zu erwärmen, insbesondere langsam zu erwärmen, insbesondere um entstehende Spannungen in dem Glas zu reduzieren und einen möglichen Glasbruch zu vermeiden. Die Sterilisationszone kann eingerichtet sein, Wasser an und in den Gefäßen zu verdampfen. Weiterhin kann die Sterilisationszone eingerichtet sein, die Gefäße zu sterilisieren, insbesondere bei einer Temperatur von über 300 °C, vorzugsweise bei 330 °C. Die Abkühlzone kann eingerichtet sein, die Gefäße abzukühlen, insbesondere langsam abzukühlen, insbesondere bis auf eine Temperatur von kleiner gleich 60 °C, insbesondere um aufgebaute Spannungen im Glas kontrolliert abzubauen. Die Medikamentenanlage kann eingerichtet sein, die Gefäße von der Abkühlzone in die Abfüllanlage zu transportieren. Die Abfüllanlage kann eingerichtet sein, die Gefäße mit ein oder mehreren der Medikamente zu befüllen. Weiterhin kann die Abfüllanlage eingerichtet sein, die Gefäße nach der Befüllung zu versiegeln. Die Inspektionsmaschine kann eingerichtet sein, die abgefüllten Medikamente auf Kontaminationen, insbesondere Partikel, zu untersuchen. Die Inspektionsmaschine kann insbesondere für eine optische Kontrolle der Medikamente eingerichtet sein. Werden keine Kontaminationen festgestellt, wird das Medikament zur Verpackung freigegeben.

Die Anwärmzone kann mindestens einen ersten Zuluftkanal, mindestens einen ersten Ventilator und einen ersten Schwebstofffilter aufweisen. Der erste Ventilator und der erste Schwebstofffilter können in dem Zuluftkanal angeordnet sein. Der erste Schwebstofffilter kann auch als Vorfilter bezeichnet werden. Der erste Ventilator kann eingerichtet sein, durch den ersten Schwebstofffilter Umgebungsluft anzusaugen, insbesondere Umgebungsluft aus dem Reinraum. Der erste Ventilator kann weiterhin eingerichtet sein, die angesaugte Umgebungsluft dem ersten Schwebstofffilter zuzuführen. Der Zuluftkanal kann eingerichtet sein, die gefilterte Umgebungsluft als laminare Strömung bereitzustellen. Die gefilterte Luft kann einen Transportbandbereich des Sterilisationstunnels durchströmen.

Die Absaugvorrichtung kann mindestens einen weiteren Ventilator umfassen. Der weitere Ventilator kann insbesondere unterhalb des Transportbands angeordnet sein. Der weitere Ventilator kann eingerichtet sein, Luft, insbesondere feuchte Luft, unterhalb des Transportbands abzusaugen.

Die Sterilisationszone kann im Umlaufverfahren arbeiten. Die Sterilisationszone kann mindestens einen zweiten Ventilator und mindestens einen zweiten Schwebstofffilter aufweisen. Der zweite Ventilator kann eingerichtet sein, Luft von unterhalb des Transportbands abzusaugen, in einen Bereich oberhalb des Transportbands zu lenken. Die Sterilisationszone kann weiterhin mindestens ein Heizaggregat aufweisen, welches eingerichtet ist, die Luft zu erwärmen. Der zweite Ventilator kann weiterhin eingerichtet sein, die erwärmte Luft, insbesondere Heißluft, dem zweiten Schwebstofffilter zuzuführen. Die Heißluft kann, insbesondere laminar, oberhalb des Transportbandbereichs austreten.

Der weitere Ventilator kann eingerichtet sein, Luft, insbesondere mit Wasserdampf gesättigte Luft, in der Sterilisationszone abzusaugen, insbesondere um zu verhindern, dass sich die mit Wasserdampf gesättigte Luft in der Sterilisationszone anreichert. Die Sterilisationszone kann weiterhin mindestens einen dritten Schwebstofffilter aufweisen. Über den dritten Schwebstofffilter kann Umgebungsluft bei Bedarf nachströmen.

Die Abkühlzone kann mindestens einen dritten Ventilator und mindestens einen vierten Schwebstofffilter aufweisen. Der dritte Ventilator kann eingerichtet sein, Umgebungsluft, insbesondere kühle Umgebungsluft, insbesondere kühle Reinraumlift, anzusaugen und dies durch den vierten Schwebstofffilter zu drücken. Die austretende laminare Strömung kann eingerichtet sein, die Gefäße abzukühlen.

Der Sterilisationstunnel kann weiterhin mindestens einen vierten Ventilator und mindestens einen Ablaufkanal aufweisen. Der vierte Ventilator kann insbesondere ein Ventilator der Abkühlzone sein. Der vierte Ventilator kann eingerichtet sein, Luft unterhalb des Transportbands, insbesondere erwärmte Luft, insbesondere Luft in der Abkühlzone, abzusaugen und dem Abluftkanal zuzuführen.

Die laminare Luftströmung in den einzelnen Zonen, insbesondere in der Anwärmzone, der Sterilisationszone und der Abkühlzone, kann von oben nach unten strömen. Eventuell vorhandene Partikel im Sterilisationstunnel werden dadurch nach unten gedrückt, wodurch grundsätzlich sichergestellt wird, dass keine Partikel in die Gefäße gelangen. Im Sterilisationstunnel, insbesondere im gesamten Sterilisationstunnel, kann außerdem ein Überdruck, insbesondere ein geringer Überdruck, herrschen, insbesondere damit keine Partikel in den Sterilisationstunnel hineingelangen können.

In einem weiteren Aspekt der vorliegenden Erfindung wird eine Verwendung der Partikelzählvorrichtung wie sie bereits beschrieben wurde oder im Folgenden noch beschrieben wird, zur Partikelzählung in einem Sterilisationstunnel einer Medikamentenabfüllanlage vorgeschlagen.

In einem weiteren Aspekt der vorliegenden Erfindung wird ein Verfahren zum Partikelzählen in einem Sterilisationstunnel einer Medikamentenabfüllanlage mittels der Partikelzählvorrichtung wie sie bereits beschrieben wurde oder im Folgenden noch beschrieben wird, vorgeschlagen. Das Verfahren zum Partikelzählen kann auch als Lecktest bezeichnet werden.

Man unterscheidet grundsätzlich zwischen zwei verschiedenen Betriebszuständen des Sterilisationstunnels. Ein erster Betriebszustand kann als "at rest" (englisch: "in Ruhe") bezeichnet werden. Bei "at rest" kann sich der gesamte Sterilisationstunnel bei der Durchführung des Verfahrens zum Partikelzählen in einem kalten Zustand befinden. Insbesondere können die Ventilatoren für eine Luftversorgung des Sterilisationstunnels in Betrieb sein. Die Heizung kann jedoch abgeschaltet sein und es können sich insbesondere keine Gefäße auf dem Transportband befinden. Ein zweiter Betriebszustand kann als "in operation" (englisch: "in Betrieb") bezeichnet werden. Bei "in operation" kann eine Reinraumklassenbestimmung unter Produktionsbedingungen durchgeführt werden. Der Sterilisationstunnel kann sich in einem heißen Zustand befinden. Die Ventilatoren für die Luftversorgung des Sterilisationstunnels können laufen, die Heizung kann angeschaltet sein und es können sich ebenfalls keine Gefäße auf dem Transportband befinden.

Das Verfahren zum Partikelzählen wird grundsätzlich im Anlagenzustand "at rest" durchgeführt. Der Schwebstofffilter, insbesondere der HEPA-Filter, kann bei einem Nennvolumenstrom auf einer Rohluftseite beaufschlagt werden. Unter einem Nennvolumenstrom ist grundsätzlich eine Luftmenge gemeint, bei welcher der Schwebstofffilter unter Betriebsbedingungen im Sterilisationstunnel eingesetzt sein kann. Eine Luft im Zuluftkanal vor dem Schwebstofffilter kann als Rohluft definiert werden. Eine Luft nach einer Durchströmung eines Schwebstofffilters kann als Reinluft definiert werden.

Bei der Durchführung des Verfahrens zum Partikelzählen, kann ein Aerosolgenerator ein konstantes Testaerosol mit definierten Eigenschaften erzeugen. Eine Partikelkonzentration kann sich über einen regelbaren Durchflussmesser mit Nadelventil einstellen lassen. Als Partikelmaterial kann Di-2-ethylhexyl-Sebacat (DEHS) zum Einsatz kommen. Vor dem Schwebstofffilter, auf der Rohluftseite, kann dieses Testaerosol über einen Prüfstutzen eingeleitet werden. Die Partikelkonzentration auf der Rohluftseite kann von einem Partikelzähler mit einer davor geschalteten Verdünnungsstufe überwacht werden. Die Verdünnungsstufe kann die angesaugten Partikel mit einem Verdünnungsfaktor 1:1000 reduzieren. Der Partikelzähler kann die Luft mit den Partikeln ansaugen, deren Größe und Anzahl messen und diese auswerten. Ein Sensor des Partikelzählers kann Partikel mit einer Größe von 0,3 $\mu$m bis 10 $\mu$m messen und zählen. Die Verdünnungsstufe kann notwendig sein, da der Partikelzähler grundsätzlich eine maximale Konzentration der Partikel messen kann und diese Konzentration ohne Verdünnungsstufe überschritten werden kann.

Auf der Reinluftseite kann die Partikelanzahl mit einer definierten isokinetischen Sonde, deren Rohr mit einem geeigneten Schlauch mit einem weiteren Partikelzähler verbunden ist, gemessen werden. Während der Messung kann die gesamte Filterfläche schrittweise abgefahren werden, wie nachfolgend noch näher erläutert wird. Der weitere Partikelzähler kann die austretende Reinluft über die Sonde ansaugen und eine gemessene Partikelanzahl auswerten.

Im Rahmen des Verfahrens zum Partikelzählen kann die definierte reinluftseitige Partikelanzahl pro Messvorgang abhängig sein von der Partikelanzahl auf der Rohluftseite. Die genauen Verhältnisse von Partikelanzahl auf der Rohluftseite und erlaubter Partikelanzahl auf der Reinluftseite können firmenintern beschrieben und festgelegt sein. Durch eine Überprüfung der Funktion des Schwebstofffilters kann sichergestellt werden, dass auch bei einer definiert erhöhten Anzahl der Partikel auf der Rohluftseite, die Reinraumzone innerhalb des Sterilisationstunnels nicht verschmutzt, insbesondere nicht punktuell verschmutzt, wird. Im Rahmen des Verfahrens zum Partikelzählen kann der Scanner zeitgleich mit einem Start des Partikelzählens die nachfolgend aufgelisteten Schritte durchführen, insbesondere eine Fläche unterhalb des Schwebstofffilters selbstständig abfahren.

Das Verfahren umfasst die nachfolgend aufgelisteten Schritte. Das Verfahren kann weitere, nicht genannte Schritte umfassen.

Das Verfahren zum Partikelzählen umfasst die folgenden Schritte:

a) eine Bewegung, insbesondere eine schrittweise Bewegung, des Querläufers in die Transportrichtung des Transportbands mittels des Fahrgestells; und
b) ein Führen des Sondenhalters quer, insbesondere im Wesentlichen senkrecht, zu der Transportrichtung mittels der Linearführung, vorzugsweise mit einer konstanten Geschwindigkeit.

Die Schritte a) und b) werden nacheinander und wiederholt ausgeführt.

Während des Schritts a) kann das Fahrgestell um eine definierte Strecke in die Transportrichtung des Transportbands bewegt werden. Die Strecke kann als Schrittweite bezeichnet werden. Vor Durchführung des Schritts a) kann der Sondenhalter an einem ersten Ende der Linearführung angeordnet sein. Während des Schritts b) kann der Sondenhalter von dem ersten Ende zu einem zweiten Ende der Linearführung geführt werden. Dadurch kann sich eine mäandierende Fahrbahn des Sondenhalters ergeben. Weiterhin kann die Sonde eine Sondenöffnung, insbesondere einen Sondentrichter, mit einem äußeren Durchmesser umfassen, wobei der Schritt a) derart durchgeführt wird, dass eine Schrittweite der Partikelzählvorrichtung in die Transportrichtung des Transportbands mittels des Fahrgestells kleiner ist als der äußere Durchmesser der Sondenöffnung. Dadurch kann der Bereich unterhalb des Schwebstofffilters in überlappenden Bahnen abgefahren werden. Weitere Details hierzu finden sich in Figur 16 und der dazugehörigen, nachfolgend aufgeführten, Beschreibung. Die Schritte a) und/oder b) können insbesondere in einem Handbetrieb oder automatisch ausgeführt werden. Für weitere Details wird auf obige Beschreibung verwiesen. Insbesondere kann das Verfahren ein Partikelzählen mittels des Partikelzählers umfassen. Insbesondere kann das Partikelzählen während des Schritts b) oder während der Schritte a) und b) erfolgen.

Bei dem Verfahren kann es sich insbesondere um ein computerimplementiertes Verfahren handeln. Der Begriff "computerimplementiert", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Prozess, welcher vollständig oder teilweise unter Verwendung von Datenverarbeitungsmitteln implementiert ist, insbesondere unter Verwendung mindestens eines Prozessors, beziehen. Auf Grund der einprogrammierten Fahrgeschwindigkeit, sowie des Fahrweges kann eine Reproduzierbarkeit des Partikelzählens, auch an hintersten Filterflächen, gewährleistet sein.

Wie oben ausgeführt, kann die Partikelzählvorrichtung den mindestens einen Temperatursensor umfassen.

Während der Durchführung des Verfahrens kann mittels des mindestens eines Temperatursensors eine Temperatur in dem Sterilisationstunnel erfasst werden und bei einem Überschreiten der Temperatur über einen definierten Grenzwert kann der Schritt a) abgebrochen werden.

Wie oben ausgeführt, kann die Linearführung den mindestens einen Antrieb aufweisen. Der Antrieb kann den mindestens einen ersten Motor aufweisen. Weiterhin kann die Partikelzählvorrichtung den mindestens einen zweiten Motor aufweisen, welcher eingerichtet ist, das Fahrgestell anzutreiben.

Die Steuerung kann für den ersten Motor und für den zweiten Motor jeweils mindestens einen digitalen Ausgang für eine Motortreiber-Freigabe und mindestens einen digitalen Ausgang für eine Bewegungsrichtung aufweisen. Bei einer Durchführung von Schritt a) und/oder von Schritt b) kann folgende Schrittkette ausgeführt werden:

i. Aktivierung des digitalen Ausgangs für die Motortreiber-Freigabe; und
ii. Generierung eines Impulssignals.

Insbesondere kann bei der Durchführung von Schritt a) der digitale Ausgang für Motortreiber-Freigabe des zweiten Motortreibers des zweiten Motors aktiviert werden. Insbesondere kann bei der Durchführung von Schritt b) der digitale Ausgang für Motortreiber-Freigabe des ersten Motortreibers des ersten Motors aktiviert werden.

Die Schritte i. und ii. können zeitlich versetzt voneinander ausgeführt werden. Insbesondere können die Schritte i. und ii. in einem zeitlichen Abstand von 100 ms oder von 200 ms ausgeführt werden. Auch andere zeitliche Abstände sind grundsätzlich denkbar.

Insbesondere kann nach Durchführung des Schritts i. eine Aktivierung des digitalen Ausgangs für die Bewegungsrichtung erfolgen. Insbesondere kann die Aktivierung des digitalen Ausgangs für die Bewegungsrichtung in Schritt a) bei einer Bewegung des Fahrgestells in die Transportrichtung, insbesondere bei einer Vorwärtsbewegung, erfolgen. Bei einer Bewegung des Fahrgestells gegen die Transportrichtung, insbesondere bei einer Rückwärtsbewegung, kann der digitale Ausgang für die Bewegungsrichtung deaktiviert sein. Insbesondere kann die Aktivierung des digitalen Ausgangs für die Bewegungsrichtung in Schritt b) bei einer Führung des Sondenhalters von dem ersten Ende der Führungsschiene zu dem zweiten Ende der Führungsschiene erfolgen und bei einer Führung des Sondenhalters von dem zweiten Ende der Führungsschiene zu dem ersten Ende der Führungsschiene kann der digitale Ausgang für die Bewegungsrichtung deaktiviert sein, oder umgekehrt.

Wie oben ausgeführt, kann die Partikelzählvorrichtung, insbesondere die Steuerung, den mindestens einen ersten Vorwärts-Rückwärts-Zähler, den mindestens einen zweiten Vorwärts-Rückwärts-Zähler und den mindestens einen weiteren Vorwärts-Rückwärts-Zähler umfassen. Mittels des mindestens eines ersten Vorwärts-Rückwärts-Zählers können erste Impulse des ersten Motors gezählt werden, mittels des mindestens eines zweiten Vorwärts-Rückwärts-Zählers können zweite Impulse des zweiten Motors gezählt werden. Mittels der ersten Impulse des ersten Motors kann eine Position der Sonde auf der Linearführung und mittels der zweiten Impulse des zweiten Motors kann eine Position des Fahrgestells auf dem Transportband ermittelt werden. Nach Durchführung des Schritts a) kann der weitere Vorwärts-Rückwärts-Zähler des zweiten Motors auf Null zurückgesetzt werden. Nach Durchführung des Schritts b) kann der erste Vorwärts-Rückwärts-Zähler des ersten Motors auf Null zurückgesetzt werden, wenn sich die Sonde am ersten Endanschlag befindet. Bei einer Überschreitung einer Partikelzahl über einen definierten Grenzwert kann die Position der Sonde auf dem Transportband erfasst, insbesondere gespeichert, werden, wie oben näher ausgeführt ist.

In einem weiteren Aspekt der vorliegenden Erfindung wird ein Computerprogramm vorgeschlagen. Das Computerprogramm führt bei Ablauf auf der Steuerung einer Partikelzählvorrichtung, wie sie bereits beschrieben wurde oder im Folgenden noch beschrieben wird, das Verfahren, wie es bereits beschrieben wurde oder im Folgenden noch beschrieben wird, aus.

In einem weiteren Aspekt der vorliegenden Erfindung wird ein Computerprogramm-Produkt, mit auf einem maschinenlesbaren Träger gespeicherten Programmcode-Mitteln vorgeschlagen, um ein Verfahren, wie es bereits beschrieben wurde oder im Folgenden noch beschrieben wird, durchzuführen, wenn das Programm auf der Steuerung einer Partikelzählvorrichtung, wie sie bereits beschrieben wurde oder im Folgenden noch beschrieben wird, ausgeführt wird.

Dabei wird unter einem Computer-Programmprodukt das Programm als handelbares Produkt verstanden. Es kann grundsätzlich in beliebiger Form vorliegen, so zum Beispiel auf Papier oder einem computerlesbaren Datenträger und kann insbesondere über ein Datenübertragungsnetz verteilt werden. Insbesondere können die Programmcode-Mittel auf einem computerlesbaren Datenträger und/oder einem computerlesbaren Speichermedium gespeichert sein. Die Begriffe "computerlesbarer Datenträger" und "computerlesbares Speichermedium", wie sie hier verwendet werden, können sich insbesondere auf nicht-transitorische Datenspeicher beziehen, beispielsweise auf ein Hardware-Datenspeichermedium, auf welchem Computer-ausführbare Instruktionen gespeichert sind. Der computerlesbare Datenträger oder das computerlesbare Speichermedium können insbesondere ein Speichermedium wie ein Random-Access Memory (RAM) und/oder ein Read-Only Memory (ROM) sein oder umfassen.

Außerdem wird im Rahmen der vorliegenden Erfindung ein Datenträger vorgeschlagen, auf dem eine Datenstruktur gespeichert ist, die nach einem Laden in einen Arbeits- und/oder Hauptspeicher eines Computers oder Computer-Netzwerkes das Verfahren, wie es bereits beschrieben wurde oder im Folgenden noch beschrieben wird, ausführen kann.

Schließlich wird im Rahmen der vorliegenden Erfindung ein moduliertes Datensignal vorgeschlagen, welches von einem Computersystem oder Computernetzwerk ausführbare Instruktionen zum Ausführen eines Verfahrens wie es bereits beschrieben wurde oder im Folgenden noch beschrieben wird, enthält.

Im Hinblick auf die computer-implementierten Aspekte der Erfindung können einer, mehrere oder sogar alle Verfahrensschritte des Verfahrens gemäß einer oder mehreren der hier vorgeschlagenen Ausgestaltungen mittels eines Computers oder Computer-Netzwerks durchgeführt werden. Somit können, allgemein, jegliche der Verfahrensschritte, einschließlich der Bereitstellung und/oder Manipulation von Daten mittels eines Computers oder Computer-Netzwerks durchgeführt werden. Allgemein können diese Schritte jegliche der Verfahrensschritte umfassen, ausgenommen der Schritte, welche manuelle Arbeit erfordern, beispielsweise das Bereitstellen von Proben und/oder bestimmte Aspekte der Durchführung tatsächlicher Messungen.

Die vorgeschlagenen Vorrichtungen und Verfahren weisen gegenüber bekannten Vorrichtungen und Verfahren zahlreiche Vorteile auf.

Durch die Partikelmessung in den Sterilisationstunneln wird im Allgemeinen gewährleistet, dass die darin eingesetzten Schwebstofffilter einwandfrei funktionieren. Mit der aus dem Stand der Technik bekannten manuellen Führung der Sonde kann ein Prüfer eine gesamte Filterfläche eines Schwefelfilters erfassen.

Jedoch unterliegen die Geschwindigkeit der Sondenbewegung und der Verfahrweg mehr oder weniger großen Schwankungen bei jeder Prüfung. Durch die derzeitige manuelle Führung der Sonde durch einen Prüfer, können Fehler auftreten, wodurch eventuelle undichte Stellen nicht erfasst werden. Bei Messung einer punktuell erhöhten Partikelkonzentration hat der Prüfer außerdem die Schwierigkeit die genaue Stelle, an der die Abweichung initial gemessen wurde, wieder zu finden. Dies kann wiederum mit einem erhöhten Zeitaufwand verbunden sein.

Durch die vorgeschlagenen Vorrichtungen und Verfahren kann eine teilautomatisierte Partikelmessung erzielt werden. Insbesondere kann durch den Scanner ein teilautomatisches Führen der Sonde, insbesondere zum Abfahren verschieden großer Filterflächen im Rahmen der Partikelmessung von Schwebstofffiltern in verschiedenen Sterilisationstunneln erfolgen. Die Partikelzählvorrichtung kann flexibel in Sterilisationstunneln mit unterschiedlichen Dimensionen eingesetzt werden.

Durch eine Teilautomatisierung des Lecktests für Schwebstofffilter können Zeit und Kosten eingespart werden. Die Teilautomatisierung des Prozesses stellt grundsätzlich sicher, dass die Sonde für die Partikelmessung die Filterfläche, insbesondere die gesamte Filterfläche, zumindest weitgehend lückenlos, in einem optimalen Weg und mit einer konstanten Geschwindigkeit abfahren kann. Einerseits kann dies zu einer hohen Qualität von abgefüllten Medikamenten führen. Zudem können grundsätzlich Ausschüsse reduziert werden, weil eventuelle Leckagen im Schwebstofffilter entdeckt und lokalisiert werden können. Andererseits kann der Prüfer entlastet werden, da er nicht mehr mit einem langen und umständlichen Gestänge, insbesondere der Rohrleitung, hantieren muss.

Eine Prozessautomatisierung kann weiterhin zu einer Verbesserung des Messvorgangs führen, da der Messvorgang in kürzerer Zeit und unter reproduzierbaren Bedingungen durchgeführt werden kann. Dadurch kann sich auch eine Anlagenverfügbarkeit der Medikamentenabfüllanlage erhöhen.

Die Sonde kann über eine zu messende Filterfläche des Schwebstofffilters in einem definierten Weg und einer definierten Geschwindigkeit schlupffrei geführt werden. Gefahrene Bahnen des vorgegebenen Weges können sich um einen bestimmten Wert überschneiden, überschreiten diesen Wert jedoch nicht. Die teilautomatische Führung der Sonde kann weiterhin flexibel an verschiedene Sterilisationstunneltypen, welche im Unternehmen betrieben werden, angepasst werden. Dadurch kann eine flächendeckende Führung der Sonde erreicht werden. Der Verfahrweg kann optimal eingestellt werden, um einerseits reproduzierbar die gesamte Filterfläche des Schwebstofffilters im Sterilisationstunnel abfahren zu können und andererseits, um die Zeit der Prüfung zu minimieren und somit die Anlagenverfügbarkeit zu erhöhen.

Die Partikelzählvorrichtung ist leicht handhabbar und kann flexibel an verschiedene Abmessungen der verschiedenen Sterilisationstunneltypen anpassbar sein. Eine Baugröße und ein Gewicht der Partikelzählvorrichtung kann möglichst geringgehalten sein. Eine Gesamthöhe der Partikelzählvorrichtung kann kleiner sein als eine niedrigste vorkommende Durchfahrtshöhe in dem Sterilisationstunnel, jedoch so niedrig wie konstruktiv machbar sein. Die Sonde kann einen höchsten Punkt der Partikelzählvorrichtung darstellen. Die Komponenten können so ausgewählt sein, dass eine Programmierung und Visualisierung des Verfahrweges möglich ist. Zusätzlich kann eine Sicherheit für einen Bediener beachtet werden, um Verletzungen durch eine sachgemäße Anwendung zu vermeiden.

Um die Handhabung der Partikelzählvorrichtung für den Prüfer zu erleichtern, kann das Gesamtgewicht der Partikelzählvorrichtung möglichst gering sein. Für eine Gewichtsreduktion können vorzugsweise leichte Materialien wie Aluminium oder Kunststoff, z.B. Polytetrafluorethylen (PTFE) oder Polyoxymethylen (POM), eingesetzt werden. Auch nichtrostende, austenitische Stähle können zum Einsatz kommen, welche aufgrund eines höheren Gewichts nur bedingt verwendet werden. Da die Partikelzählvorrichtung in einer Produktionsanlage im Pharmaumfeld zum Einsatz kommen kann, können eingesetzte Materialien mit Flächendesinfektionsmitteln, welche beispielsweise Isopropanol umfassen können, gereinigt werden. Die in der Verordnung (EG) Nr. 1935/2004 beschriebenen Anforderungen können erfüllt werden. Das Verfahren zur Partikelzählung kann bei Raumtemperatur durchgeführt werden. Deshalb ist eine Hitzebeständigkeit der Materialien grundsätzlich von geringerer Bedeutung. Es können zumindest weitgehend Standardbauteile verwendet werden, um bei Bedarf benötigte Teile schnell und unkompliziert beschaffen und austauschen zu können. Dadurch können auch Herstellungskosten minimiert werden.

Die Transportbänder der Sterilisationstunnel können aus einem Drahtgittergeflecht aus nichtrostendem Stahl hergestellt sein. Das Drahtgittergeflecht kann dem Transportband eine notwendige Luftdurchlässigkeit, eine Flexibilität und eine Hitzebeständigkeit verleihen, um bei den vorliegenden Bedingungen, beispielsweise einer hohen Temperatur, zuverlässig zu funktionieren. Allerdings kann durch eine grobe Geflechtstruktur des Transportbandes eine resultierende Kontaktfläche zwischen dem Scanner und dem Drahtgeflecht sehr klein sein.

Dementsprechend kann hier eine geringe Reibung entstehen. Die Reibung kann durch Anbringung von O-Ringen an den Rädern, insbesondere an den Antriebsrädern und/oder an den Hinterrädern, erhöht werden.

Weiterhin kann eine schlupffreie Bewegung des Scanners erfolgen, sodass keine oder nur geringe Abweichungen des Verfahrwegs zwischen zwei Messungen vorliegen. Zudem wird ein Abrieb an Komponenten des Scanners und zwischen dem Sterilisationstunnel und dem Scanner vermieden oder zumindest reduziert. Hierdurch kann vermieden werden, dass Partikel in den Sterilisationstunnel eingebracht werden. Auch können Komponenten verwendet werden, welche keine Schmierung mit Ölen oder Fetten benötigen. So kann grundsätzlich eine Verunreinigung im Sterilisationstunnel vermieden werden.

Der Scanner kann mindestens zwei Module aufweisen, den Querläufer und das Fahrgestell. Die Partikelzählvorrichtung kann eine maximale Bauhöhe von 160 mm aufweisen und eine maximale Breite von 600 mm aufweisen. Insbesondere kann die Partikelzählvorrichtung eine Bauhöhe von 116 mm aufweisen. Dies kann eine Handhabung bei einer Einführung und Positionierung der Partikelzählvorrichtung vor dem Messvorgang in den Sterilisationstunnel durch den Prüfer erleichtern.

Der Querläufer kann austauschbar sein. Dadurch kann möglich sein, die Partikelzählvorrichtung für verschiedene Tunnelbreiten des Sterilisationstunnels flexibel anzupassen und einzusetzen. Eine Befestigung des Querläufers auf dem Fahrgestell kann werkzeugfrei erfolgen. Eine Länge des Querläufers kann kleiner sein als eine Transportbandbreite. Ein Abstand der Sonde zu einer Seitenbegrenzung des Transportbands kann so gering wie möglich sein. Eine Verfahrgeschwindigkeit der Sonde von 5,9 cm/s bei einer Führung quer zu der Transportrichtung des Transportbands kann realisiert werden. Schlupffreie Bewegungen können realisiert werden. Die Partikelzählvorrichtung kann eine sehr hohe Wiederholgenauigkeit aufweisen.

Es kann ein Gesamtgewicht der Partikelzählvorrichtung von 5090 g realisiert werden. Dadurch kann eine kompakte und für den Prüfer leicht handhabbare Partikelzählvorrichtung realisiert werden. Der Querläufer kann durch den Prüfer einfach und ohne zusätzliches Werkzeug getauscht und die Partikelzählvorrichtung kann damit an die jeweilige Tunnelbreite der verschiedenen Sterilisationstunneltypen angepasst werden.

Mit Hilfe der Partikelzählvorrichtung kann in einer vorgegebenen Zeit auf einem vorgegebenen Weg und mit einer vorgegebenen Geschwindigkeit das Verfahren zur Partikelzählung präzise durchgeführt werden. Auch eine im Wesentlichen einwandfreie Reproduzierbarkeit der Messungen ist durchführbar.

Zusätzlich kann durch eine Programmierung und Visualisierung der Partikelzählvorrichtung, insbesondere des Scanners, grundsätzlich jederzeit die Position der Sonde auf dem Transportband bestimmt und überwacht werden.

Zusammenfassend werden, ohne Beschränkung weiterer möglicher Ausgestaltungen, folgende Ausführungsformen vorgeschlagen:

Ausführungsform 1: Partikelzählvorrichtung zum Partikelzählen in einem Sterilisationstunnel einer Medikamentenabfüllanlage, wobei der Sterilisationstunnel mindestens ein Transportband umfasst, wobei die Partikelzählvorrichtung umfasst:

- mindestens eine mit einem Partikelzähler verbindbare Sonde zum Aufnehmen von Partikeln in dem Sterilisationstunnel;
- mindestens einen Scanner mit mindestens einem Sondenhalter zum Befestigen der Sonde, wobei der Scanner umfasst:

  ◦ mindestens einen Querläufer mit mindestens einer Linearführung, wobei die Linearführung eingerichtet ist, den Sondenhalter quer, insbesondere im Wesentlichen senkrecht, zu einer Transportrichtung des Transportbands des Sterilisationstunnels zu führen;
  ◦ mindestens ein Fahrgestell, wobei der Querläufer auf dem Fahrgestell befestigt ist, wobei das Fahrgestell eingerichtet ist, die Linearführung in der Transportrichtung des Transportbands zu bewegen; und
  ◦ mindestens eine Steuerung, insbesondere eine mit dem Fahrgestell verbundene Steuerung, wobei die Steuerung eingerichtet ist, um eine Bewegung des Scanners zu steuern.

Ausführungsform 2: Partikelzählvorrichtung nach der vorhergehenden Ausführungsform, wobei die Steuerung eine speicherprogrammierbare Steuerung umfasst.

Ausführungsform 3: Partikelzählvorrichtung nach einer der vorhergehenden Ausführungsformen, wobei der Sondenhalter an der Linearführung befestigt ist.

Ausführungsform 4: Partikelzählvorrichtung nach einer der vorhergehenden Ausführungsformen, wobei die Partikelzählvorrichtung weiterhin mindestens einen mit der Sonde verbindbaren Partikelzähler umfasst.

Ausführungsform 5: Partikelzählvorrichtung nach der vorhergehenden Ausführungsform, wobei der Partikelzähler als stationärer Partikelzähler ausgestaltet ist und wobei der Partikelzähler und die Sonde mittels mindestens einer Rohrleitung, insbesondere einer flexiblen Rohrleitung, miteinander verbunden sind.

Ausführungsform 6: Partikelzählvorrichtung nach einer der vorhergehenden Ausführungsformen, weiterhin umfassend mindestens eine stationäre Benutzerschnittstelle, wobei die Benutzerschnittstelle mit dem Scanner verbunden ist und wobei mittels der Benutzerschnittstelle eine Bewegung des Scanners steuerbar ist.

Ausführungsform 7: Partikelzählvorrichtung nach der vorhergehenden Ausführungsform, wobei die Benutzerschnittstelle eine grafische Benutzeroberfläche aufweist, insbesondere eine grafische Benutzeroberfläche mit mindestens einem Touch Screen.

Ausführungsform 8: Partikelzählvorrichtung nach einer der beiden vorhergehenden Ausführungsformen, wobei mittels der Benutzerschnittstelle mindestens ein Fahrweg und/oder Messpositionen für die Partikelzählung vorgebbar sind.

Ausführungsform 9: Partikelzählvorrichtung nach der vorhergehenden Ausführungsform, wobei der Fahrweg ein Mäandermuster mit abwechselnden Bewegungen der Sonde quer und parallel zu der Transportrichtung umfasst.

Ausführungsform 10: Partikelzählvorrichtung nach einer der beiden vorhergehen Ausführungsformen, wobei der mindestens eine Fahrweg und/oder die Messpositionen in einem Handbetrieb oder in einem automatischen Betrieb vorgebbar sind.

Ausführungsform 11: Partikelzählvorrichtung nach der vorhergehenden Ausführungsform, wobei weiterhin mindestens eine Geschwindigkeit für den mindestens einen Fahrweg vorgebbar ist, insbesondere in dem Handbetrieb.

Ausführungsform 12: Partikelzählvorrichtung nach einer der sechs vorhergehenden Ausführungsformen, wobei die Benutzerschnittstelle weiterhin eingerichtet ist, um die Sonde gezielt zu mindestens einer vorgebbaren Sondenposition zu bewegen und dort eine Partikelzählung durchzuführen.

Ausführungsform 13: Partikelzählvorrichtung nach einer der sieben vorhergehenden Ausführungsformen, wobei die Benutzerschnittstelle eingerichtet ist, um Partikelzahlen in Abhängigkeit von einer Sondenposition zu erfassen und insbesondere darzustellen, wobei insbesondere die Benutzerschnittstelle zusätzlich mit einem Partikelzähler verbunden ist.

Ausführungsform 14: Partikelzählvorrichtung nach einer der acht vorhergehenden Ausführungsformen, wobei die Benutzerschnittstelle eingerichtet ist, die Linearführung in die Transportrichtung des Transportbands mittels des Fahrgestells schrittweise zu bewegen, wobei die Benutzerschnittstelle weiterhin eingerichtet ist, den Sondenhalter quer, insbesondere im Wesentlichen senkrecht, zu der Transportrichtung mittels des Querläufers zu führen.

Ausführungsform 15: Partikelzählvorrichtung nach der vorhergehenden Ausführungsform, wobei die Sonde eine Sondenöffnung, insbesondere einen Sondentrichter, umfasst, wobei die Benutzerschnittstelle eingerichtet ist, die schrittweise Bewegung der Linearführung in die Transportrichtung des Transportbands mittels des Fahrgestells derart durchzuführen, dass eine Schrittweite der Partikelzählvorrichtung in die Transportrichtung des Transportbands mittels des Fahrgestells kleiner ist das der äußere Durchmesser der Sondenöffnung.

Ausführungsform 16: Partikelzählvorrichtung nach einer der vorhergehenden Ausführungsformen, wobei die Partikelzählvorrichtung mindestens einen y-Positionssensor zur Bestimmung einer Position der Sonde in einer Dimension in Transportrichtung auf dem Transportband aufweist.

Ausführungsform 17: Partikelzählvorrichtung nach der vorhergehenden Ausführungsform, wobei der y-Positionssensor mit dem Fahrgestell, insbesondere einem Antrieb des Fahrgestells, verbunden ist.

Ausführungsform 18: Partikelzählvorrichtung nach der vorhergehenden Ausführungsform, wobei das Fahrgestell einen Schrittmotor aufweist, wobei der y-Positionssensor einen Inkrementalgeber des Schrittmotors umfasst.

Ausführungsform 19: Partikelzählvorrichtung nach einer der vorhergehenden Ausführungsformen, wobei die Partikelzählvorrichtung mindestens einen x-Positionssensor zur Bestimmung einer Position der Sonde in einer Dimension quer zur Transportrichtung, insbesondere im Wesentlichen senkrecht zur Transportrichtung, auf dem Transportband aufweist.

Ausführungsform 20: Partikelzählvorrichtung nach der vorhergehenden Ausführungsform, wobei der x-Positionssensor mit der Linearführung, insbesondere einem Antrieb der Linearführung, verbunden ist.

Ausführungsform 21: Partikelzählvorrichtung nach der vorhergehenden Ausführungsform, wobei die Linearführung einen Schrittmotor aufweist, wobei der x-Positionssensor einen Inkrementalgeber des Schrittmotors umfasst.

Ausführungsform 22: Partikelzählvorrichtung nach einer der vorhergehenden Ausführungsformen, wobei der Querläufer mindestens zwei Endlagenschalter, insbesondere mindestens zwei Rollenendschalter, für eine Bestimmung der Positionen der Endlagen der Sonde quer zu der Transportrichtung des Transportbands umfasst.

Ausführungsform 23: Partikelzählvorrichtung nach einer der vorhergehenden Ausführungsformen, wobei das Fahrgestell mindestens einen Endlagenschalter, insbesondere mindestens einen Federstabendschalter, für eine Bestimmung mindestens einer Position mindestens einer Endlage der Partikelzählvorrichtung in der Transportrichtung aufweist.

Ausführungsform 24: Partikelzählvorrichtung nach einer der vorhergehenden Ausführungsformen, wobei die Partikelzählvorrichtung weiterhin mindestens einen Temperatursensor umfasst, wobei der Temperatursensor eingerichtet ist, eine Temperatur in dem Sterilisationstunnel zu erfassen.

Ausführungsform 25: Partikelzählvorrichtung nach der vorhergehenden Ausführungsform, wobei die Partikelzählvorrichtung eingerichtet ist, um bei Überschreiten mindestens einer Temperaturschwelle eine Warnung auszugeben.

Ausführungsform 26: Partikelzählvorrichtung nach der vorhergehenden Ausführungsform, wobei die Partikelzählvorrichtung eingerichtet ist, um bei Überschreiten der mindestens einen Temperaturschwelle eine Bewegung der Linearführung in der Transportrichtung des Transportbands durch das Fahrgestell abzubrechen.

Ausführungsform 27: Partikelzählvorrichtung nach einer der vorhergehenden Ausführungsformen, wobei die Linearführung auf einer Grundplatte befestigt ist, wobei die Linearführung mittels der Grundplatte auf dem Fahrgestell befestigt ist.

Ausführungsform 28: Partikelzählvorrichtung nach der vorhergehenden Ausführungsform, wobei die Grundplatte aus Aluminium hergestellt ist.

Ausführungsform 29: Partikelzählvorrichtung nach der vorhergehenden Ausführungsform, wobei die Grundplatte auf dem Fahrgestell mittels mindestens einer Verbindung befestigt ist ausgewählt aus der Gruppe bestehend aus: mindestens einer Schraubverbindung, mindestens einer Klick-Verbindung, mindestens einer Spannhebelverbindung.

Ausführungsform 30: Partikelzählvorrichtung nach einer der vorhergehenden Ausführungsformen, wobei die Linearführung ein Gleitlager aufweist.

Ausführungsform 31: Partikelzählvorrichtung nach einer der vorhergehenden Ausführungsform, wobei die Linearführung eine Führungsschiene, insbesondere eine T-Führungsschiene, und einen an der Führungsschiene befestigten Führungsschlitten aufweist, wobei die Sonde an dem Führungsschlitten befestigbar ist.

Ausführungsform 32: Partikelzählvorrichtung nach der vorhergehenden Ausführungsform, wobei die Führungsschiene als Loslager eingerichtet ist, insbesondere als Loslager in einer Richtung quer zu der Transportrichtung des Transportbands.

Ausführungsform 33: Partikelzählvorrichtung nach einer der vorhergehenden Ausführungsformen, wobei die Linearführung mindestens einen Antrieb aufweist ausgewählt aus der Gruppe bestehend aus: einem Spindelantrieb; einem Zahnriemenantrieb; einem Zahnstangenantrieb, wobei die Linearführung mindestens einen Zahnstangenantrieb aufweist, wobei der Zahnstangenantrieb mindestens eine Zahnstange und mindestens ein Stirnzahnrad aufweist.

Ausführungsform 34: Partikelzählvorrichtung nach der vorhergehenden Ausführungsform, wobei die Zahnstange einen runden Querschnitt aufweist.

Ausführungsform 35: Partikelzählvorrichtung nach einer der beiden vorhergehenden Ausführungsformen, wobei die Zahnstange aus einem austenitischen rostfreiem Stahl hergestellt ist.

Ausführungsform 36: Partikelzählvorrichtung nach einer der drei vorhergehenden Ausführungsformen, wobei die Zahnstange mittig über der Linearführung angeordnet ist.

Ausführungsform 37: Partikelzählvorrichtung nach einer der vier vorhergehenden Ausführungsformen, wobei das Stirnzahnrad aus Polyoxymethylene (POM) hergestellt ist.

Ausführungsform 38: Partikelzählvorrichtung nach einer der fünf vorhergehenden Ausführungsformen, wobei der Antrieb mindestens einen ersten Motor umfasst ausgewählt aus der Gruppe bestehend aus: einem Servomotor; einem Schrittmotor.

Ausführungsform 39: Partikelzählvorrichtung nach der vorhergehenden Ausführungsform, wobei der Antrieb den Zahnzahnstangenantrieb umfassend die Zahnstange und das Stirnzahnrad umfasst, wobei das Stirnzahnrad auf einer Welle des ersten Motors verspannt ist, wobei der erste Motor mit einem Befestigungswinkel auf dem Führungsschlitten befestigt ist.

Ausführungsform 40: Partikelzählvorrichtung nach einer der neun vorhergehenden Ausführungsformen, wobei der Sondenhalter auf dem Führungsschlitten befestigt ist.

Ausführungsform 41: Partikelzählvorrichtung nach einer der vorhergehenden Ausführungsformen, wobei der Sondenhalter aus Polyoxymethylene (POM) hergestellt ist.

Ausführungsform 42: Partikelzählvorrichtung nach einer der vorhergehenden Ausführungsformen, wobei der Sondenhalter mindestens eine Nut für eine Aufnahme der Sonde aufweist, wobei der Sondenhalter weiterhin mindestens eine Klemmplatte aufweist, wobei die Klemmplatte eingerichtet ist, die Sonde zu fixieren.

Ausführungsform 43: Partikelzählvorrichtung nach einer der vorhergehenden Ausführungsformen, wobei der Querläufer mittig auf dem Fahrgestell befestigt ist.

Ausführungsform 44: Partikelzählvorrichtung nach einer der vorhergehenden Ausführungsformen, wobei das Fahrgestell aus mindestens einem austenitischen rostfreiem Stahl hergestellt ist.

Ausführungsform 45: Partikelzählvorrichtung nach einer der vorhergehenden Ausführungsformen, wobei das Fahrgestell mindestens zwei Antriebsräder aufweist.

Ausführungsform 46: Partikelzählvorrichtung nach der vorhergehenden Ausführungsform, wobei die Antriebsräder aus Polyoxymethylene (POM) hergestellt sind.

Ausführungsform 47: Partikelzählvorrichtung nach einer der beiden vorhergehenden Ausführungsformen, wobei die Antriebsräder jeweils mehrere O-Ringe aufweisen, welche voneinander beabstandet auf mindestens einer Umfangsfläche der Antriebsräder angeordnet sind.

Ausführungsform 48: Partikelzählvorrichtung nach der vorhergehenden Ausführungsform, wobei die O-Ringe jeweils in Nuten der Umfangsfläche der Antriebsräder aufgenommen sind.

Ausführungsform 49: Partikelzählvorrichtung nach einer der vier vorhergehenden Ausführungsformen, wobei die Antriebsräder jeweils auf einer Radwelle befestigt sind.

Ausführungsform 50: Partikelzählvorrichtung nach einer der fünf vorhergehenden Ausführungsformen, wobei die Partikelzählvorrichtung mindestens einen zweiten Motor umfasst ausgewählt aus der Gruppe bestehend aus: einem Schrittmotor, einem Servomotor, wobei der zweite Motor eingerichtet ist, mindestens eines der Antriebsräder anzutreiben.

Ausführungsform 51: Sterilisationstunnel einer Medikamentenabfüllanlage, wobei der Sterilisationstunnel umfasst:

- mindestens ein Transportband, wobei das Transportband eingerichtet ist, mindestens ein Gefäß entlang einer Transportrichtung des Transportbands zu führen;
- mindestens einen Schwebstofffilter; und
- mindestens eine zwischen dem Schwebstofffilter und dem Transportband angeordnete Partikelzählvorrichtung nach einer der vorhergehenden Ausführungsformen, wobei die Sonde der Partikelzählvorrichtung mit einer Sondenöffnung, insbesondere einem Sondentrichter, dem Schwebstofffilter zuweist.

Ausführungsform 52: Sterilisationstunnel nach der vorhergehenden Ausführungsform, wobei der Sterilisationstunnel weiterhin mindestens einen Zuluftkanal umfasst, wobei der Zuluftkanal mindestens einen Ventilator zum Ansaugen von Umgebungsluft umfasst.

Ausführungsform 53: Sterilisationstunnel nach einer der beiden vorhergehenden Ausführungsformen, wobei der Sterilisationstunnel weiterhin mindestens eine Absaugvorrichtung umfasst, wobei die Absaugvorrichtung eingerichtet ist, Luft unterhalb des Transportbands abzusaugen.

Ausführungsform 54: Verwendung der Partikelzählvorrichtung nach einer der vorhergehenden, eine Partikelzählvorrichtung betreffenden Ausführungsformen zur Partikelzählung in einem Sterilisationstunnel einer Medikamentenabfüllanlage.

Ausführungsform 55: Verfahren zum Partikelzählen in einem Sterilisationstunnel einer Medikamentenabfüllanlage mittels der Partikelzählvorrichtung nach einer der vorhergehenden, eine Partikelzählvorrichtung betreffenden Ausführungsformen, wobei das Verfahren die folgenden Schritte umfasst:

a) eine Bewegung, insbesondere eine schrittweise Bewegung, der Linearführung in die Transportrichtung des Transportbands mittels des Fahrgestells;
b) ein Führen des Sondenhalters quer, insbesondere im Wesentlichen senkrecht, zu der Transportrichtung mittels der Linearführung;

wobei die Schritte a) und b) nacheinander und wiederholt ausgeführt werden.

Ausführungsform 56: Verfahren nach der vorhergehenden Ausführungsform, wobei vor Durchführung des Schritts a) der Sondenhalter an einem ersten Ende der Linearführung angeordnet ist, wobei in Schritt b) der Sondenhalter von dem ersten Ende zu einem zweiten Ende der Linearführung geführt wird.

Ausführungsform 57: Verfahren nach einer der beiden vorhergehenden Ausführungsformen, wobei weiterhin während der Durchführung des Verfahrens mittels mindestens eines Temperatursensors eine Temperatur in dem Sterilisationstunnel erfasst wird, wobei bei einem Überschreiten der Temperatur über einen definierten Grenzwert der Schritt a) abgebrochen wird.

Ausführungsform 58: Verfahren nach einer der drei vorhergehenden Ausführungsformen, wobei die Schritte a) und/oder b) in einem Handbetrieb ausgeführt werden.

Ausführungsform 59: Verfahren nach einer der vier vorhergehenden Ausführungsformen, wobei die Schritte a) und/oder b) automatisch ausgeführt werden.

Ausführungsform 60: Verfahren nach einer der fünf vorhergehenden Ausführungsformen, wobei die Linearführung mindestens einen Antrieb aufweist, wobei der Antrieb mindestens einen ersten Motor aufweist, wobei die Partikelzählvorrichtung weiterhin mindestens einen zweiten Motor aufweist, wobei der zweite Motor eingerichtet ist, das Fahrgestell anzutreiben, wobei die Steuerung für den ersten Motor und für den zweiten Motor jeweils mindestens einen digitalen Ausgang für eine Bewegungsrichtung aufweist und mindestens einen digitalen Ausgang für eine Motortreiber-Freigabe aufweist, wobei bei einer Durchführung von Schritt a) und/oder von Schritt b) folgende Schrittkette ausgeführt wird:

i. Aktivierung des digitalen Ausgangs für die Motortreiber-Freigabe; und
ii. Generierung eines Impulssignals.

Ausführungsform 61: Verfahren nach der vorhergehenden Ausführungsform, wobei die Schritte i. und ii. zeitlich versetzt voneinander ausgeführt werden.

Ausführungsform 62: Verfahren nach einer der beiden vorhergehenden Ausführungsformen, wobei nach Durchführung des Schritts i. eine Aktivierung des digitalen Ausgangs für die Bewegungsrichtung erfolgt.

Ausführungsform 63: Verfahren nach einer der vier vorhergehenden Ausführungsformen, wobei mittels mindestens eines ersten Vorwärts-Rückwärts-Zählers erste Impulse des ersten Motors gezählt werden, wobei mittels mindestens eines zweiten Vorwärts-Rückwärts-Zählers zweite Impulse des zweiten Motors gezählt werden, wobei mittels der ersten Impulse des ersten Motors eine Position der Sonde auf der Linearführung und mittels der zweiten Impulse des zweiten Motors eine Position des Fahrgestells auf dem Transportband ermittelt wird.

Ausführungsform 64: Verfahren nach der vorhergehenden Ausführungsform, wobei die Linearführung einen ersten Endanschlag und einen zweiten Endanschlag aufweist, wobei nach Durchführung des Schritts b) der erste Vorwärts-Rückwärts-Zähler des ersten Motors auf Null zurückgesetzt wird, wenn sich die Sonde am ersten Endanschlag befindet.

Ausführungsform 65: Verfahren nach einer der zehn vorhergehenden Ausführungsformen, wobei bei einer Überschreitung einer Partikelzahl über einen definierten Grenzwert die Position der Sonde auf dem Transportband erfasst wird.

Ausführungsform 66: Verfahren nach einer der elf vorhergehenden Ausführungsformen, wobei die Sonde eine Sondenöffnung, insbesondere einen Sondentrichter, mit einem äußeren Durchmesser umfasst, wobei der Schritt a) derart durchgeführt wird, dass eine Schrittweite der Partikelzählvorrichtung in die Transportrichtung des Transportbands mittels des Fahrgestells kleiner ist als der äußere Durchmesser der Sondenöffnung.

Ausführungsform 67: Computerprogramm, wobei das Computerprogramm bei Ablauf auf der Steuerung einer Partikelzählvorrichtung nach einer der vorhergehenden, eine Partikelzählvorrichtung betreffenden Ausführungsformen, ein Verfahren nach einer der vorhergehenden, ein Verfahren betreffenden Ausführungsformen ausführt.

Ausführungsform 68: Computerprogramm-Produkt mit auf einem maschinenlesbaren Träger gespeicherten Programmcode-Mitteln, um ein Verfahren nach einer der vorhergehenden, ein Verfahren betreffenden Ausführungsformen durchzuführen, wenn das Programm auf der Steuerung einer Partikelzählvorrichtung nach einer der vorhergehenden, eine Partikelzählvorrichtung betreffenden Ausführungsformen ausgeführt wird.

Kurze Beschreibung der Figuren

Weitere Einzelheiten und Merkmale ergeben sich aus der nachfolgenden Beschreibung von Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:

Figur 1             eine Ausführungsform eines Sterilisationstunnels einer Medikamentenabfüllanlage in einer seitlichen Ansicht;

Figur 2             einen schematischen Aufbau eines Lecktests in einem Sterilisationstunnel;

Figuren 3 bis 14    eine Ausführungsform einer Partikelzählvorrichtung oder Teile davon in verschiedenen Ansichten;

Figur 15   eine schematische Draufsicht einer Partikelzählvorrichtung in einem Sterilisationstunnel;

Figur 16   geometrische Abmessungen einer Partikelzählvorrichtung in einem Sterilisationstunnel;

Figuren 17A bis 17C   eine exemplarische Schrittkette für ein erfindungsgemäßes Verfahren zum Partikelzählen (Figuren 17A und 17C) und ein exemplarisches Zeitdiagramm von Steuersignalen (Figur 17B);

Figuren 18A bis 18F   verschiedene Bedienoberflächen einer Benutzerschnittstelle (Figu-ren 18A, 18B, 18D, 18E und 18F) sowie eine Impulsdarstellung (Fi-gur 18C); und

Figuren 19 bis 20   zeigen Ausführungsbeispiele einer Programmierung einer Steuerung.

Beschreibung der Ausführungsbeispiele

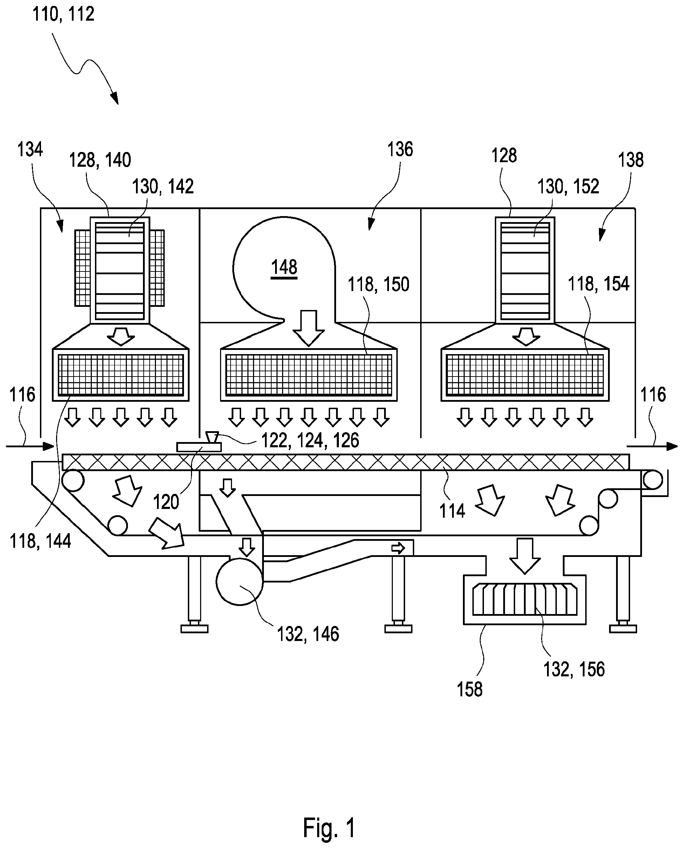

Figur 1 zeigt eine Ausführungsform eines Sterilisationstunnels 110 einer Medikamentenabfüllanlage 112 in einer seitlichen Ansicht. Der Sterilisationstunnel 110 umfasst mindestens ein Transportband 114. Das Transportband 114 ist eingerichtet, mindestens ein Gefäß (nicht in Figur 1 dargestellt) entlang einer Transportrichtung 116 des Transportbands 114 zu führen. Die Transportrichtung 116 des Transportbands 114 ist in Figur 1 durch Pfeile 116 angedeutet.

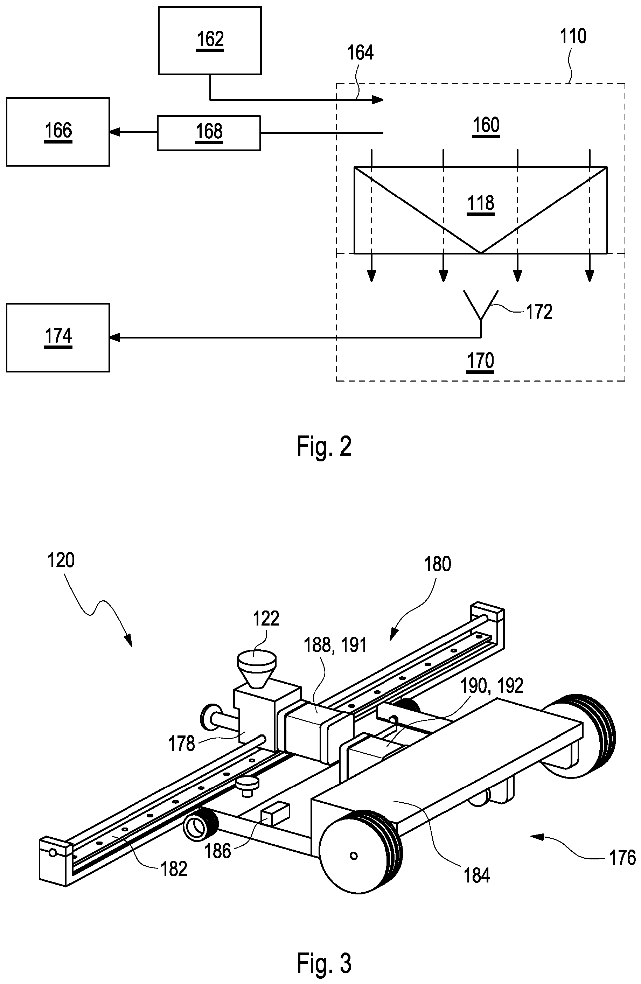

Weiterhin umfasst der Sterilisationstunnel 110 mindestens einen Schwebstofffilter 118 und mindestens eine zwischen dem Schwebstofffilter 118 und dem Transportband 114 angeordnete Partikelzählvorrichtung 120 wie sie im Folgenden noch näher beschrieben wird. In Figur 1 ist die Partikelzählvorrichtung 120 leidglich schematisch dargestellt. Für eine ausführliche Beschreibung der Partikelzählvorrichtung 120 wird auf die Beschreibung der Figuren 3 bis 14 verwiesen. Wie in Figur 1 dargestellt, kann die Partikelzählvorrichtung 120 insbesondere zumindest teilweise auf dem Transportband 114 angeordnet sein. Eine Sonde 122 der Partikelzählvorrichtung 120 weist mit einer Sondenöffnung 124, insbesondere einem Sondentrichter 126, dem Schwebstofffilter 118 zu.

Der Sterilisationstunnel 110 kann weiterhin mindestens einen Zuluftkanal 128 umfassen. Der Zuluftkanal 128 kann mindestens einen Ventilator 130 zum Ansaugen von Umgebungsluft umfassen. Der Sterilisationstunnel 110 kann weiterhin mindestens eine Absaugvorrichtung 132 umfassen. Die Absaugvorrichtung 132 kann eingerichtet sein, Luft unterhalb des Transportbands 114 abzusaugen.

Wie in Figur 1 gezeigt, kann der Sterilisationstunnel 110 insbesondere mindestens drei Zonen umfassen. Bei einer ersten Zone kann es sich um eine Anwärmzone 134 handeln. Die Anwärmzone 134 kann auch als Einlauf bezeichnet werden. Bei der zweiten Zone kann es sich um eine Sterilisationszone 136 handeln. Die Sterilisationszone 136 kann auch als Heißteil bezeichnet werden. Bei einer dritten Zone kann es sich um eine Abkühlzone 138 handeln. Die dritte Zone kann auch als Abkühlzone 138 bezeichnet werden. Das Transportband 114 kann eingerichtet sein, sich, insbesondere mit einer konstanten Geschwindigkeit, durch den Sterilisationstunnel 110, insbesondere durch die eine oder mehreren Zonen des Sterilisationstunnels 110, zu bewegen.

Die Anwärmzone 134 kann eingerichtet sein, die Gefäße langsam an eine Temperatur der Sterilisationszone 136 heranzuführen. Insbesondere kann die Anwärmzone 134 eingerichtet sein, Glas der Gefäße zu erwärmen, insbesondere langsam zu erwärmen, insbesondere um entstehende Spannungen in dem Glas zu reduzieren und einen möglichen Glasbruch zu vermeiden. Die Sterilisationszone 136 kann eingerichtet sein, Wasser an und in den Gefäßen zu verdampfen. Weiterhin kann die Sterilisationszone 136 eingerichtet sein, die Gefäße zu sterilisieren, insbesondere bei einer Temperatur von über 300 °C, vorzugsweise bei 330 °C. Die Abkühlzone 138 kann eingerichtet sein, die Gefäße abzukühlen, insbesondere langsam abzukühlen, insbesondere bis auf eine Temperatur von kleiner gleich 60 °C, insbesondere um aufgebaute Spannungen im Glas kontrolliert abzubauen. Die Medikamentenabfüllanlage 112 kann eingerichtet sein, die Gefäße von der Abkühlzone 138 in eine Abfüllanlage zu transportieren, welche in Figur 1 jedoch nicht dargestellt ist.

Die Anwärmzone 134 kann mindestens einen ersten Zuluftkanal 140, mindestens einen ersten Ventilator 142 und einen ersten Schwebstofffilter 144 aufweisen. Der erste Ventilator 142 und der erste Schwebstofffilter 144 können in dem Zuluftkanal 128, 140 angeordnet sein. Der erste Schwebstofffilter 144 kann auch als Vorfilter bezeichnet werden. Der erste Ventilator 142 kann eingerichtet sein, durch den ersten Schwebstofffilter 144 Umgebungsluft anzusaugen, insbesondere Umgebungsluft aus dem Reinraum. Der erste Ventilator 142 kann weiterhin eingerichtet sein, die angesaugte Umgebungsluft dem ersten Schwebstofffilter 144 zuzuführen. Der Zuluftkanal 128, 140 kann eingerichtet sein, die gefilterte Umgebungsluft als laminare Strömung bereitzustellen. Die gefilterte Luft kann einen Transportbandbereich des Sterilisationstunnels 110 durchströmen.

Die Absaugvorrichtung 132 kann mindestens einen weiteren Ventilator 146 umfassen. Der weitere Ventilator 146 kann insbesondere unterhalb des Transportbands 114 angeordnet sein. Der weitere Ventilator 146 kann eingerichtet sein, Luft, insbesondere feuchte Luft, unterhalb des Transportbands 114 abzusaugen.

Die Sterilisationszone 136 kann im Umlaufverfahren arbeiten. Die Sterilisationszone 136 kann mindestens einen zweiten Ventilator 148 und mindestens einen zweiten Schwebstofffilter 150 aufweisen. Der zweite Ventilator 148 kann eingerichtet sein, Luft von unterhalb des Transportbands 114 abzusaugen und in einen Bereich oberhalb des Transportbands 114 zu lenken. Die Sterilisationszone 136 kann weiterhin mindestens ein Heizaggregat aufweisen (nicht in Figur 1 dargestellt), welches eingerichtet ist, die Luft zu erwärmen. Der zweite Ventilator 148 kann weiterhin eingerichtet sein, die erwärmte Luft, insbesondere Heißluft, dem zweiten Schwebstofffilter 150 zuzuführen. Die Heißluft kann, insbesondere laminar, oberhalb des Transportbandbereichs austreten.

Der weitere Ventilator 146 kann eingerichtet sein, Luft, insbesondere mit Wasserdampf gesättigte Luft, in der Sterilisationszone 136 abzusaugen, insbesondere um zu verhindern, dass sich die mit Wasserdampf gesättigte Luft in der Sterilisationszone 136 anreichert. Die Sterilisationszone 136 kann weiterhin mindestens einen dritten Schwebstofffilter aufweisen, wobei der dritte Schwebstofffilter nicht in Figur 1 dargestellt ist. Über den dritten Schwebstofffilter kann Umgebungsluft bei Bedarf nachströmen.

Die Abkühlzone 138 kann mindestens einen dritten Ventilator 152 und mindestens einen vierten Schwebstofffilter 154 aufweisen. Der dritte Ventilator 152 kann eingerichtet sein, Umgebungsluft, insbesondere kühle Umgebungsluft, insbesondere kühle Reinraumluft, anzusaugen und diese durch den vierten Schwebstofffilter 154 zu drücken. Die austretende laminare Strömung kann eingerichtet sein, die Gefäße abzukühlen.

Der Sterilisationstunnel 110 kann weiterhin mindestens einen vierten Ventilator 156 und mindestens einen Ablaufkanal 158 aufweisen. Der vierte Ventilator 156 kann insbesondere ein Ventilator der Abkühlzone 138 sein. Der vierte Ventilator 156 kann eingerichtet sein, Luft unterhalb des Transportbands 114, insbesondere erwärmte Luft, insbesondere Luft in der Abkühlzone 138, abzusaugen und dem Abluftkanal 158 zuzuführen.

Die laminare Luftströmung in den einzelnen Zonen, insbesondere in der Anwärmzone 134, der Sterilisationszone 136 und der Abkühlzone 138, kann von oben nach unten strömen. Eventuell vorhandene Partikel im Sterilisationstunnel 110 werden dadurch nach unten gedrückt, wodurch grundsätzlich sichergestellt wird, dass keine Partikel in die Gefäße gelangen. Im Sterilisationstunnel 110, insbesondere im gesamten Sterilisationstunnel 110, kann außerdem ein Überdruck, insbesondere ein geringer Überdruck, herrschen, insbesondere damit keine Partikel in den Sterilisationstunnel 110 hineingelangen können.

Figur 2 zeigt einen schematischen Aufbau eines Lecktests in einem Sterilisationstunnel 110. Der Aufbau kann insbesondere in einem Verfahren zum Partikelzählen in einem Sterilisationstunnel 110 durchgeführt werden, wie es im Folgenden, beispielsweise in den Figuren 17A bis 17C, noch näher beschrieben wird. Der Lecktest kann in einem ersten und/oder einem zweiten Betriebszustand des Sterilisationstunnels 110 durchgeführt werden. Der erste Betriebszustand kann insbesondere als "at rest" (englisch: "in Ruhe") bezeichnet werden. Bei "at rest" kann sich der gesamte Sterilisationstunnel 110 bei der Durchführung des Verfahrens zum Partikelzählen in einem kalten Zustand befinden. Insbesondere können die Ventilatoren 130 für eine Luftversorgung des Sterilisationstunnels 110 in Betrieb sein. Eine Heizung kann jedoch abgeschaltet sein und es können sich insbesondere keine Gefäße auf dem Transportband 114 befinden. Der zweite Betriebszustand kann als "in operation" (englisch: "in Betrieb") bezeichnet werden. Bei "in operation" kann eine Reinraumklassenbestimmung unter Produktionsbedingungen durchgeführt werden. Der Sterilisationstunnel 110 kann sich in einem heißen Zustand befinden. Die Ventilatoren 130 für die Luftversorgung des Sterilisationstunnel 110 können laufen, die Heizung kann angeschaltet sein und es können sich ebenfalls keine Gefäße auf dem Transportband 114 befinden.

Das Verfahren zum Partikelzählen wird grundsätzlich im Anlagenzustand "at rest" durchgeführt. Der Schwebstofffilter 118, insbesondere der HEPA-Filter, kann bei einem Nennvolumenstrom auf einer Rohluftseite 160 beaufschlagt werden. Unter einem Nennvolumenstrom ist grundsätzlich eine Luftmenge gemeint, bei welcher der Schwebstofffilter 118 unter Betriebsbedingungen im Sterilisationstunnel 110 eingesetzt sein kann. Eine Luft im Zuluftkanal 128 vor dem Schwebstofffilter 118 kann als Rohluft definiert werden. Eine Luft nach einer Durchströmung eines Schwebstofffilters 118 kann als Reinluft definiert werden.

Bei der Durchführung des Verfahrens zum Partikelzählen, kann ein Aerosolgenerator 162 ein konstantes Testaerosol mit definierten Eigenschaften erzeugen. Eine Partikelkonzentration kann sich über einen regelbaren Durchflussmesser mit Nadelventil einstellen lassen. Als Partikelmaterial kann Di-2-ethylhexyl-Sebacat (DEHS) zum Einsatz kommen. Vor dem Schwebstofffilter 118, auf der Rohluftseite 160, kann dieses Testaerosol über einen Prüfstutzen 164 eingeleitet werden. Die Partikelkonzentration auf der Rohluftseite 160 kann von einem Partikelzähler 166 mit einer davor geschalteten Verdünnungsstufe 168 überwacht werden. Die Verdünnungsstufe 168 kann die angesaugten Partikel mit einem Verdünnungsfaktor 1:1000 reduzieren. Der Partikelzähler 166 kann die Luft mit den Partikeln ansaugen, deren Größe und Anzahl messen und diese auswerten. Ein Sensor des Partikelzählers 166 kann Partikel mit einer Größe von 0,3 $\mu m$ bis 10 $\mu m$ messen und zählen. Die Verdünnungsstufe 168 kann notwendig sein, da der Partikelzähler 166 grundsätzlich eine maximale Konzentration der Partikel messen kann und diese Konzentration ohne Verdünnungsstufe überschritten werden kann.

Auf einer Reinluftseite 170 kann die Partikelanzahl mit einer definierten isokinetischen Sonde 172, welche beispielsweise die Sonde 122 der Partikelzählvorrichtung 120 sein kann, deren Rohr mit einem geeigneten Schlauch mit einem weiteren Partikelzähler 174 verbunden ist, gemessen werden. Während der Messung kann die gesamte Filterfläche schrittweise abgefahren werden, wie nachfolgend noch näher erläutert wird. Der weitere Partikelzähler 174 kann die austretende Reinluft über die Sonde ansaugen und eine gemessene Partikelanzahl auswerten.

Im Rahmen des Verfahrens zum Partikelzählen kann die definierte reinluftseitige Partikelanzahl pro Messvorgang abhängig sein von der Partikelanzahl auf der Rohluftseite 160. Die genauen Verhältnisse von Partikelanzahl auf der Rohluftseite 160 und erlaubter Partikelanzahl auf der Reinluftseite 170 können firmenintern beschrieben und festgelegt sein. Durch eine Überprüfung der Funktion des Schwebstofffilters 118 kann sichergestellt werden, dass auch bei einer definiert erhöhten Anzahl der Partikel auf der Rohluftseite 160, die Reinraumzone innerhalb des Sterilisationstunnels 110 nicht verschmutzt, insbesondere nicht punktuell verschmutzt, wird.

Figuren 3 bis 14 zeigen eine Ausführungsform der Partikelzählvorrichtung 120 oder Teile davon in verschiedenen Ansichten. Figur 3 zeigt eine perspektivische Ansicht der Partikelzählvorrichtung 120. Die Partikelzählvorrichtung 120 zum Partikelzählen in dem Sterilisationstunnel 110 der Medikamentenabfüllanlage 112, wobei der Sterilisationstunnel 110, wie in Figur 1 exemplarisch gezeigt, das mindestens eine Transportband 114 umfasst, umfasst die mindestens eine mit dem Partikelzähler 174 verbindbare Sonde 122 zum Aufnehmen von Partikeln in dem Sterilisationstunnel 110. Weiterhin umfasst die Partikelzählvorrichtung 120 mindestens einen Scanner 176 mit mindestens einem Sondenhalter 178 zum Befestigen der Sonde 122. Der Scanner 176 umfasst mindestens einen Querläufer 180 mit mindestens einer Linearführung 182. Die Linearführung 182 ist eingerichtet, den Sondenhalter 178 quer, insbesondere im Wesentlichen senkrecht, zu der Transportrichtung 116 des Transportbands 114 des Sterilisationstunnels 110 zu führen. Weiterhin umfasst der Scanner 176 mindestens ein Fahrgestell 184. Der Querläufer 180 ist auf dem Fahrgestell 184 befestigt. Das Fahrgestell 184 ist eingerichtet, die Linearführung 182 in der Transportrichtung 116 des Transportbands 114 zu bewegen. Weiterhin umfasst der Scanner 176 mindestens eine Steuerung 186, insbesondere eine mit dem Fahrgestell 184 verbundene Steuerung 186, wobei die Steuerung 186 eingerichtet ist, um eine Bewegung des Scanners 176 zu steuern.

Das Fahrgestell 184 kann eingerichtet sein, sich und den Querläufer 180, insbesondere den Querläufer 180 mit der Sonde 122, in einem zweidimensionalen Raum zu bewegen. Die Partikelzählvorrichtung 120 kann insbesondere jeweils einen Antrieb für eine Führung des Sondenhalters 178 quer (gekennzeichnet durch Bezugszeichen 188), insbesondere im Wesentlichen senkrecht, zu der Transportrichtung 116 des Transportbands 114 des Sterilisationstunnels 110 und für eine Bewegung des Fahrgestells 184 (gekennzeichnet durch Bezugszeichen 190) entlang der Transportrichtung 116 des Transportbandes 114 umfassen. Beide Antriebe 188, 190 können jeweils mit Hilfe eines Motors, beispielsweise mit einem ersten Motor 191 und einem zweiten Motor 192, bewegt werden, wie nachfolgend noch näher ausgeführt wird. Insbesondere kann die Partikelzählvorrichtung 120 derart ausgestaltet sein, dass die Bewegung des Fahrgestells 184 unabhängig ist von einer Führung des Sondenhalters 178.

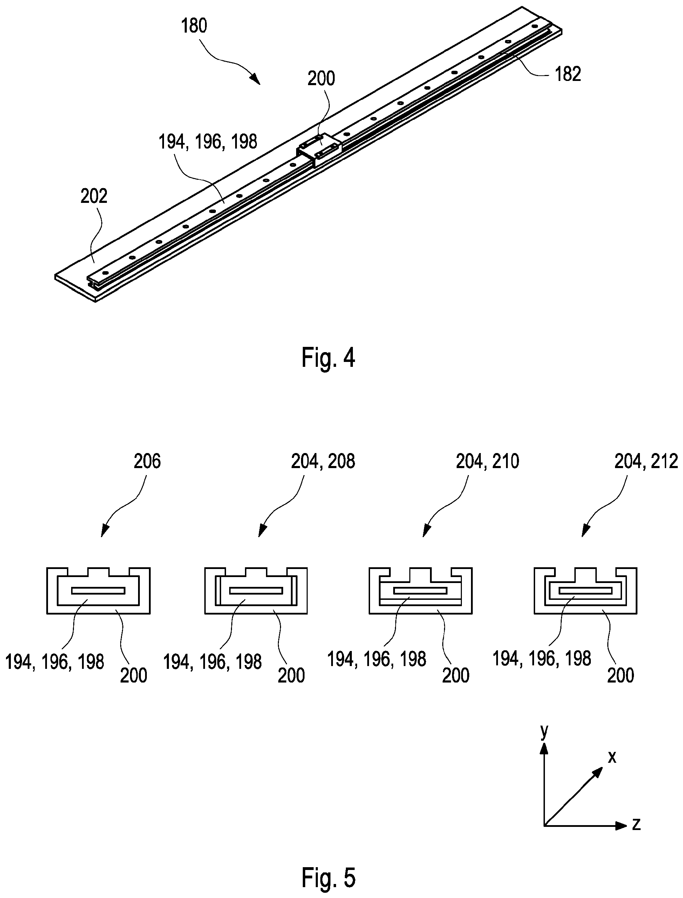

Figur 4 zeigt eine perspektivische Detailansicht des Querläufers 180. Wie in Figur 4 zu sehen ist, kann die Linearführung 182 des Querläufers 180 mindestens eine Führungsschiene 194, insbesondere eine profilierte Führungsschiene 196, insbesondere eine T-Führungsschiene 198, umfassen. Alternativ und/oder zusätzlich kann die Linearführung 182 auch eine Rundwelle umfassen. Weiterhin kann die Linearführung 182 mindestens einen Führungsschlitten 200 umfassen, insbesondere mindestens einen auf der Führungsschiene 194 oder der Rundwelle gelagerten Führungsschlitten 200. Die Sonde 122 kann an dem Führungsschlitten 200 befestigbar oder befestigt sein, insbesondere mittels des Sondenhalters 178. Die Linearführung 182 kann daher eingerichtet sein, die Sonde 122 zu führen. Die Linearführung 182, insbesondere die Führungsschiene 194, kann auf einer Grundplatte 202, insbesondere auf einer Grundplatte 202 aus Aluminium, befestigt sein, insbesondere mittels einer Schraubverbindung. Die Grundplatte 202 kann austauschbar ausgeführt sein. Sollte die Führungsschiene 194 Verschleiß aufweisen, kann sie bei Bedarf jederzeit ausgetauscht werden. Die Linearführung 182 kann mittels der Grundplatte 202 auf dem Fahrgestell 184 befestigt sein.

Die Linearführung 182 kann insbesondere mindestens ein Gleitlager aufweisen, insbesondere für den Führungsschlitten 200. Das Gleitlager kann schmiermittelfrei ausgestaltet sein. Andere Lager wie Kugel- oder Rollenlager sind jedoch ebenfalls grundsätzlich denkbar. Durch die T-Führungsschiene 198 ist es grundsätzlich möglich, dass der Führungsschlitten 200 mit Loslager 204 in der Richtung quer, insbesondere senkrecht, zu der Transportrichtung 116 des Transportbands 114 und/oder in der Transportrichtung 116 des Transportbands 114 ausgeführt werden kann. Mögliche Ausführungen des Loslagers 204 sind in Figur 5 gezeigt. Hierin sind kein Loslager 206, ein Loslager in z-Richtung 208, ein Loslager in y-Richtung 210 und ein Loslager in yz-Richtung 212 gezeigt.

In diesem Ausführungsbeispiel umfasst die Linearführung 182 das Loslager in y-Richtung 210. Die anderen dargestellten Loslager 204 sind jedoch ebenfalls möglich. Das Loslager 204 ermöglicht, dass der Führungsschlitten 200 in die gewählte Richtung etwas Spiel hat. So können Fertigungstoleranzen in der Konstruktion ausgeglichen werden. Ohne Loslager 204 könnte man ein starres System erhalten, wodurch beispielsweise ein Verkanten des Führungsschlittens 200 möglich wäre. Insbesondere kann die Führungsschiene 194 als Loslager 204 eingerichtet sein, insbesondere als Loslager 204 in einer Richtung quer, insbesondere senkrecht, zu der Transportrichtung 116 des Transportbands 114 (gekennzeichnet durch Bezugszeichen 210). Hierdurch kann der Führungsschlitten 200 in der Lage sein, kleine Höhenunterschiede, insbesondere in einem Gesamtsystem, auszugleichen. Eine Länge des Führungsschlittens 200 kann einer Flanschbreite eines Motors 192 entsprechen.

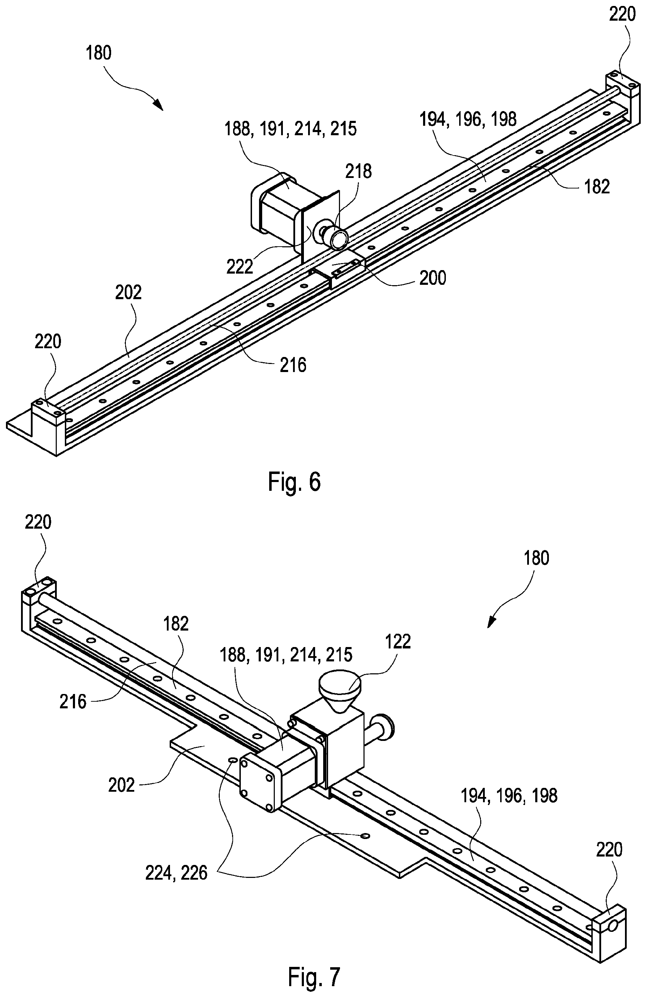

In Figur 6 ist eine weitere perspektivische Detailansicht des Querläufers 180 gezeigt. Wie oben ausgeführt, kann die Partikelzählvorrichtung 120 einen Antrieb 188 für eine Führung des Sondenhalters 178 umfassen. Beispielsweise kann die Linearführung 182 den mindestens einen Antrieb 188, insbesondere einen linearen Antrieb, aufweisen. Der Antrieb 188 kann eingerichtet sein, den Führungsschlitten 200 auf der Führungsschiene 194 zu bewegen. Der Antrieb 188 kann eingerichtet sein, eine gesamte Transportbandbreite des Transportbands 114 des Sterilisationstunnels 110 abzufahren. Wie in Figur 6 zu sehen, kann der Antrieb 188 einen Zahnstangenantrieb 214 umfassen. Auch andere Ausführungsformen, wie ein Spindelantrieb und/oder ein Zahnriemenantrieb, sind jedoch grundsätzlich denkbar. Der Antrieb 188 kann insbesondere einen Schrittmotor 215 umfassen.

Der Zahnstangenantrieb 214 kann mindestens eine Zahnstange 216 und mindestens ein Stirnzahnrad 218 aufweisen. Der Querläufer 180 kann weiterhin mindestens eine, vorzugsweise mindestens zwei, Zahnstangenhalterungen 220 aufweisen. Die Zahnstangenhalterung 220 kann eingerichtet sein, die Zahnstange 216 zu fixieren, insbesondere auf der Grundplatte 202 des Querläufers 180. Die Zahnstangenhalterung 220 kann insbesondere aus Aluminium hergestellt sein. Der Zahnstangenantrieb 214 kann insbesondere eingerichtet sein, mittels einer Drehbewegung des Stirnzahnrads 218 den Führungsschlitten 200 über die Zahnstange 216, welche insbesondere auf der Grundplatte 202 des Querläufers 180 fixiert sein kann, zu bewegen. Das Stirnzahnrad 218 kann insbesondere aus Polyoxymethylene (POM) hergestellt sein. Ein Befestigungswinkel 222, insbesondere aus Aluminium, kann an Gewindebohrungen des Führungsschlittens 200 befestigt, insbesondere verschraubt, werden. Der Schrittmotor 215 kann ebenfalls am Befestigungswinkel 222 befestigt, insbesondere verschraubt, werden.

Eine weitere perspektivische Detailansicht des Querläufers 180 ist in Figur 7 dargestellt. Wie in Figur 7 zu sehen ist, kann die Grundplatte 202 auf dem Fahrgestell 184 mittels mindestens einer Schraubverbindung 224 befestigt sein. Andere Verbindungsarten, wie beispielsweise eine Klick-Verbindung und/oder eine Spannhebelverbindung, sind jedoch ebenfalls möglich.

Die Schraubverbindung 224 kann insbesondere Rändelschrauben und/oder Zylinderkopfschrauben umfassen. Insbesondere kann die Grundplatte 202 eine Vielzahl von Bohrungen 226 umfassen, insbesondere um den Querläufer 180 auf dem Fahrgestell 184 zu befestigen. Aufgrund einer hohen Anzahl von jährlich notwendigen Wechseln des Querläufers 180, ca. sechs Mal pro Jahr, kann eine Befestigung des Querläufers 180 auf dem Fahrgestell 184 mit Rändelschrauben vorteilhaft sein Dadurch lässt sich eine kompakte Konstruktion erstellen. So ist die Anforderung des werkzeugfreien Wechsels grundsätzlich gegeben und der Querläufer 180 kann fest, aber lösbar, mit dem Fahrgestell 184 verbunden sein. Wie in Figur 7 dargestellt, kann die Grundplatte 202, insbesondere um Gewicht einzusparen, durch Ausklinken von nicht benötigtem Material angepasst werden. Auch weitere Ausführungsformen für eine Befestigung des Querläufers 180 auf dem Fahrgestell 184 sind grundsätzlich denkbar, wie Klick-Systeme oder Spannhebel.

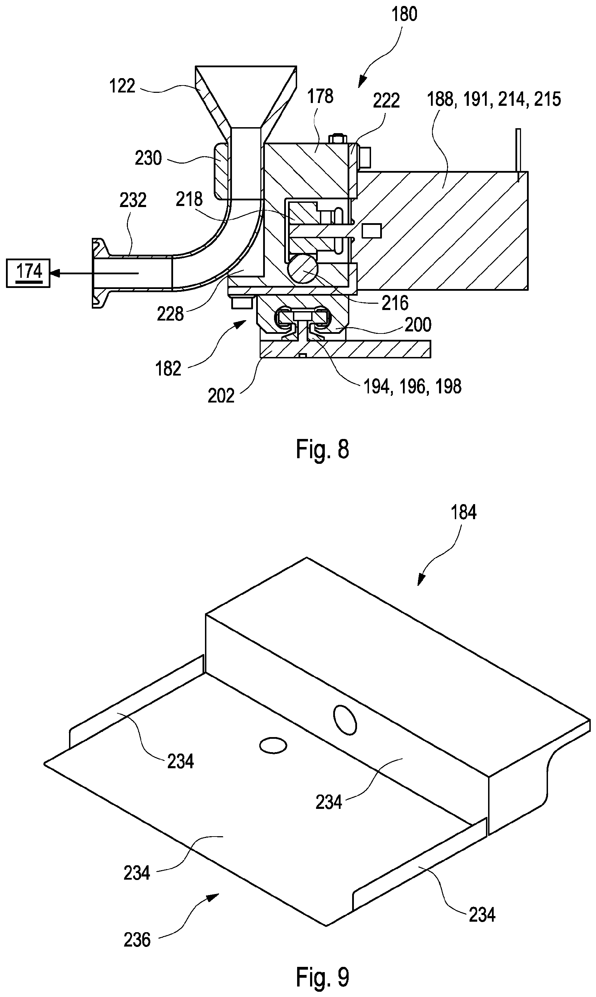

In Figur 8 ist der Querläufer 180 in einer seitlichen Schnittdarstellung gezeigt. Wie in dieser Ansicht zu sehen ist, kann der Sondenhalter 178 mindestens eine Nut 228 für eine Aufnahme der Sonde 122 aufweisen. Weiterhin kann der Sondenhalter 178 mindestens eine Klemmplatte 230 aufweisen, welche eingerichtet ist, die Sonde 122 zu fixieren. Alternativ und/oder zusätzlich kann der Sondenhalter 178 auf dem Führungsschlitten 200 befestigt sein.

Weiterhin ist in Figur 8 zu sehen, dass die Zahnstange 216 insbesondere einen runden Querschnitt aufweisen kann. Die Zahnstange 216 kann mittig über der Linearführung 182 angeordnet sein.

Wie in Figur 8 schematisch angedeutet, kann die Partikelzählvorrichtung 120 weiterhin den mindestens einen mit der Sonde 122 verbindbaren Partikelzähler 174 umfassen. Insbesondere kann der Partikelzähler 174 als stationärer Partikelzähler ausgestaltet sein und der Partikelzähler 174 und die Sonde 122 können mittels mindestens einer Rohrleitung 232, insbesondere einer flexiblen Rohrleitung, miteinander verbunden sein. Die Rohrleitung 232 kann dabei Teil des Partikelzählers 174 und/oder Teil der Partikelzählvorrichtung 120 sein.

In Figur 9 ist das Fahrgestell 184 der Partikelzählvorrichtung 120 in einer perspektivischen Ansicht gezeigt. Das Fahrgestell 184 kann insbesondere einen Rahmen 234 aufweisen. Der Rahmen 234 kann auch als Grundgestell bezeichnet werden. Der Rahmen 234 kann insbesondere aus einem Blech, insbesondere aus einem Blech aus einem austenitischen nichtrostendem Stahl, hergestellt sein, Das Blech kann insbesondere eine Stärke von 1 mm bis 5 mm, vorzugsweise von 1,5 mm bis 2,5 mm und besonders bevorzugt von 2 mm aufweisen. Wie oben ausgeführt, ist der Querläufer 180 auf dem Fahrgestell 184 befestigt. Insbesondere kann der Querläufer 180, insbesondere die Grundplatte 202 des Querläufers 180, auf dem Rahmen 234 befestigt sein. Insbesondere kann der Querläufer 180 mittig auf dem Fahrgestell 184, insbesondere auf dem Rahmen 134, befestigt sein. Weiterhin kann der Querläufer 180 bündig auf Fahrgestell 184, insbesondere auf dem Rahmen 234, befestigt sein. Insbesondere kann der Querläufer 180 bündig an einer Hinterseite 236 des Fahrgestells 184, insbesondere des Rahmens 234, befestigt sein. Aufgrund von geringen Auslaufflächen vor und nach dem Sterilisationstunnel 110, kann eine Baugröße somit minimiert werden und ein Partikelzählen kann selbst ohne Auslaufzonen realisiert werden.

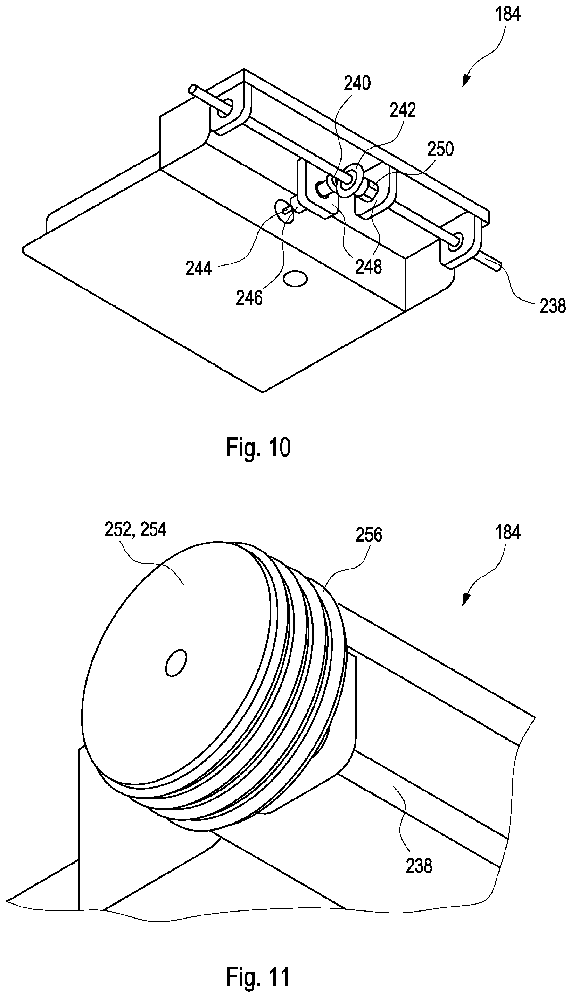

Figur 10 zeigt eine weitere perspektivische Ansicht des Fahrgestells 184. Der zweite Motor 192 der Partikelzählvorrichtung 120 kann beispielsweise mindestens einen Schrittmotor umfassen. Auch andere Arten von Motoren, wie beispielsweise Servomotoren, sind jedoch grundsätzlich denkbar. Insbesondere kann der zweite Motor 192 ein Schrittmotor NEMA 17 (Stepperonline, China) mit einem zugehörigen Schrittmotortreiber sein. Der Schrittmotor Nema17 kann trotz seiner geringen Baugröße geeignet sein, eine erforderliche Leistung zum Antrieb der Bewegung des Fahrgestells 184 aufzubringen. Für weitere Details wird auf obige Beschreibung verwiesen.

Das Fahrgestell 184 kann insbesondere eine Antriebsachse, welche auch als Radwelle 238 bezeichnet wird, aufweisen. Der Schrittmotor kann quer, insbesondere um 90°, versetzt zu der Antriebsachse am Grundgestell befestigt sein. Das Fahrgestell 184 kann weiterhin mehrere Kegelräder aufweisen. Um eine Drehbewegung des Schrittmotors auf die Antriebsachse zu übertragen, können Kegelräder mit einer Übersetzung 2:1 gewählt werden. Ein erstes Kegelrad 240 kann beispielsweise eine Zähnezahl z von 15 und ein zweites Kegelrad 242 kann eine Zähnezahl z von 30 aufweisen. Das erste Kegelrad 240 und das zweite Kegelrad 242 können jeweils aus POM hergestellt sein und ein Modul m von 1 aufweisen.

Das erste Kegelrad 240 kann eingerichtet sein, von dem zweiten Motor 192 angetrieben zu werden und eine Drehbewegung über das zweite Kegelrad 242 auf die Antriebsachse zu übertragen. Durch das Übersetzungsverhältnis kann eine Drehzahl $n_1$ des zweiten Motors 192 an der Antriebsachse halbiert werden und das übertragene Drehmoment $M_1$ verdoppelt werden. Daraus resultierend kann grundsätzlich ein Schrittmotor mit kleinem Haltemoment verwendet werden.

Zur Übertragung des Drehmoments und der Drehzahl vom zweiten Motor 192 auf die Welle 244 des ersten Kegelrads 240 kann eine drehstarre, aber winkel- und quer-nachgiebige Ausgleichskupplung 246 verwendet werden. Hierdurch können grundsätzlich Toleranzen oder Fluchtungsfehler in der Konstruktion ausgeglichen werden. Eine verwendete Ausgleichskupplung 246 kann grundsätzlich spielfrei und drehsteif sein und sowohl radialen als auch axialen Winkelversatz ausgleichen. Wellen der Zahnräder können durch einen Halter 248 in eine passende Position gehalten werden. In dem Halter 248 können Rillenkugellager eingepresst sein, welche die Welle 244 des ersten Kegelrads 240 und die Radwelle 238 lagern und einen leichtgängigen Lauf gewährleisten. Rillenkugellager haben grundsätzlich den Vorteil, dass sie beim Anlaufen kein erhöhtes Reibmoment haben. Auch weisen sie bei niedrigen Drehzahlen grundsätzlich einen geringen Verschleiß auf und sind wartungsfrei. Das erste Kegelrad 240 kann kraftschlüssig mit einem Gewindestift auf der Welle 244 befestigt werden. Das zweite Kegelrad 242 kann ebenfalls mit einem Gewindestift auf der Radwelle 238 befestigt werden und kann zusätzlich mit einem Stellring 250 gegen unabsichtliches Verschieben auf der Radwelle 238 gesichert werden.

Wie oben ausgeführt, ist das Fahrgestell 184 eingerichtet, die Linearführung 182 in der Transportrichtung 116 des Transportbands 114 zu bewegen. Wie in Figur 11 in einer perspektivischen Ansicht gezeigt, kann das Fahrgestell 184 mindestens zwei Räder 252, insbesondere mindestens zwei Antriebsräder 254, aufweisen, welche eingerichtet sind, die Linearführung 182 in der Transportrichtung 116 des Transportbands 114 zu bewegen. In diesem Ausführungsbeispiel kann der zweite Motor 192 eingerichtet sein, mindestens eines der Antriebsräder 254 anzutreiben, insbesondere über die Radwelle 238.

Insbesondere können die Räder 252 zumindest teilweise aus einem Elastomer hergestellt sein. Das Elastomer kann ausgewählt sein aus einer Gruppe bestehend aus: Silikon, Ethylen-Propylen-Dien-(Monomer)-Kautschuk (EPDM). Auch andere Elastomere sind jedoch grundsätzlich denkbar. Insbesondere können die Räder 252 jeweils ein oder mehrere O-Ringe 256 aufweisen, welche aus dem Elastomer hergestellt sind. Das Elastomer kann eingerichtet sein, eine Rollreibung zwischen den Rädern 252 und dem Transportband 114 zu erzeugen. Durch eine elastische Verformbarkeit und einen hohen Reibwert der Elastomere kann eine Kontaktfläche zwischen den O-Ringen 256 und dem Transportband 114 erhöht sein. Somit kann ein schlupffreier Betrieb möglich sein.

Die Räder 252 können insbesondere derart ausgestaltet sein, dass die O-Ringe 256 aus dem Elastomer daran befestigt werden können. Die Räder 252, insbesondere die Antriebsräder 254, können insbesondere aus Polyoxymethylene (POM) hergestellt sein. Insbesondere können die Räder 252 eine oder mehrere Nuten aufweisen, welche für eine Aufnahme der O-Ringe eingerichtet sind. Ein Durchmesser der Räder 252 kann so gewählt werden, dass eine geringe Bodenfreiheit zwischen dem Rahmen 234 und dem Transportband 114 erreicht wird. So kann eine spätere Gesamthöhe des Scanners 176 klein gehalten werden. Die Räder 252, insbesondere die Antriebsräder 254, können jeweils mehrere O-Ringe 256 aufweisen. In diesem Ausführungsbeispiel weisen die Räder 252 vorzugsweise mindestens vier O-Ringe 256 auf, insbesondere um die Kontaktfläche zwischen den Rädern 252 und dem Transportband 114 zu erhöhen. Die O-Ringe 256 können voneinander beabstandet auf mindestens einer Umfangsfläche der Antriebsräder 254 angeordnet sein. Die O-Ringe 256 können jeweils in Nuten der Umfangsfläche der Antriebsräder 254 aufgenommen sein.

Die Antriebsräder 254 können jeweils auf der Radwelle 238 befestigt sein. Eine Drehbewegung der Radwelle 238 auf die Antriebsräder 254 kann formschlüssig mit Passfedern übertragen werden. So kann ein Durchdrehen der Räder 252 verhindert werden. Zusätzlich können die Antriebsräder 254 mit einem Gewindestift gesichert werden, insbesondere gegen ein Verschieben auf der Radwelle 238.

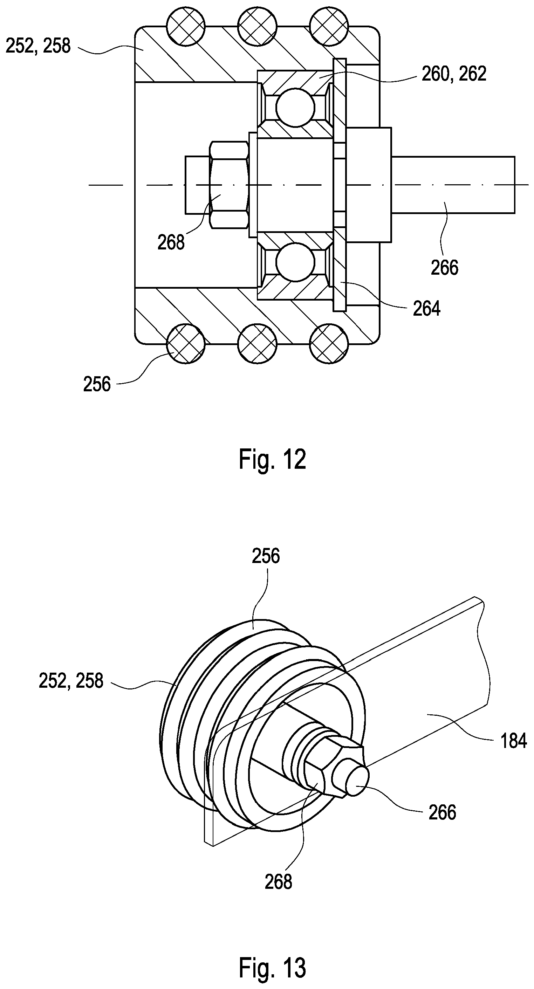

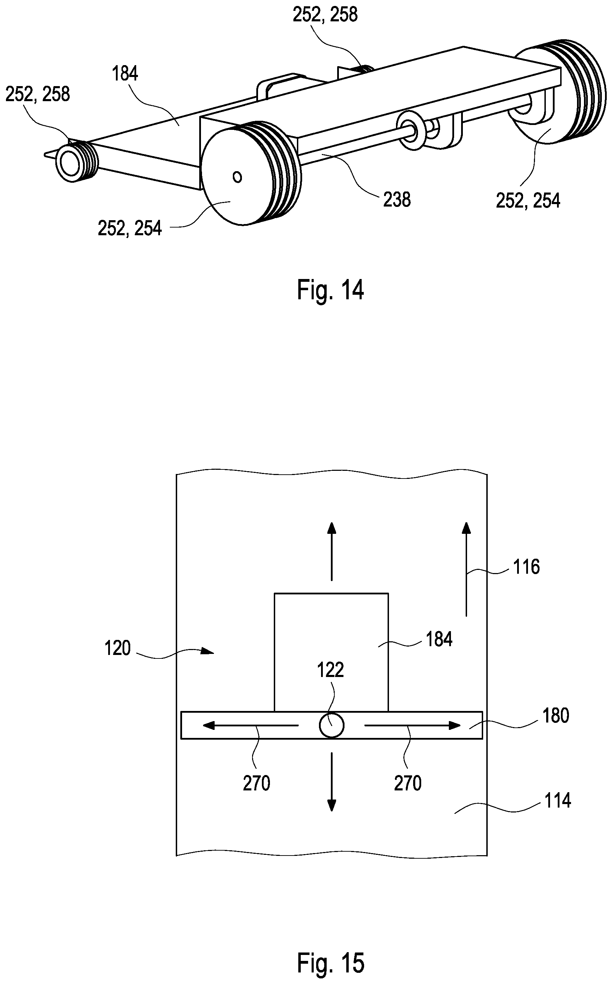

Die Räder 252 können weiterhin ein oder mehrere, insbesondere zwei, Hinterräder 258 umfassen. In Figur 12 ist eine Schnittdarstellung eines der Hinterräder 258 gezeigt. Eine perspektivische Ansicht der Hinterräder 258 ist in Figur 13 dargestellt. Das Fahrgestell 184 inklusive der Räder 252, insbesondere inklusive der Antriebsräder 254 und der Hinterräder 258, ist in Figur 14 in einer perspektivischen Ansicht zu sehen.

Die Hinterräder 258 können antriebsfrei ausgestaltet sein. Die Hinterräder 258 können insbesondere einen leichtgängigen Lauf aufweisen. Für weitere Details der Ausgestaltung der Hinterräder 258 wird auf obige Beschreibung, insbesondere auf die Beschreibung der Antriebsräder 254, verwiesen. Die Hinterräder 258 können aus POM hergestellt sein. Weiterhin können die Hinterräder 258 Nuten aufweisen, welche eingerichtet sein können, O-Ringe 256 aufzunehmen. Die O-Ringe 256 können aus mindestens einem Elastomer hergestellt sein. Ein Durchmesser der Hinterräder 258 kann so gewählt sein, dass die Hinterräder 258 keine Behinderung für die Bewegung des Querläufers 180 darstellen. In die Hinterräder 258 können, insbesondere für einen leichtgängigen Lauf, Kugellager 260, insbesondere Rillenkugellager 262, eingepresst sein. Die Hinterräder 258 können einen Sicherungsring 264 aufweisen, welcher eingerichtet ist, das Kugellager 260, insbesondere das Rillenkugellager 262, gegen ein unbeabsichtigtes Lösen zu sichern. Die Hinterräder 258 können jeweils auf eine Welle 266 geschoben und mit einer selbstsichernden Mutter 268 befestigt sein. Die selbstsichernde Mutter 268 kann eingerichtet sein, ein Lösen der selbstsichernden Mutter 268 bei einer Drehbewegung des Hinterrads 258 zu vermeiden. Die Wellen 266 der Hinterräder 258 können weiterhin jeweils mit selbstsichernden Muttern 268 am Fahrgestell 184 befestigt, insbesondere verschraubt, sein.

In Figur 15 ist eine schematische Draufsicht der Partikelzählvorrichtung 120 in einem Sterilisationstunnel 110 dargestellt. Eine Vorgabe für Lecktests ist grundsätzlich, dass die Filterfläche der Schwebstofffilter 118 in teilweise überlappenden Bahnen abgefahren werden soll. Um den geforderten Verfahrweg der Sonde 122 zu erreichen, können zumindest zwei Bewegungsrichtungen notwendig sein. Die Partikelzählvorrichtung 120 kann eingerichtet sein, die Sonde 122 entlang einer Transportbandbreite des Transportbands 114 (gekennzeichnet durch Pfeile 270), bevorzugt mit einer konstanten Geschwindigkeit, zu bewegen. Zusätzlich kann die Partikelzählvorrichtung 120 eingerichtet sein, eine schrittweise Bewegung in die Transportrichtung 116 des Transportbandes 114 durchzuführen. Diese Schritte können insbesondere derart eingestellt werden, dass die geforderte Überlappung eingehalten wird. Nach Beendigung des Partikelzählens kann die Partikelzählvorrichtung 120 wieder an den Ausgangspunkt zurückfahren.

Der Antrieb 190 des Fahrgestells 184 kann die Bewegung der gesamten Partikelzählvorrichtung 120 in Transportrichtung 116 des Transportbandes 114 antreiben. Das Fahrgestell 184 kann dabei das Transportband 114, und somit den über ihm befindlichen Schwebstofffilter 118, der Länge nach abfahren und kann daher für Sterilisationstunnel 110 jeglicher Art und Größe verwendet werden. Um die Partikelzählvorrichtung 120, insbesondere umfassend das Fahrgestell 184 und den Querläufer 180, auch im Sterilisationstunnel 110 mit kleineren Transportbandbreiten, beispielsweise von bis zu 600 mm, gut ausrichten zu können, kann eine Breite des Fahrgestells 184 diese Breite nicht überschreiten.

Die Breite der Schwebstofffilter 118 können durch eine Bewegung der Sonde 122 quer, insbesondere orthogonal, zur Transportrichtung 116 des Transportbandes 114, entlang der Transportbandbreite abgefahren werden. Dies kann insbesondere mittels des Querläufers 180 ermöglicht werden. Die Breite der Schwebstofffilter 118 entspricht grundsätzlich der Breite des Transportbandes 114 oder kleiner. Die Breite des Transportbandes 114 und damit auch die Abmessungen der Schwebstofffilter 118 können je nach Art des Sterilisationstunnels 110 variieren. Eine Länge des Querläufers 180 kann daher kleiner sein als die Breite des Transportbandes 114, da insbesondere der Abstand von einer Seitenbegrenzung des Transportbandes 114 zu einer Seitenwand des Sterilisationstunnels 110 teilweise nur wenige Millimeter beträgt. Den unterschiedlichen Breiten der verschiedenen Sterilisationstunnel 110 kann durch einen austauschbaren Querläufer 180 begegnet werden, um in jedem Sterilisationstunnel 110 die gesamte Breite abfahren zu können. Somit kann die Partikelzählvorrichtung 120 an verschiedene Breiten der Schwebstofffilter 118 flexibel angepasst werden. Ein Wechsel des Querläufers 180 kann ohne Verwendung von Werkzeugen durchführbar sein. Die Komponenten des Querläufers 180 können insbesondere derart gewählt werden, dass die Sonde 122 eine vorgegebene Verfahrgeschwindigkeit von 5,9 cm/s erreichen und konstant gehalten werden kann. Die Sonde 122 kann außerdem so nahe wie konstruktiv möglich an die Außenbegrenzungen auf beiden Seiten des Transportbandes 114 herangefahren werden, um zu gewährleisten, dass eine möglichst große Fläche abgefahren werden kann. Eine maximale Durchfahrtshöhe im Sterilisationstunnel 110 kann ebenfalls variieren. Die Partikelzählvorrichtung 120 kann für den Einsatz in allen Sterilisationstunneln 110 geeignet sein, da eine maximale Bauhöhe der Partikelzählvorrichtung 120 die kleinste maximale Durchfahrtshöhe von 160 mm nicht überschreitet.

In Figur 16 sind geometrische Abmessungen der Partikelzählvorrichtung 120, insbesondere eines mäandrierenden Verfahrwegs 272 der Partikelzählvorrichtung 120, in einem Sterilisationstunnel 110 gezeigt.

Die Filterfläche der Schwebstofffilter 118 kann hierbei mit einer kreisrunden, isokinetischen Sonde 122 mit einem Durchmesser D von 36 mm in teilweise überlappenden Bahnen abgefahren werden. Die Überlappung der Bahnen ist im Allgemeinen in Standardrichtlinien mit 6 mm festgelegt und soll bei der Bewegung der Sonde 122 in die nächste Bahn als Bahnabstand berücksichtigt werden. Zur einfacheren Berechnung wird eine fiktive Rechtecksonde mit den Kantenlängen $Wp$ und $Dp$ betrachtet. Zur Berechnung der Anzahl der abzufahrenden Bahnen pro Schwebstofffilter 118 und zur Berechnung der Messdauer werden die Kantenlängen $Wp$ und $Dp$ benötigt. Die Kantenlänge $Wp$ kann dabei dem Bahnabstand, um den sich die Partikelzählvorrichtung 120 schrittweise nach vorne bewegen soll, entsprechen. Die Kantenlänge $Dp$ ist der Abstand von Schnittpunkten der sich um 6 mm überlappenden Sondenbahnen.

Die Kantenlänge $Wp$ kann bestimmt werden zu:

$$W_p = D - 6\ mm \tag{30}$$

$$W_p = 36\ mm - 6\ mm \tag{31}$$

$$W_p = 30\ mm \tag{32}$$

Die Kantenlänge $Dp$ kann bestimmt werden zu:

$$D_p = 2 * \sqrt{\left(\frac{D}{2}\right)^2 - \left(\frac{D-6\ mm}{2}\right)^2} \tag{33}$$

$$D_p = 2 * \sqrt{\left(\frac{36\ mm}{2}\right)^2 - \left(\frac{36\ mm-6\ mm}{2}\right)^2} \tag{34}$$

$$D_p = 2 * \sqrt{18\ mm^2 - 15\ mm^2} \tag{35}$$

$$D_p = 2 * 9{,}95\ mm \tag{36}$$

$$D_p = 19{,}9\ mm \tag{37}$$

Weiterhin kann eine Messdauer t pro Schwebstofffilter 118, insbesondere pro Filterelement, berechnet werden. Ein maximal zulässiger Seitenabstand $a$ der Sonde 122 zu einer Außenkante des Transportbandes 114 kann jeweils auf 20 mm auf beiden Seiten festgelegt werden. Dieser Wert kann durch eine geeignete Konstruktion des Querläufers 180 eingehalten werden. Zur Berechnung der Messdauer $t$ pro Filterelement kann zusätzlich eine Filterbreite $B$ und eine Filterlänge $L$ des Filterelements herangezogen werden. Wie oben ausgeführt kann der Schwebstofffilter 118 eine Filterlänge $L$ von 250 mm bis 580 mm und eine Filterbreite $B$ von 600 mm bis 720 mm aufweisen. Durch eine festgelegte, insbesondere vorgeschriebene, Scangeschwindigkeit $v$ von 59 mm/s ergibt sich folgende Formel:

$$t = \frac{B-2*a}{v} * \frac{L}{W_p} \tag{38}$$

Somit ergibt sich die Messzeit t für einen Schwebstofffilter 118 der Breite 700 mm und der Länge 570 mm:

$$t = \frac{700\ mm - 2*20\ mm}{59\ \frac{mm}{s}} * \frac{570\ mm}{30\ mm} \tag{39}$$

$$t = 212\ s \tag{40}$$

Im Folgenden sind die berechneten Werte für die Gesamtmessdauer pro Filterelement aufgelistet, dabei wurden nur die Gesamt-Querbewegungen der Sonde berücksichtigt: Für einen Schwebstofffilter 118 der Breite 700 mm ergibt sich für eine Länge von 450 mm $t = 167$ s und für eine Länge von 570 mm $t = 212$ s. Für einen Schwebstofffilter 118 der Breite 600 mm ergibt sich für eine Länge von 400 mm $t = 126$ s und für eine Länge von 580 mm $t = 183$ s. Für einen Schwebstofffilter 118 der Breite 720 mm ergibt sich für eine Länge von 260 mm $t = 100$ s, für eine Länge von 400 mm $t = 153$ s und für eine Länge von 560 mm $t = 215$ s. Zusätzlich kann eine Dauer der Partikelzählvorrichtung 120 für die Schritte der Vorwärtsbewegung in die Transportrichtung 116 des Transportbandes 114 berücksichtigt werden.

Unter Berücksichtigung eines Durchmessers der Antriebsräder 254 mit O-Ringen 256 können grundsätzlich die erforderlichen Schritte bestimmt werden, die der Schrittmotor für eine Bewegung, insbesondere eine Vorwärtsbewegung, des Scanners 176 leistet. Beispielsweise kann das Antriebsrad 254 mit O-Ring 256 einen Außendurchmesser von 79 mm aufweisen. Somit kann das Antriebsrad einen Umfang $U$ von 248,2 mm aufweisen. Bei einem Schrittwinkel von 1,8° kann der Motor, insbesondere der Schrittmotor, 200 Schritte für eine Umdrehung benötigen.

$$n_{Schritte} = \frac{200 * W_P}{U} \tag{41}$$

$$n_{Schritte} = \frac{200 * 30 \, mm}{248,2 \, mm} = 24,17 \approx 24 \tag{42}$$

Der Schrittmotor muss somit 24 Schritte fahren, um das Fahrgestell 184 um einen geforderten Bahnabstand von 30 mm zu bewegen.

Eine Programmierung der Partikelzählvorrichtung 120, insbesondere des Scanners 176, sowie eine Projektierung der Benutzerschnittstelle kann über die Software TIA Portal, insbesondere in der Version 13, realisiert werden. Hierbei kann in erster Linie die Programmiersprache SCL (strukturierter Text, englisch: structured control language) zur Anwendung kommen. In einzelnen Fällen können Funktionsbausteine in FUP (Funktionsplan, grafische Programmiersprache innerhalb von STEP-7) programmiert sein.

Ein Programm kann mindestens einen operativen Teil, welcher insbesondere einzelne zum Betrieb benötigte Funktionen umfasst, und mindestens einen dokumentarischen Teil, welcher Systemmeldungen beinhaltet, umfassen. Durch diese Untergliederung kann eine Übersichtlichkeit erhöht werden, was insbesondere bei einer Störungssuche hilfreich sein kann. Ein Operationsbaustein OB1, welcher insbesondere für eine Steuerung des Scanners 176 zuständig sein kann, kann in weitere Funktionsbausteine, welche im Folgenden als FB bzw. FC bezeichnet werden, unterteilt werden. Die Steuerung des Scanners 176 kann modular aufgebaut sein. Die vier möglichen Bewegungsarten, welche als links, rechts, vorwärts und rückwärts bezeichnet werden können, können als separate Funktionen im OB1 programmiert sein. Je nach Anforderung, beispielsweise bei einer Führung des Sondenhalters 178 quer zu der Transportrichtung 116, was auch als Seitwärtsbewegung bezeichnet werden kann, im Handbetrieb oder in einem Vortrieb in einem automatischen Messmodus, können diese Funktionen aktiviert werden oder können deaktiviert bleiben. Die Funktionsweise des Motortreibers kann zudem grundsätzlich, technisch bedingt, eine Aktivierung von gegensätzlichen Fahrtrichtungen verhindern. Zusätzlich können im OB1 sekundäre Funktionen, für Schrittzähler, Umrechnungen, Temperaturüberwachung hinterlegt sein.

Die Programmierung der Fahrtrichtungen kann insbesondere mittels einer Schrittkette umgesetzt sein.

Die Steuerung 186 kann für den ersten Motor 191 und für den zweiten Motor 192 jeweils mindestens einen digitalen Ausgang für eine Motortreiber-Freigabe und mindestens einen digitalen Ausgang für eine Bewegungsrichtung aufweisen. Der digitale Ausgang für die Motortreiber-Freigabe kann als ENA bezeichnet werden. Der digitale Ausgang für die Bewegungsrichtung kann insbesondere ein digitaler Ausgang für eine Fahrtrichtung sein und kann als DIR bezeichnet werden. Darüber hinaus kann die Steuerung 186 für den ersten Motor 191 und für den zweiten Motor 192 jeweils mindestens einen digitalen Ausgang für eine Generierung eines Impulssignals haben, welcher auch als PUL bezeichnet werden kann. Figur 17A zeigt eine exemplarische Schrittkette für ein erfindungsgemäßes Verfahren zum Partikelzählen. Nach Ansteuerung einer Fahrtrichtung, was schematisch mit Pfeil 510 gezeigt ist, kann der digitale Ausgang für die Motortreiber-Freigabe, ENA, aktiviert werden. In der Schrittkette entspricht dies dem Feld 512. Anschließend kann eine Aktivierung des digitalen Ausgangs für die Bewegungsrichtung, DIR; erfolgen. In der Schrittkette entspricht dies dem Feld 512. Dieser Schritt kann optional sein. Für die Auswahl eine entgegengesetzte Fahrtrichtung kann DIR deaktiviert bleiben. Im Anschluss kann ein Impulssignal generiert werden. Das Impulssignal kann für eine eigentliche Bewegung erforderlich sein. In der Schrittkette entspricht dies dem Feld 514. Die Schrittkette kann in einem zeitlichen Versatz erfolgen. Beispielsweise können die Aktivierung des digitalen Ausgangs für die Motortreiber-Freigabe, ENA, und die Aktivierung des digitalen Ausgangs für die Bewegungsrichtung, DIR, in einem zeitlichen Abstand von 100 ms erfolgen. Weiterhin können die Aktivierung des digitalen Ausgangs für die Bewegungsrichtung, DIR, und die Generierung des Impulssignals in einem zeitlichen Abstand von 100 ms erfolgen.

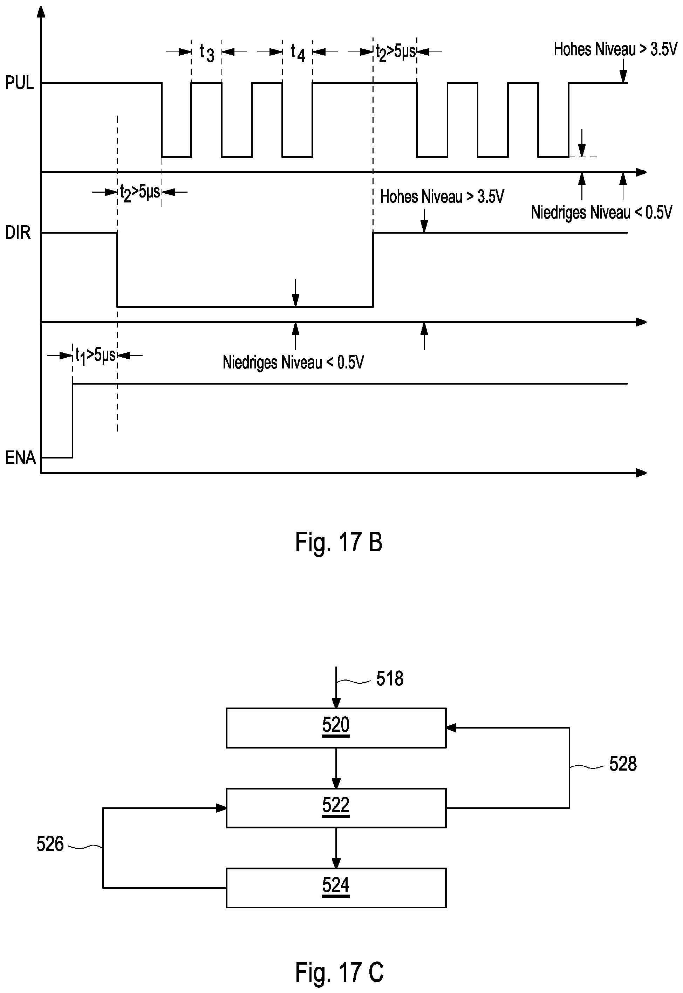

Die Programmierung der Fahrtrichtungen kann mittels der in Figur 17A dargestellten Schrittkette insbesondere gemäß dem in Figur 17B dargestellten Zeitdiagramm von Steuersignalen umgesetzt werden. Dabei entspricht $t_1$ einem erforderlichen Zeitversatz zwischen den binären Signalen für ENA und DIR, $t_2$ einen erforderlichen Zeitversatz zwischen den binären Signalen für DIR und PUL, $t_3$ einer Dauer, wie lange das binäre PUL-Signal anliegt und t4 einer Dauer, wie lange das binäre PUL-Signal nicht anliegt. "Hohes Niveau" entspricht einer Spannung von größer als 3,5 V DC, damit dies definitiv als (binäres) 1-Signal identifiziert wird und "niedriges Niveau" entspricht einer Spannung von kleiner als 0,5 V DC damit dies definitiv als (binäres) 0-Signal identifiziert wird.

Für die Bewegungsfunktionen ergeben sich somit folgende binäre Zustände, welche in den Tabellen 1 und 2 gezeigt sind.

**Tabelle 1:** Logiktabelle Quertrieb, insbesondere Führung des Sondenhalters 178 quer zu der Transportrichtung 116

| Bewegungsfunktion | ENA | DIR | PUL |
|---|---|---|---|
| digitaler Ausgang | DQ0.2 | DQ0.3 | DQe0.1 |

(fortgesetzt)

| Bewegungsfunktion | ENA | DIR | PUL |
|---|---|---|---|
| Links | 1 | 1 | 1 |
| Rechts | 1 | 0 | 1 |

**Tabelle 2:** Logiktabelle Vortrieb, insbesondere Bewegung des Fahrgestells 184 in der Transportrichtung 116

| Bewegungsfunktion | ENA | DIR | PUL |
|---|---|---|---|
| digitaler Ausgang | DQ0.0 | DQ0.1 | DQe0.0 |
| Vorwärts | 1 | 1 | 1 |
| Rückwärts | 1 | 0 | 1 |

Aus den resultierenden Fahrtrichtungsfunktionen können die Ansteuerungen für die Bewegung erfolgen. Im Handbetrieb kann eine Anwahl einer Fahrtrichtung einer entsprechend hinterlegten Funktion aktiviert werden. Insbesondere kann mit einem Button, welcher einen in eine linke Richtung weisenden Pfeil zeigt, eine Bewegungsfunktion "links" gezeigt werden. Insbesondere kann mit einem Button, welcher einen in eine rechte Richtung weisenden Pfeil zeigt, eine Bewegungsfunktion "rechts" gezeigt werden. Insbesondere kann mit einem Button, welcher einen nach oben weisenden Pfeil zeigt, eine Bewegungsfunktion "vorwärts" gezeigt werden. Insbesondere kann mit einem Button, welcher einen nach unten weisenden Pfeil zeigt, eine Bewegungsfunktion "rückwärts" gezeigt werden.

Im Messmodus, welcher auch als "Parmessbetrieb" bezeichnet werden kann, können von einer programmierten Schleifenfunktion für eine mäandrierende Fahrbahn, auch als mäandrierender Verfahrweg 272 bezeichnet, abwechselnd erforderliche Fahrtrichtungsfunktionen aktiviert werden.

Figur 17C zeigt eine weitere exemplarische Schrittkette für ein erfindungsgemäßes Verfahren zum Partikelzählen in einem automatischen Betreib. Insbesondere kann nach Start einer Messung, was mit Pfeil 518 dargestellt ist, eine Bewegungsfunktion ausgelöst werden. Insbesondere kann der Sondenhalter 178 zunächst eine linke Endlagenposition aufweisen und es kann zunächst eine Bewegungsfunktion nach rechts gestartet werden, was schematisch mit Feld 520 dargestellt ist. Bei Vorliegen des Sondenhalters 178 in der rechten Endlagenposition kann eine Bewegungsfunktion in eine Vorwärtsrichtung gestartet werden, was schematisch mit Feld 522 dargestellt ist. Bei Vorliegen des Sondenhalters 178 in der rechten Endlagenposition und unter Berücksichtigung eines temporären Zählers, insbesondere von 30, kann eine Bewegungsfunktion nach links gestartet werden, was schematisch mit Feld 524 dargestellt ist. Im Anschluss kann, bei Vorliegen des Sondenhalters 178 in der linken Endlagenposition, wiederum die Bewegungsfunktion in die Vorwärtsrichtung gestartet werden, was schematisch mit Pfeil 526 dargestellt ist. Nach Starten der Bewegungsfunktion in die Vorwärtsrichtung, bei Vorliegen des Sondenhalters 178 in der linken Endlagenposition, und unter Berücksichtigung eines temporären Zählers, insbesondere von 30 kann die Bewegungsfunktion nach rechts gestartet werden, was schematisch mit Pfeil 528 dargestellt ist.

Zur kartesischen Positionsdarstellung sowie für einen Abstand von quer zur Transportrichtung 116 verlaufenden Messbahnen können Impulse der Motortreiber verwendet werden. Hierbei kann eine Positionsermittlung grundsätzlich ohne eine zusätzliche Verwendung von Inkrementalgebern erfolgen. Über jeweils einen Vorwärts-Rückwärts-Zähler für die Transportrichtung 116 und für die Richtung quer zu der Transportrichtung 116 können Impulse gezählt werden.

Für eine Ermittlung eines Bahnabstandes bei der Bewegung in Transportrichtung 116 kann ein weiterer, insbesondere ein zusätzlicher, Vorwärts-Rückwärts-Zähler verwendet werden, der, nach Erreichen einer nächsten Messbahn, von der Steuerung 186 auf den Wert Null zurückgesetzt wird. Um langfristig einer Inkrementenverschleppung vorzubeugen, wird der Vorwärts-Rückwärts-Zähler für die Richtung quer zu der Transportrichtung 116 an einem linken Endanschlag, an der Vorwärts-Rückwärts-Zähler ohnehin den Wert Null haben müsste, genullt.

Da die Vorwärts-Rückwärts-Zähler grundsätzlich eingerichtet sind, Impulse der Motortreiber zu zählen, können für eine Darstellung von Koordinaten die Impulse in einem separaten Funktionsbaustein umgerechnet werden.

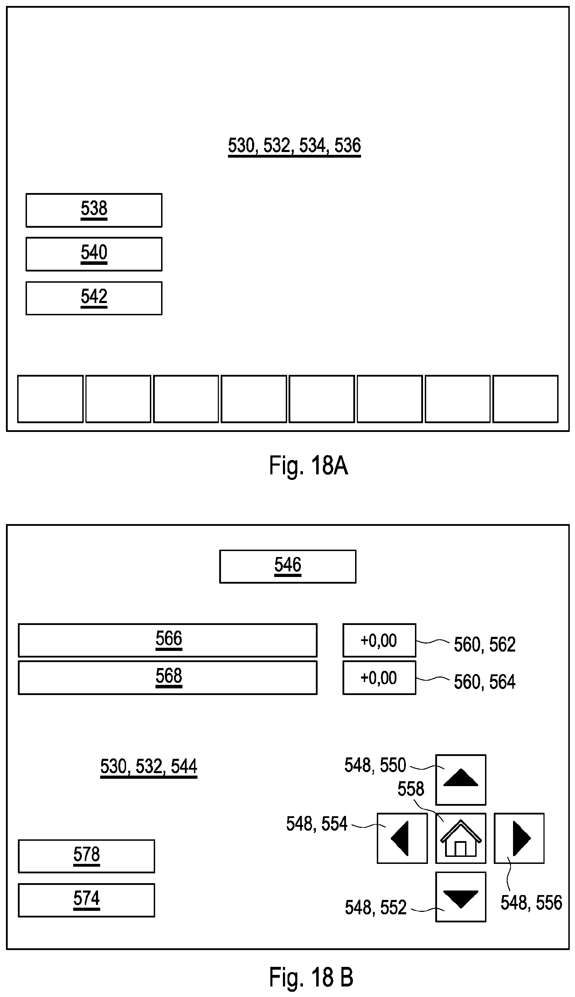

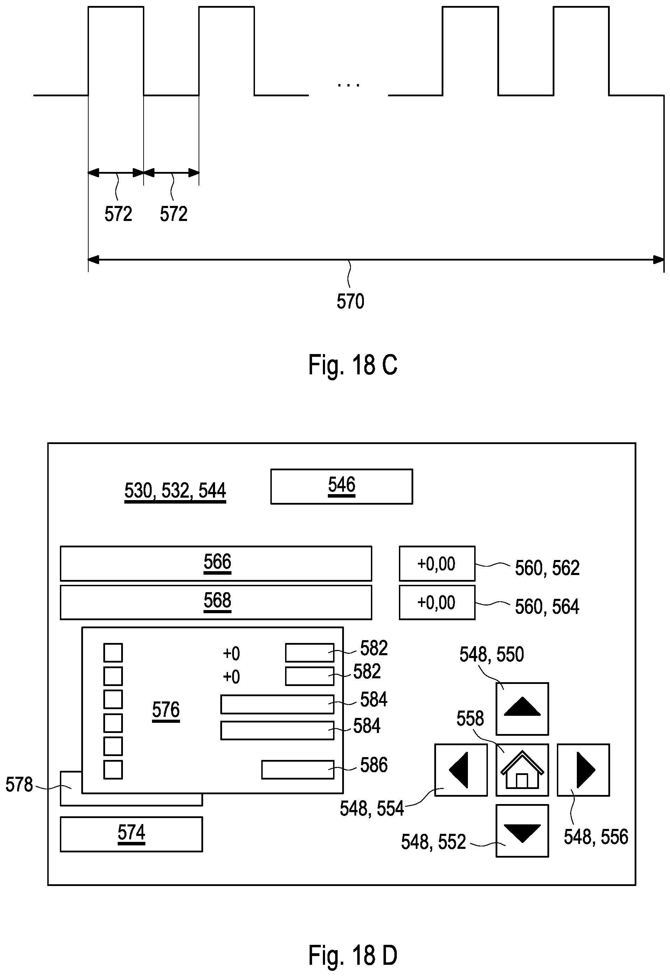

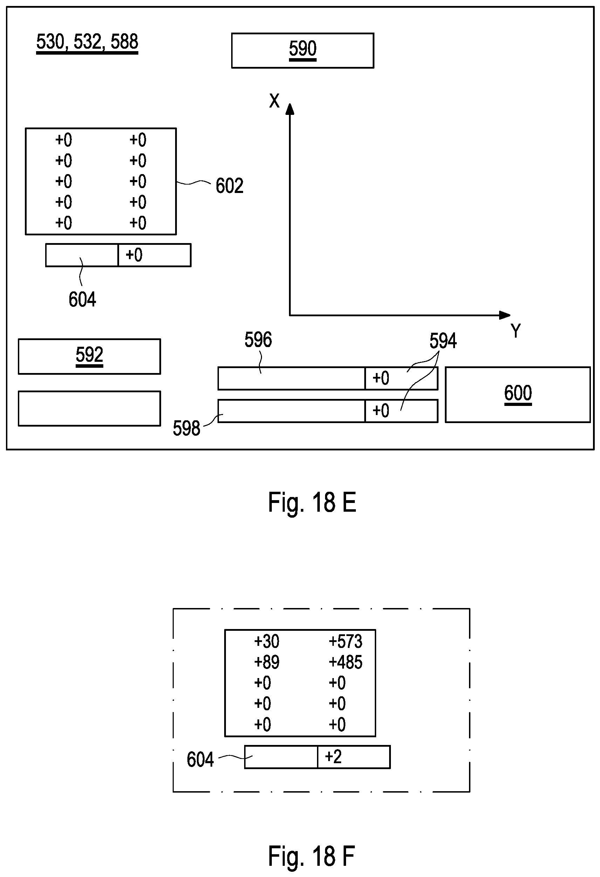

Figuren 18A bis 18F zeigen verschiedene Bedienoberflächen einer Benutzerschnittstelle 530, insbesondere einer graphischen Benutzeroberfläche 532, (Figuren 18A, 18B, 18D, 18E und 18F) sowie eine Impulsdarstellung (Figur 18C).

In Figur 18A ist ein Hauptmenü 534 dargestellt, welches auch als Grundbildschirm 536 bezeichnet werden kann. Von dem Hauptmenü 534 aus können drei Bedienoberflächen, welche auch als untergeordnete Menüpunkte bezeichnet werden können, erreicht werden, insbesondere durch eine TIA-interne Funktion, welche als "AktiviereBild" bezeichnet werden kann. Insbesondere kann das Hauptmenü 534 einen ersten Button 538 aufweisen, welcher die Bezeichnung "Parmessbetrieb" tragen kann und über welchen man zu einer ersten Bedienoberfläche gelangt, welche einem automatischen, insbesondere einem teil- oder vollautomatischen Betrieb entspricht. Weiterhin kann das Hauptmenü 534 einen zweiten Button 540 aufweisen, welcher die Bezeichnung "Handbetrieb" tragen kann und über welchen man zu einer zweiten Bedienoberfläche gelangt, welche einer manuellen Bedienung, insbesondere einen Handbetrieb entspricht. Weiterhin kann das Hauptmenü 534 einen dritten Button 542 aufweisen, welcher die Bezeichnung "Service" tragen kann und über welchen man zu einer dritten Bedienoberfläche gelangt, welche einer Oberfläche für Systemeinstellungen entspricht.

Figur 18B zeigt eine exemplarische Ausführungsform einer zweiten Bedienoberfläche 544. Die zweite Bedienoberfläche 544 kann insbesondere eine Stelle 546 aufweisen, an welcher die Bezeichnung "Handbetrieb" abgebildet ist. Die zweite Bedienoberfläche 544 kann insbesondere eine separate Ansteuerungsmöglichkeit von Bewegungsrichtungen, insbesondere Bewegungsrichtungen des Fahrgestells 184 und/oder des Sondenhalters 178, bereitstellen. Die zweite Bedienoberfläche 544 kann eingerichtet sein für ein manuelles Fahren des Scanners 176, insbesondere für ein manuelles Führen des Sondenhalters 178 quer zu der Transportrichtung 116 des Transportbands 114 und/oder für ein manuelles Fahren des Fahrgestells 184 in die Transportrichtung 116 des Transportbands 114. Die zweite Bedienoberfläche 544 kann insbesondere mehrere Buttons 548 aufweisen, welche eingerichtet sind, den ersten Motor 191 der Linearführung 182 und/oder den zweiten Motor 192 des Fahrgestells 184 anzusteuern, insbesondere in einer Fahrtrichtung 116. Insbesondere können die Buttons 548 mindestens einen ersten Button 550 umfassen für eine Steuerung einer Vorwärtsbewegung des Fahrgestells 184 in die Transportrichtung 116. Weiterhin können die Buttons 548 mindestens einen zweiten Button 552 umfassen für eine Steuerung einer Rückwärtsbewegung des Fahrgestells 184 gegen die Transportrichtung 116. Weiterhin können die Buttons 548 mindestens einen dritten Button 554 umfassen für eine Steuerung des Sondenhalters 178 quer zu der Transportrichtung 116, insbesondere von dem ersten Ende der Führungsschiene 194 zu dem zweiten Ende der Führungsschiene 194. Weiterhin können die Buttons 548 mindestens einen vierten Button 556 umfassen für eine Steuerung des Sondenhalters 178 quer zu der Transportrichtung 116, insbesondere von dem zweiten Ende der Führungsschiene 194 zu dem ersten Ende der Führungsschiene 194. Die Führung des Sondenhalters 178 von dem ersten Ende der Führungsschiene 194 zu dem zweiten Ende der Führungsschiene 194 kann auch als Bewegung oder Führung des Sondenhalters 178 nach links bezeichnet werden. Weiterhin kann die Führung des Sondenhalters 178 von dem zweiten Ende der Führungsschiene 194 zu dem ersten Ende der Führungsschiene 194 auch als Bewegung oder Führung des Sondenhalters 178 nach rechts bezeichnet werden, oder umgekehrt. Der erste Button 550, der zweite Button 552, der dritte Button 554 und der vierte Button 556 können insbesondere als Steuerkreuz angeordnet sein. Weiterhin kann die zweite Bedienoberfläche 544 einen Homebutton 558 umfassen, welcher insbesondere zentriert in dem Steuerkreuz angeordnet sein kann.

Die zweite Bedienoberfläche 544 kann insbesondere eingerichtet sein für ein Zurückfahren des Scanners 176 nach Durchführen des Verfahrens zum Partikelzählen in eine Ausgangsposition, insbesondere für das Zurückfahren des Fahrgestells 184 in die Ausgangsposition. Insbesondere kann das Zurückfahren des Fahrgestells 184 in die Ausgangsposition eine Rückwärtsbewegung des Fahrgestells 184 in die Ausgangsposition umfassen. Insbesondere aus diesem Grund kann die zweite Bedienoberfläche 544 mindestens ein, insbesondere mindestens zwei Eingabefelder 560 für eine Geschwindigkeit umfassen. Insbesondere kann die zweite Bedienoberfläche 455 ein erstes Eingabefeld 562 für eine Geschwindigkeit des Fahrgestells 184 in Transportrichtung 116 und ein zweites Eingabefeld 564 für eine Geschwindigkeit des Sondenhalters 178 quer zu der Transportrichtung 116 umfassen. Vor dem ersten Eingabefeld 560, insbesondere an einer Stelle 566, kann die zweite Bedienoberfläche 544 die Bezeichnung "Geschwindigkeit Längsrichtung" umfassen. Vor dem zweiten Eingabefeld 564, insbesondere an einer Stelle 568, kann die zweite Bedienoberfläche 544 die Bezeichnung "Geschwindigkeit Querrichtung" umfassen. Eingegebene Werte der Geschwindigkeit können eingerichtet sein, Haltezeiten von Einschalterverzögerungen beziehungsweise Ausschalterverzögerungen, welche für Pulsgeber verwendet werden können, zu manipulieren.

Da die Eingabefelder vom Datentyp REAL und die Haltezeiten vom Datentyp TIME sein können, kann der eingegebene Wert erst umgerechnet, beziehungsweise in den Datentyp TIME umgewandelt werden. Für die reale Geschwindigkeit $Geschw_{Real}$ gilt grundsätzlich:

$$Geschw_{Real} = \frac{Weg}{Zeit} \qquad (43)$$

Wenn man nun für die Geschwindigkeitsberechnung eine Umdrehung annimmt, kann sich für den Weg der Umfang des treibenden Zahnrades ergeben:

$$Weg = \pi * Durchmesser_{Zahnrad} \qquad (44)$$

Eine Umdrehung kann, je nach Parametereinstellung, eine definierte Anzahl von Impulsen umfassen, welche jeweils als Zeitverzögerungsglieder mit einer identischen Verzögerungszeit programmiert werden können. Dies ist schematisch in der Impulsdarstellung in Figur 18C gezeigt. Mit Pfeil 570 ist eine komplette Umdrehung dargestellt und mit Pfeilen 572 einzelne Zeitverzögerungsglieder. Daher kann in der Grundformel der Geschwindigkeitsberechnung für die Zeit ein Produkt von einzelnen Zeitverzögerungen herangezogen werden:

$$Zeit = \sum_{i=1}^{Imp_{pro\ Umdrehung}} (2 * Impuls_{Länge}) \qquad (45)$$

Somit kann sich für eine Grundformel der Geschwindigkeitsberechnung ergeben:

$$Geschwindikeit_{Real} = \frac{\pi * Durchmesser_{Zahnrad}}{\sum_{i=1}^{Imp_{pro\ Umdrehung}} (2 * Impuls_{Länge})} \qquad (46)$$

Auf Grund von stets identischen Impulslängen kann geschrieben werden:

$$Geschwindigkeit_{Real} = \frac{\pi * Durchmesser_{Zahnrad}}{2 * Impuls_{pro\ Umdrehung} * Impuls_{Länge}} \qquad (47)$$

Da für die Programmierung aus dem eingegebenen Geschwindigkeitswert *Geschwindigkeit*$_{Real}$ die Impulslänge benötigt wird, folgt:

$$Impuls_{Länge} = \frac{\pi * Durchmesser_{Zahnrad}}{2 * Impuls_{pro\ Umdrehung} * Geschwindigkeit_{Real}} \qquad (48)$$

Nach der Umrechnung kann eine Umwandlung des Datentyps erfolgen, insbesondere in zwei Schritten. So kann der Wert mittels der folgenden Anweisungen:

$$Imp_{Länge(DWord)} := REAL\_TO\_DWORD(IN := Imp_{Länge(Real)}) \qquad (49)$$

$$Imp_{Länge(Time)} := DWORD\_TO\_TIME(IN := Imp_{Länge(DWord)}) \qquad (50)$$

gewandelt und kann mit den entsprechenden Timern verknüpft werden.

Weiterhin kann die zweite Bedienoberfläche 544 mindestens einen "zurück"-Button 574 aufweisen, über welchen die zweite Bedienoberfläche 544 mit einem Bildwechsel in das Hauptmenü 534 wechselt und insbesondere den Handbetrieb beendet. Weiterhin kann die zweite Bedienoberfläche 544 ein oder mehrere Informationsfenster 576 aufweisen, insbesondere da über die zweite Bedienoberfläche 544 ein oder mehrere Testfahrten, beispielsweise für eine Störungssuche, durchgeführt werden können. Ein exemplarisches Informationsfenster ist in Figur 18D dargestellt. Die zweite Bedienoberfläche 544 kann insbesondere einen Service-Button 578 aufweisen, über welchen das Informationsfenster 576 aktiviert wird. Die Aktivierung kann insbesondere durch eine Funktion einer Sichtbarkeit von Bildelementen in der Programmierung umgesetzt werden. Insbesondere kann der Service-Button 578 eingerichtet sein, ein Bit zu aktivieren, welches für eine Sichtbarkeitsabfrage von Elementen verwendet wird. Das Informationsfenster 576 kann insbesondere einen Schließ-Button aufweisen, welcher insbesondere eingerichtet sein kann, den Bit zu deaktivieren, wodurch die Elemente ihre Sichtbarkeit verlieren. Das Informationsfenster 576 kann mehrere Felder 580 aufweisen, welche jeweils den digitalen Ausgängen der Motortreiber entsprechen und jeweils mit "x-ENA", "x-DIR", "x-PUL", "y-ENA", "y-DIR" und "y-PUL" bezeichnet werden können. Das Informationsfenster 576 kann darüber hinaus ein oder mehrere Buttons 582 für ein Zurücksetzen von Inkrementen für die Bewegung des Fahrgestells 184 und/oder für die Führung des Sondenhalters 178 umfassen. Darüber hinaus kann das Informationsfenster ein oder mehrere Buttons 584 für eine Simulierung von Endanschlägen aufweisen. Über einen Schließ-Button 586 kann das Informationsfenster 576 geschlossen werden.

Figur 18E zeigt eine exemplarische Ausführungsform einer ersten Bedienoberfläche 588. Die erste Bedienoberfläche 588 kann insbesondere eine Stelle 590 aufweisen, an welcher die Bezeichnung "Parmessbetrieb" abgebildet ist. Die erste Bedienoberfläche 588 kann insbesondere ein Starten und ein Stoppen einer definierten, automatischen Fahrt des Fahrgestells 184 und/oder der Sonde 122 umfassen. Die erste Bedienoberfläche 588 kann insbesondere einen Start-Button 592 umfassen, über welchen eine einprogrammierte Schrittkette gestartet wird. Weiterhin kann die erste Bedienoberfläche 592 eingerichtet sein, einen Stopp-Button sichtbar zu machen (nicht in Figur 18 E dargestellt), welcher insbesondere an einer Position der ersten Bedienoberfläche 588 erscheint, welche einer Position des Start-Buttons 592 entspricht. Weiterhin kann die erste Bedienoberfläche 588 eingerichtet sein, über den Stopp-Button die einprogrammierte Schrittkette anzuhalten und insbesondere die Sichtbarkeit des Stopp-Buttons zu deaktivieren. Weiterhin kann die erste Bedienoberfläche 588 zwei Ausgabefelder 594 aufweisen, welche eine aktuelle kartesische Position der Sonde 122 anzeigen. In einer Stelle 596 vor einem der Ausgabefelder 594 kann die Bezeichnung "Position x-Richtung" dargestellt sein. In einer weiteren Stelle 598 vor einem der Ausgabefelder 594 kann die Bezeichnung "Position y-Richtung" dargestellt sein. Weiterhin kann die erste Bedienoberfläche 588 einen Button 600 "Position speichern" aufweisen. Die erste Bedienoberfläche 588 kann eingerichtet sein, durch Drücken des Buttons "Position speichern" erfasste Koordination, insbesondere zu dem Zeitpunkt des Drückens erfasste Koordinaten, zwischenzuspeichern, insbesondere in einem Datenbaustein. Dadurch kann ein Zähler, insbesondere ein Leckage-Zähler, eine Anzahl möglicher Lecks erhöhen. Eine Darstellung von Positionen möglicher Lecks 602 kann mehrere, insbesondere fünf, Variablen, jeweils für eine Position in der Transportrichtung 116 und für eine Position quer zu der Transportrichtung 116, aufweisen. Die Positionen können auch als x- und y-Positionen bezeichnet werden. Werte dieser Variablen können default-mäßig Null betragen, insbesondere wenn der Leckagezähler den Wert Null aufweist. Sobald der Leckagezähler auf den Wert Eins erhöht wird, werden in einem ersten Variablenpaar, insbesondere einem ersten x-y-Variablenpaar, aktuelle Positionswerte angezeigt werden. Die Darstellung von Positionen möglicher Lecks kann insbesondere einen clear"-Button 604 aufweisen, welcher eingerichtet ist, die Variablen auf den Wert Null zurückzusetzen, insbesondere über eine Zurücksetzung des Leckagezählers. Wie bereits ausgeführt, kann insbesondere ein Speichern von bis zu 5 Leckagen vorgesehen sein, da bereits diese Anzahl an Auffälligkeiten auf einen kritischen Zustand des überprüften Filterelementes hindeutet. Figur 18F zeigt eine Detaildarstellung der Darstellung von Positionen möglicher Lecks 602.

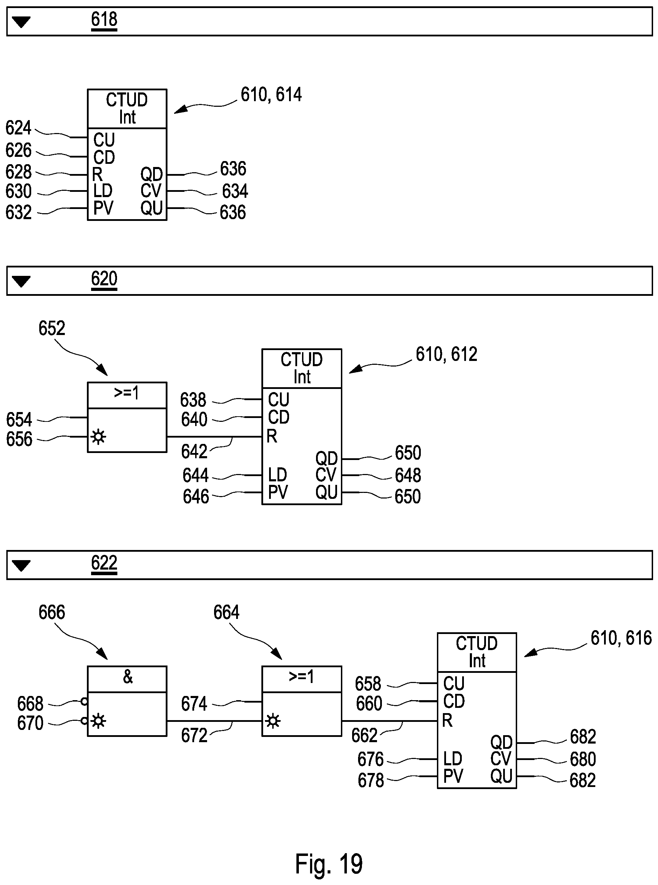

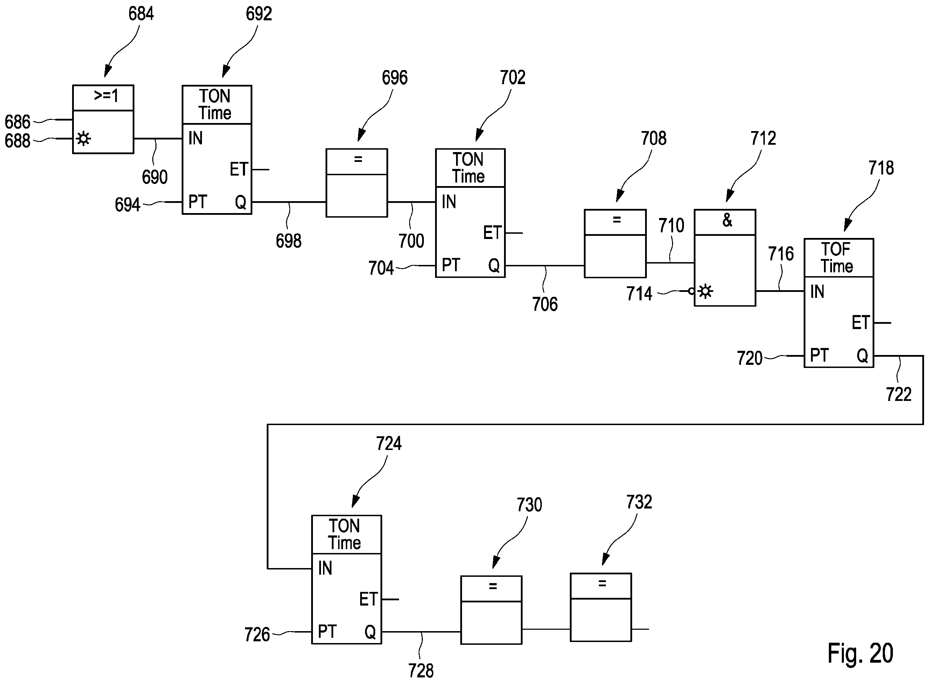

Figuren 19 und 20 zeigen verschiedene Ausführungsbeispiele einer Programmierung der Steuerung 186, insbesondere einer speicherprogrammierbareren Steuerung (SPS). In diesen Ausführungsbeispielen ist die Programmierung über die Software TIA Portal in der Version 13 mit der Programmiersprache SCL realisiert. Insbesondere zeigt Figur 19 ein Ausführungsbeispiel einer Programmierung der Steuerung 186 umfassend mindestens einen Vorwärts-Rückwärts-Zähler 610. Die Steuerung 186 kann einen ersten Vorwärts-Rückwärts-Zähler 612, einen zweiten Vorwärts-Rückwärts-Zähler 614 und einen dritten Vorwärts-Rückwärts-Zähler 616 umfassen. Wie in Figur 19 gezeigt, kann der zweite Vorwärts-Rückwärts-Zähler 614 in einem ersten Netzwerk 618, der erste Vorwärts-Rückwärts-Zähler 612 in einem zweiten Netzwerk 620 und der dritte Vorwärts-Rückwärts 616 in einem dritten Netzwerk 622 der Steuerung 186 enthalten sein.

Die Partikelzählvorrichtung 120, insbesondere die Steuerung 186, kann eingerichtet sein, mittels des mindestens einen zweiten Vorwärts-Rückwärts-Zählers 614 zweite Impulse des zweiten Motors 192, insbesondere des zweiten Schrittmotortreibers, zu zählen. Der zweite Vorwärts-Rückwärts-Zähler 614 kann also insbesondere ein Zähler der x-Achse sein. Ein zweiter Zähleingang zum Vorwärtszählen 624 kann mit einem Signal "x-PUL_V" belegt sein. Ein zweiter Zähleingang zum Rückwärtszählen 626 kann mit einem Signal "x-PUL_R" belegt sein. Ein zweiter Rücksetzeingang 628 kann mit einem Signal "Reset_x-Achse" belegt sein. Ein zweiter Ladeeingang 630 kann mit einem Signal "false" belegt sein. Ein zweiter Ladewert 632 kann mit einem Wert 0 belegt sein. Weiterhin kann der zweite Vorwärts-Rückwärts-Zähler 614 einen zweiten Ausgang zum Zählerstand 634 und weitere zweite Ausgänge zur Abfrage des Zählerstatus 636 aufweisen. Dem zweiten Ausgang zum Zählerstand 634 kann insbesondere die "x-Position" entnommen werden.

Die Partikelzählvorrichtung 120, insbesondere die Steuerung 186, kann eingerichtet sein, mittels des mindestens einen ersten Vorwärts-Rückwärts-Zählers 612 erste Impulse des ersten Motors 191, insbesondere des ersten Schrittmotortreibers, zu zählen. Der erste Vorwärts-Rückwärts-Zähler 614 kann also insbesondere ein Zähler der y-Achse sein. Ein erster Zähleingang zum Vorwärtszählen 638 kann mit einem Signal "y-PUL_R" belegt sein. Ein erster Zähleingang zum Rückwärtszählen 640 kann mit einem Signal "y-PUL_L" belegt sein. Ein erster Rücksetzeingang 642 kann mit einem Signal von einem ODER-Glied 652 belegt sein, an welchen die Eingangssignale 654 "Reset_y-Achse" und 656 "Endlage_Links" anliegen. Ein erster Ladeeingang 644 kann mit einem Signal "false" belegt sein. Ein erster Ladewert 646 kann mit einem Wert 0 belegt sein. Weiterhin kann der erste Vorwärts-Rückwärts-Zähler 612 einen ersten Ausgang zum Zählerstand 648 und weitere erste Ausgänge zur Abfrage des Zählerstatus 650 aufweisen. Dem ersten Ausgang zum Zählerstand 648 kann insbesondere die "y-Position" entnommen werden.

Die Partikelzählvorrichtung 120, insbesondere die Steuerung 186, kann also eingerichtet sein, mittels der ersten Impulse des ersten Motors 191 eine Position der Sonde 122 auf der Linearführung 182 und mittels der zweiten Impulse des zweiten Motors 192 eine Position des Fahrgestells 184 auf dem Transportband 114 zu ermitteln.

Der dritte Vorwärts-Rückwärts-Zähler 616 kann also insbesondere ein Zähler "x-Achse_Temp" sein. Ein dritter Zähleingang zum Vorwärtszählen 658 kann mit einem Signal "x-PUL__V" belegt sein. Ein dritter Zähleingang zum Rückwärtszählen 660 kann mit einem Signal "x-PUL_R" belegt sein. Ein dritter Rücksetzeingang 662 kann mit einem Signal eines ODER-Glieds 664 mit vorgeschalteten UND-Glied 666 belegt sein. An dem UND-Glied 666 liegen die Eingangssignale 668 "Endlage_Links" und 670 "Endlage_Rechts" an. Das ODER Glied 664 wird mit einem Ausgangssignale 672 des UND-Glieds 666 und einem Eingangssignal 674 "Reset_x-Achse" versorgt. Ein dritter Ladeeingang 676 kann mit einem Signal "Rechtsfahrt" belegt sein. Ein dritter Ladewert 678 kann mit einem Wert 0 belegt sein. Weiterhin kann der dritte Vorwärts-Rückwärts-Zähler 616 einen dritten Ausgang zum Zählerstand 680 und weitere dritte Ausgänge zur Abfrage des Zählerstatus 682 aufweisen. Dem dritten Ausgang zum Zählerstand 680 kann insbesondere die "x-Position_Temp" entnommen werden.

Darüber hinaus kann die Partikelzählvorrichtung 120, insbesondere die Steuerung 186, mindestens einen weiteren Vorwärts-Rückwärts-Zähler (nicht dargestellt), welcher für eine Zählung von Impulsen des zweiten Motors 192, insbesondere des zweiten Schrittmotortreibers, eingerichtet ist, umfassen. Der weitere Vorwärts-Rückwärts-Zähler kann daher insbesondere für eine Ermittlung eines Bahnabstandes bei einer Bewegung in die Transportrichtung 116 eingerichtet sein. Die Partikelzählvorrichtung 120, insbesondere die Steuerung 186, kann eingerichtet sein, nach einer schrittweisen Bewegung des Fahrgestells 184, insbesondere nach einem Erreichen einer nächsten Messbahn, den weiteren Vorwärts-Rückwärts-Zähler auf Null zurückzusetzen. Weiterhin kann die Linearführung 182 einen ersten Endanschlag und einen zweiten Endanschlag aufweisen und die Steuerung 186 kann eingerichtet sein, den ersten Vorwärts-Rückwärts-Zähler 612 auf Null zurückzusetzen, wenn sich die Sonde 122 am ersten Endanschlag befindet. Insbesondere kann es sich bei dem ersten Endanschlag um einen linken Endanschlag handeln.

Figur 20 zeigt ein Ausführungsbeispiel einer Programmierung der Steuerung 186 umfassend eine Schrittkette zur Steuerung der Bewegung der Partikelvorrichtung 120. Die in Figur 20 gezeigte Schrittkette beginnt mit einem ODER-Glied 684 an dem die Eingangssignale 686 "Start_L" oder 688 "Initialfahrt" anliegen. Das Ausgangssignal 690 des ODER-Glieds 684 kann als Startbedingung eines Funktionsbausteins 692 mit der Funktion "Links_ENA" anliegen. An Funktionsbaustein 692 kann eine Einschaltverzögerung mit einer Zeitvorgabe 694 von beispielsweise 100 ms vorliegen. Ein Speicherglied "y-ENA" 696 kann das Ausgangssignal 698 des Funktionsbausteins 692 als Eingangssignal 700 für eine Startbedingung eines weiteren Funktionsbausteins 702 mit der Funktion "Links_DIR" weitergeben. An Funktionsbaustein 702 kann eine Einschaltverzögerung mit einer Zeitvorgabe 704 von beispielsweise 100 ms vorliegen. Ein Ausgangssignal 706 des Funktionsbausteins 702 kann über ein Speicherglied "y-DIR" 708 als Eingangssignal 710 eines UND-Glieds 712 weitergegeben werden. An dem UND-Glied 712 kann ein weiteres Eingangssignal "Impuls_Neustart" 714 anliegen. Ein Ausgangssignal 716 des UND-Glieds 712 kann als Startbedingung eines weiteren Funktionsbausteins 718 mit der Funktion "Links_PUL" anliegen. An Funktionsbaustein 718 kann eine Ausschaltverzögerung mit einer Zeitvorgabe 720 "Zeit_PUL" vorliegen. Ein Ausgangssignal 722 des Funktionsbausteins 718 kann als Startbedingung für einen weiteren Funktionsbaustein 724 mit der Funktion "Wartezeit_für_Neustart_L" dienen. An Funktionsbaustein 724 kann eine Einschaltverzögerung mit einer Zeitvorgabe 726 "Zeit_PUL" anliegen. Ein Ausgangssignal 728 des Funktionsbausteins 724 kann als Eingangssignal von nacheinander geschalteten Speichergliedern 730 "Implus_Neustart" und 732 "y-PUL" anliegen.

Beispiele

Die folgenden Beispiele dienen der Erläuterung der Erfindung. Sie dürfen nicht als Einschränkung des Schutzumfangs ausgelegt werden.

Die Ansteuerung der Schrittmotoren wurde zur Funktionsüberprüfung mit Hilfe eines Boards mit Mikrokontroller (Arduino) realisiert. Das Board wurde über eine USB-Schnittstelle mit einem Computer verbunden. Mit der dazugehörigen Software wurde das Programm zur Steuerung der Bewegung der Linearführung bzw. der Bewegung des Fahrgestells erstellt und auf das Board geladen. Das Programm gab unter anderem die Schritte, die Geschwindigkeit und die Drehrichtung des Schrittmotors vor. Das Board übertrug die Signale über eine Steckplatine an den Motortreiber. Der Motortreiber gab Signale an den Schrittmotor weiter und versorgte diesen mit der benötigten Spannung. Ein ebenfalls an den Motortreiber angeschlossenes Netzteil wandelte eine Spannung von 230 V auf die vom Motortreiber benötigten 24 V um. Das Netzteil wurde so gewählt, dass es zwei Motortreiber und somit auch beide Schrittmotoren der Partikelzählvorrichtung 120 versorgen kann. Durch diesen Aufbau konnte ein realitätsnaher Funktionstest durchgeführt werden.

Beispiel 1: Überprüfung einer Wiederholgenauigkeit des Schrittmotors der Linearführung

Zum Überprüfen der Wiederholgenauigkeit des Schrittmotors der Linearführung wurde ein Bewegungsablauf des Sondenhalters bzw. des Zahnstangenantriebs in mehreren aufeinanderfolgenden Bahnen getestet. Hierbei wurde überprüft, ob bei der Drehung des Schrittmotors Schritte übersprungen werden. Dies könnte grundsätzlich dazu führen, dass ein Verfahrweg nicht vollständig abgefahren wird. Es könnten sich dadurch Folgefehler, wie beispielsweise ein Zusammenstoß mit der Zahnstangenhalterung ergeben. Für den Schwebstofffilter 118, insbesondere den HEPA-Filter, in einem Tunneltyp mit einer Länge $L$ von 570 mm werden grundsätzlich viele Bahnen zum Abfahren der gesamten Filterfläche benötigt. Dabei ist grundsätzlich zu beachten, dass auf jede Führung des Sondenhalters quer zu der Transportrichtung eine Vorwärtsbewegung des Fahrgestells folgt. Eine Anzahl von abzufahrenden Bahnen $n_B$ wurde ermittelt und programmiert. Dabei wurde ein Bahnabstand $W_P$ von 30 mm eingesetzt.

$$n_B = \frac{L}{W_P} \qquad\qquad (51)$$

$$n_B = \frac{570\ mm}{30\ mm} = 19 \tag{52}$$

Der Führungsschlitten wurde mit einem initialen Abstand von 5 mm zu der Zahnstangenhalterung positioniert. Mit einem digitalen Messschieber wurde eine Startposition gemessen und das Programm wurde gestartet. Der Führungsschlitten fuhr die Anzahl der berechneten Bahnen $n_B$ mit der Geschwindigkeit von 5,9 cm/s nacheinander ab und stoppte anschließend an der Endposition. Die Endposition war in diesem Fall gleich der Startposition. Mit dem digitalen Messschieber wurde wieder der Abstand zwischen Zahnstangenhalterung und Endposition gemessen.

Um ein aussagekräftiges Ergebnis über eine durchschnittliche Wiederholgenauigkeit zu erhalten und einen Mittelwert bilden zu können, wurde dieser Vorgang fünfmal nacheinander wiederholt und es wurde die Differenz zwischen Start- und Endposition bestimmt. Dies ist in der folgenden Tabelle 3 zusammengefasst.

Tabelle 3: Messergebnisse der Wiederholgenauigkeit des Zahnstangenantriebs der Linearführung

| Lauf | 1 | 2 | 3 | 4 | 5 |
|---|---|---|---|---|---|
| Startposition | 5,73 mm | 5,71 mm | 5,50 mm | 5, 79 mm | 5,70 mm |
| Endposition | 5,63 mm | 5,86 mm | 5,71 mm | 5,66 mm | 5,75 mm |
| Abweichung x | 0,10 mm | 0,15 mm | 0,11 mm | 0,13 mm | 0,05 mm |

Es wurde ein arithmetischer Mittelwert $x_1$ der gemessenen Abweichungen ermittelt:

$$\overline{x_1} = \frac{1}{n} \sum_{i=1}^{n} x_i \tag{53}$$

$$\overline{x_1} = \frac{0,1\ mm + 0,15\ mm + 0,11\ mm + 0,13\ mm + 0,05\ mm}{5} = 0,108\ mm \tag{54}$$

Um zu beweisen, dass keine Schritte übersprungen wurden, muss die Abweichung $x_1$ grundsätzlich kleiner sein als die zurückgelegte Strecke des Stirnzahnrades pro Schritt $S_1$.

$$S_1 = \frac{l_Z}{\frac{360°}{Schrittwinkel}} \tag{55}$$

$$S_1 = \frac{59,69\ mm}{\frac{360°}{1.8°}} = 0,299\ mm \tag{56}$$

Dabei entspricht $l_z$ einer zurückgelegten Strecke bei einer Umdrehung des Zahnrads. Für weitere Details wird auf obige Ausführungen verwiesen.

Da die Abweichung $x_1$ kleiner ist als die zurückgelegte Strecke des Stirnzahnrades pro Schritt $S_1$, konnte somit eine einwandfreie Funktion und eine präzise Wiederholgenauigkeit des Schrittmotors und damit des Zahnstangenantriebs der Linearführung gezeigt werden. Es wurde gezeigt, dass der Schrittmotor keine Schritte überspringt. Die ermittelten Abweichungen zwischen Start- und Endposition waren in allen Läufen kleiner als 0,299 mm und betrugen im Mittel 0,108 mm.

Beispiel 2: Überprüfung einer Wiederholgenauigkeit des Schrittmotors des Fahrgestells

Anschließend wurde die Funktion des Fahrgestells getestet. Ein neues Programm zur Steuerung des Fahrgestells wurde auf das Board mit Mikrocontroller geladen, welches das Fahrgestell nacheinander jeweils um einen Bahnabstand $W_P$ von 30 mm vorwärtsbewegt und zwischen den einzelnen Vorwärtsbewegungen eine Pause einhält. Mit diesem Programm wurde der Bewegungsablauf des Fahrgestells im Sterilisationstunnel simuliert. In den Bewegungspausen des Fahrgestells, kann grundsätzlich eine Führung des Sondenhalters auf dem Auerträger stattfinden. Auch hier wurde die Wiederholgenauigkeit der zurückgelegten Strecke überprüft.

Die Wiederholgenauigkeit des Fahrgestells wurde auf einer ebenen Fläche geprüft. Bei dem durchgeführten Test auf der ebenen Fläche, bewegten sich die Antriebsräder mit einer Geschwindigkeit von 30 Umdrehungen/min. Das Fahrgestell wurde, mit montiertem Querläufer, gemäß der Anzahl der abzufahrenden Bahnen $n_B$, 19-mal schrittweise um jeweils einen Bahnabstand *Wp* von 30 mm nach vorne bewegt. Die zurückgelegte Strecke des Fahrgestells musste bei 19 Wiederholungen 570 mm betragen. Die Endposition des Fahrgestells wurde von der Startposition mit einem Maßband mit 0,50 mm-Teilung gemessen. Dieser Vorgang wurde ebenfalls fünfmal nacheinander wiederholt.

**Tabelle 4:** Messergebnisse der Wiederholgenauigkeit des Antriebs des Fahrgestells

| Lauf | 1 | 2 | 3 | 4 | 5 |
|------|---|---|---|---|---|
| Startposition | 0,0 mm | 0,0 mm | 0,0 mm | 0,0 mm | 0,0 mm |
| Endposition | 570,0 mm | 570,5 mm | 569,5 mm | 570,0 mm | 569,5 mm |
| Abweichung x | 0,0 mm | 0,5 mm | 0,5 mm | 0,0 mm | 0,5 mm |

Es wurde ein arithmetischer Mittelwert der Abweichungen $x_2$ des Verfahrweges des Fahrgestells ermittelt:

$$\overline{x_2} = \frac{1}{n} \sum_{i=1}^{n} x_i \tag{57}$$

$$\overline{x_2} = \frac{0,0\ mm + 0,5\ mm + 0,5\ mm + 0,0\ mm + 0,5\ mm}{5} = 0,3\ mm \tag{58}$$

Um zu zeigen, dass keine Schritte des Motors übersprungen wurden und sich das Fahrgestell fehlerfrei vorwärtsbewegt, muss die Abweichung $x_2$ grundsätzlich kleiner sein als die zurückgelegte Strecke der Antriebsräder und damit des Fahrgestells pro Schritt $S_2$:

$$S_2 = \frac{U}{\frac{360°}{Schrittwinkel}} \tag{59}$$

$$S_2 = \frac{248,2\ mm}{\frac{360°}{1.8°}} = 1,241\ mm \tag{60}$$

Dabei entspricht *U* einem Umfang der Antriebsräder. Es wird hierbei auf obige Ausführungen verwiesen.

Da die Abweichung des Verfahrweges $x_2$ kleiner ist als die die zurückgelegte Strecke der Antriebsräder pro Schritt $S_2$, wurde eine einwandfreie Funktion und eine korrekte Wiederholgenauigkeit des Fahrgestells gezeigt. Auch hier wurde gezeigt, dass der Schrittmotor keine Schritte überspringt. Die ermittelten Abweichungen zwischen Start- und Endposition, waren in allen Läufen kleiner als 1,241 mm und betrugen im Mittel 0,3 mm.

Beispiel 3: Parametereinstellung der Motortreiber

Der Scanner kann über zwei separate Schrittmotoren mit ihren dazugehörigen Motortreibern angetrieben werden. Für einen funktionierenden Antrieb kann an den Motortreiber eine Stromaufnahme der angeschlossenen Motoren und eine Anzahl der Impulse pro Umdrehung eingestellt werden.

Es können Schrittmotoren vom Typ 17HS24-2104S verwendet werden, welche eine Stromaufnahme von bis zu 2,1A haben. Die Bestimmung der Impulsanzahl ist grundsätzlich abhängig von der Geschwindigkeit, als auch von einer niedrigst einstellbaren Zeitverzögerung der Impulsprogrammierung.

Wenn man nun eine Mindestzeitverzögerung vom 1 ms heranzieht, sowie eine Geschwindigkeit von 5cm/s und die Geometrie des antreibenden Zahnrades ergeben sich 596,90 Impulse pro Umdrehung. Hinsichtlich der Berechnung wird auf obige Formeln 22 und 23 verwiesen. Da für die Verzögerungszeit kein Wert gewählt werden kann, welcher kleiner als 1ms ist, entsprechen die errechneten 596,9 Impulse pro Umdrehungen einem Maximum, um die Geschwindigkeit von 5cm/s gewährleisten zu können.

Gemäß der Parametertabelle des Stepperdriver DM556N (siehe Tabellen 5 und 6) ist somit eine Stromaufnahme von bis zu 2,3A und eine Geschwindigkeit von 400 Impulsen pro Umdrehung einzustellen.

**Tabelle 5:** Parametertabelle des Motortreibers DM556N, Teil 1

| Spitzenstrom | root-mean square current | SW1 | SW2 | SW3 |
|--------------|--------------------------|-----|-----|-----|
| 1,4 A | 1,0 A | AN | AN | AN |

(fortgesetzt)

| Spitzenstrom | root-mean square current | SW1 | SW2 | SW3 |
|---|---|---|---|---|
| 2,1 A | 1,5 A | AUS | AN | AN |
| 2,7 A | 1,9 A | AN | AUS | AN |
| 3,2 A | 2,3 A | AUS | AUS | AN |
| 3,8 A | 2,7 A | AN | AN | AUS |
| 4,3 A | 3,0 A | AUS | AN | AUS |
| 4,9 A | 3,5 A | AN | AUS | AUS |
| 5,6 A | 4,0 A | AUS | AUS | AUS |

**Tabelle 6:** Parametertabelle des Motortreibers DM556N, Teil 2

| Mikroschritt | Pulse pro Umdrehung | SW5 | SW6 | SW7 | SW8 |
|---|---|---|---|---|---|
| 2 | 400 | AUS | AN | AN | AN |
| 4 | 800 | AN | AUS | AN | AN |
| 8 | 1600 | AUS | AUS | AN | AN |
| 16 | 3200 | AN | AN | AUS | AN |
| 32 | 6400 | AUS | AN | AUS | AN |
| 64 | 12800 | AN | AUS | AUS | AN |
| 128 | 25600 | AUS | AUS | AUS | AN |
| 5 | 1000 | AN | AN | AN | AUS |
| 19 | 2000 | AUS | AN | AN | AUS |
| 20 | 4000 | AN | AUS | AN | AUS |
| 25 | 5000 | AUS | AUS | AN | AUS |
| 40 | 8000 | AN | AN | AUS | AUS |
| 50 | 10000 | AUS | AN | AUS | AUS |
| 100 | 20000 | AN | AUS | AUS | AUS |
| 125 | 25000 | AUS | AUS | AUS | AUS |

Damit ergibt sich für die beiden Motortreiber folgende Parametereinstellung nach Tabelle 7

**Tabelle 7:** Parametereinstellung der Motortreiber

| | SW1 | SW2 | SW3 | SW4 | SW5 | SW6 | SW7 | SW8 |
|---|---|---|---|---|---|---|---|---|
| AUS | | | | | | | | |
| AN | | | | | | | | |

Bezugszeichenliste

110 Sterilisationstunnel
112 Medikamentenabfüllanlage
114 Transportband
116 Transportrichtung
118 Schwebstofffilter
120 Partikelzählvorrichtung

| 122 | Sonde |
| 124 | Sondenöffnung |
| 126 | Sondentrichter |
| 128 | Zuluftkanal |
| 130 | Ventilator |
| 132 | Absaugvorrichtung |
| 134 | Anwärmzone |
| 136 | Sterilisationszone |
| 138 | Abkühlzone |
| 140 | erster Zuluftkanal |
| 142 | erster Ventilator |
| 144 | erster Schwebstofffilter |
| 146 | weiterer Ventilator |
| 148 | zweiter Ventilator |
| 150 | zweiter Schwebstofffilter |
| 152 | dritten Ventilator |
| 154 | vierter Schwebstofffilter |
| 156 | vierter Ventilator |
| 158 | Ablaufkanal |
| 160 | Rohluftseite |
| 162 | Aerosolgenerator |
| 164 | Prüfstutzen |
| 166 | Partikelzähler |
| 168 | Verdünnungsstufe |
| 170 | Reinluftseite |
| 172 | isokinetischen Sonde |
| 174 | Partikelzähler |
| 176 | Scanner |
| 178 | Sondenhalter |
| 180 | Querläufer |
| 182 | Linearführung |
| 184 | Fahrgestell |
| 186 | Steuerung |
| 188 | Antrieb für eine Führung des Sondenhalters |
| 190 | Antrieb für eine Bewegung des Fahrgestells |
| 191 | erster Motor |
| 192 | zweiter Motor |
| 194 | Führungsschiene |
| 196 | profilierte Führungsschiene |
| 198 | T-Führungsschiene |
| 200 | Führungsschlitten |
| 202 | Grundplatte |
| 204 | Loslager |
| 206 | kein Loslager |
| 208 | Loslager in z-Richtung |
| 210 | Loslager in y-Richtung |
| 212 | Loslager in yz-Richtung |
| 214 | Zahnstangenantrieb |
| 215 | Schrittmotor |
| 216 | Zahnstange |
| 218 | Stirnzahnrad |
| 220 | Zahnstangenhalterung |
| 222 | Befestigungswinkel |
| 224 | Schraubverbindung |
| 226 | Bohrungen |
| 228 | Nut |
| 230 | Klemmplatte |
| 232 | Rohrleitung |

| 234 | Rahmen |
|---|---|
| 236 | Hinterseite des Fahrgestells |
| 238 | Radwelle |
| 240 | erstes Kegelrad |
| 242 | zweites Kegelrad |
| 244 | Welle des ersten Kegelrads |
| 246 | Ausgleichskupplung |
| 248 | Halter |
| 250 | Stellring |
| 252 | Rad |
| 254 | Antriebsrad |
| 256 | O-Ring |
| 258 | Hinterrad |
| 260 | Kugellager |
| 262 | Rillenkugellager |
| 264 | Sicherungsring |
| 266 | Welle |
| 268 | selbstsichernde Mutter |
| 270 | Transportbandbreite |
| 272 | mäandrierender Verfahrweg |
| 510 | Pfeil |
| 512 | Feld |
| 514 | Feld |
| 516 | Feld |
| 518 | Pfeil |
| 520 | Feld |
| 522 | Feld |
| 524 | Feld |
| 526 | Pfeil |
| 528 | Pfeil |
| 530 | Benutzerschnittstelle |
| 532 | graphische Benutzerschnittstelle |
| 534 | Hauptmenü |
| 536 | Grundbildschirm |
| 538 | erster Button |
| 540 | zweiter Button |
| 542 | dritter Button |
| 544 | zweite Bedienoberfläche |
| 546 | Stelle |
| 548 | Button |
| 550 | erster Button |
| 552 | zweiter Button |
| 554 | dritter Button |
| 556 | vierter Button |
| 558 | Homebutton |
| 560 | Eingabefeld |
| 562 | erstes Eingabefeld |
| 564 | zweites Eingabefeld |
| 566 | Stelle |
| 568 | Stelle |
| 570 | Pfeil |
| 572 | Pfeil |
| 574 | "Zurück"-Button |
| 576 | Informationsfenster |
| 578 | Service-Button |
| 580 | Feld |
| 582 | Button |
| 584 | Button |

| | |
|---|---|
| 586 | Schließ-Button |
| 588 | erste Bedienoberfläche |
| 590 | Stelle |
| 592 | Start-Button |
| 594 | Ausgabefeld |
| 596 | Stelle |
| 598 | weitere Stelle |
| 600 | Button |
| 602 | Darstellung Positionen möglicher Lecks |
| 604 | clear-Button |
| 610 | Vorwärts-Rückwärts-Zähler |
| 612 | erster Vorwärts-Rückwärts-Zähler |
| 614 | zweiter Vorwärts-Rückwärts-Zähler |
| 616 | dritter Vorwärts-Rückwärts-Zähler |
| 618 | erstes Netzwerk |
| 620 | zweites Netzwerk |
| 622 | drittes Netzwerk |
| 624 | zweiter Zähleingang zum Vorwärtszählen |
| 626 | zweiter Zähleingang zum Rückwärtszählen |
| 628 | zweiter Rücksetzeingang |
| 630 | zweiter Ladeeingang |
| 632 | zweiter Ladewert |
| 634 | zweiter Ausgang zum Zählerstand |
| 636 | zweiter Ausgang zur Abfrage des Zählerstatus |
| 638 | erster Zähleingang zum Vorwärtszählen |
| 640 | erster Zähleingang zum Rückwärtszählen |
| 642 | erster Rücksetzeingang |
| 644 | erster Ladeeingang |
| 646 | erster Ladewert |
| 648 | erster Ausgang zum Zählerstand |
| 650 | erster Ausgang zur Abfrage des Zählerstatus |
| 652 | ODER-Glied |
| 654 | Eingangssignal |
| 656 | Eingangssignal |
| 658 | dritter Zähleingang zum Vorwärtszählen |
| 660 | dritter Zähleingang zum Rückwärtszählen |
| 662 | dritter Rücksetzeingang |
| 664 | ODER-Glied |
| 666 | UND-Glied |
| 668 | Eingangssignal |
| 670 | Eingangssignal |
| 672 | Ausgangssignal |
| 674 | Eingangssignal |
| 676 | dritter Ladeeingang |
| 678 | dritter Ladewert |
| 680 | dritter Ausgang zum Zählerstand |
| 682 | dritter Ausgang zur Abfrage des Zählerstatus |
| 684 | ODER-Glied |
| 686 | Eingangssignal |
| 688 | Eingangssignal |
| 690 | Ausgangssignal |
| 692 | Funktionsbaustein |
| 694 | Zeitvorgabe |
| 696 | Speicherglied |
| 698 | Ausgangssignal |
| 700 | Eingangssignal |
| 702 | Funktionsbaustein |
| 704 | Zeitvorgabe |

| | |
|---|---|
| 706 | Ausgangssignal |
| 708 | Speicherglied |
| 710 | Eingangssignal |
| 712 | UND-Glied |
| 714 | Eingangssignal |
| 716 | Ausgangssignal |
| 718 | Funktionsbaustein |
| 720 | Zeitvorgabe |
| 722 | Ausgangssignal |
| 724 | Funktionsbaustein |
| 726 | Zeitvorgabe |
| 728 | Ausgangssignal |
| 730 | Speicherglied |
| 732 | Speicherglied |

**Patentansprüche**

1. Partikelzählvorrichtung (120) zum Partikelzählen in einem Sterilisationstunnel (110) einer Medikamentenabfüllanlage (112), wobei der Sterilisationstunnel (110) mindestens ein Transportband (114) umfasst, wobei die Partikelzählvorrichtung (120) umfasst:

   • mindestens eine mit einem Partikelzähler (174) verbindbare Sonde (122) zum Aufnehmen von Partikeln in dem Sterilisationstunnel (110);
   • mindestens einen Scanner (176) mit mindestens einem Sondenhalter (178) zum Befestigen der Sonde (122), wobei der Scanner (176) umfasst:

      ◦ mindestens einen Querläufer (180) mit mindestens einer Linearführung (182), wobei die Linearführung (182) eingerichtet ist, den Sondenhalter (178) quer zu einer Transportrichtung (116) des Transportbands (114) des Sterilisationstunnels (110) zu führen;
      ◦ mindestens ein Fahrgestell (184), wobei der Querläufer (180) auf dem Fahrgestell (184) befestigt ist, wobei das Fahrgestell (184) eingerichtet ist, die Linearführung (182) in der Transportrichtung (116) des Transportbands (114) zu bewegen; und
      ◦ mindestens eine Steuerung (186), wobei die Steuerung (186) eingerichtet ist, um eine Bewegung des Scanners (176) zu steuern;

   weiterhin umfassend mindestens eine stationäre Benutzerschnittstelle, wobei die Benutzerschnittstelle mit dem Scanner (176) verbunden ist und wobei mittels der Benutzerschnittstelle eine Bewegung des Scanners (176) steuerbar ist.

2. Partikelzählvorrichtung (120) nach dem vorhergehenden Anspruch, wobei die Steuerung (186) eine speicherprogrammierbare Steuerung umfasst.

3. Partikelzählvorrichtung (120) nach einem der vorhergehenden Ansprüche, wobei die Partikelzählvorrichtung (120) weiterhin mindestens einen mit der Sonde (122) verbindbaren Partikelzähler (174) umfasst.

4. Partikelzählvorrichtung (120) nach einem der vorhergehenden Ansprüche, wobei mittels der Benutzerschnittstelle mindestens ein Fahrweg und/oder Messpositionen für die Partikelzählung vorgebbar sind.

5. Partikelzählvorrichtung (120) nach einem der vorhergehenden Ansprüche, wobei die Benutzerschnittstelle weiterhin eingerichtet ist, um die Sonde (122) gezielt zu mindestens einer vorgebbaren Sondenposition zu bewegen und dort eine Partikelzählung durchzuführen.

6. Partikelzählvorrichtung (120) nach einem der vorhergehenden Ansprüche, wobei die Benutzerschnittstelle eingerichtet ist, um Partikelzahlen in Abhängigkeit von einer Sondenposition zu erfassen und insbesondere darzustellen, wobei insbesondere die Benutzerschnittstelle zusätzlich mit einem Partikelzähler (174) verbunden ist.

7. Sterilisationstunnel (110) einer Medikamentenabfüllanlage (112), wobei der Sterilisationstunnel (110) umfasst:

• mindestens ein Transportband (114), wobei das Transportband (114) eingerichtet ist, mindestens ein Gefäß entlang einer Transportrichtung (116) des Transportbands (114) zu führen;
• mindestens einen Schwebstofffilter (118); und
• mindestens eine zwischen dem Schwebstofffilter (118) und dem Transportband (114) angeordnete Partikelzählvorrichtung (120) nach einem der vorhergehenden Ansprüche, wobei die Sonde (122) der Partikelzählvorrichtung (120) mit einer Sondenöffnung (124) dem Schwebstofffilter (118) zuweist.

8. Verfahren zum Partikelzählen in einem Sterilisationstunnel (110) einer Medikamentenabfüllanlage (112) mittels der Partikelzählvorrichtung (120) nach einem der vorhergehenden, eine Partikelzählvorrichtung (120) betreffenden Ansprüche, wobei das Verfahren die folgenden Schritte umfasst:

a) eine Bewegung des Querläufers (180) in die Transportrichtung (116) des Transportbands (114) mittels des Fahrgestells (184);
b) ein Führen des Sondenhalters (178) quer zu der Transportrichtung (116) mittels der Linearführung (182);

wobei die Schritte a) und b) nacheinander und wiederholt ausgeführt werden.

9. Verfahren nach dem vorhergehenden Anspruch, wobei vor Durchführung des Schritts a) der Sondenhalter (178) an einem ersten Ende der Linearführung (182) angeordnet ist, wobei in Schritt b) der Sondenhalter (178) von dem ersten Ende zu einem zweiten Ende der Linearführung (182) geführt wird.

10. Verfahren nach einem der beiden vorhergehenden Ansprüche, wobei weiterhin während der Durchführung des Verfahrens mittels mindestens eines Temperatursensors eine Temperatur in dem Sterilisationstunnel (110) erfasst wird, wobei bei einem Überschreiten der Temperatur über einen definierten Grenzwert der Schritt a) abgebrochen wird.

11. Verfahren nach einem der drei vorhergehenden Ansprüche, wobei die Schritte a) und/oder b) in einem Handbetrieb ausgeführt werden.

12. Verfahren nach einem der vier vorhergehenden Ansprüche, wobei die Schritte a) und/oder b) automatisch ausgeführt werden.

13. Verfahren nach einem der fünf vorhergehenden Ansprüche, wobei die Linearführung (182) mindestens einen Antrieb (188) aufweist, wobei der Antrieb (188) mindestens einen ersten Motor (191) aufweist, wobei die Partikelzählvorrichtung (120) weiterhin mindestens einen zweiten Motor (192) aufweist, wobei der zweite Motor (192) eingerichtet ist, das Fahrgestell (184) anzutreiben, wobei die Steuerung (186) für den ersten Motor (191) und für den zweiten Motor (192) jeweils mindestens einen digitalen Ausgang für eine Bewegungsrichtung und mindestens einen digitalen Ausgang für eine Motortreiber-Freigabe aufweist, wobei bei einer Durchführung von Schritt a) und/oder von Schritt b) folgende Schrittkette ausgeführt wird:

i. Aktivierung des digitalen Ausgangs für die Motortreiber-Freigabe; und
ii. Generierung eines Impulssignals.

14. Verfahren nach dem vorhergehenden Anspruch, wobei die Schritte i. und ii. zeitlich versetzt voneinander ausgeführt werden.

15. Verfahren nach einem der beiden vorhergehenden Ansprüche, wobei nach Durchführung des Schritts i. eine Aktivierung des digitalen Ausgangs für die Bewegungsrichtung erfolgt.

16. Verfahren nach einem der vier vorhergehenden Ansprüche, wobei mittels mindestens eines ersten Vorwärts-Rückwärts-Zählers erste Impulse des ersten Motors (191) gezählt werden, wobei mittels mindestens eines zweiten Vorwärts-Rückwärts-Zählers zweite Impulse des zweiten Motors (192) gezählt werden, wobei mittels der ersten Impulse des ersten Motors (191) eine Position der Sonde (122) auf der Linearführung (182) und mittels der zweiten Impulse des zweiten Motors (192) eine Position des Fahrgestells (184) auf dem Transportband (114) ermittelt wird.

17. Verfahren nach dem vorhergehenden Anspruch, wobei die Linearführung (182) einen ersten Endanschlag und einen zweiten Endanschlag aufweist, wobei nach Durchführung des Schritts b) der erste Vorwärts-Rückwärts-Zähler des ersten Motors (191) auf Null zurückgesetzt wird, wenn sich die Sonde (122) am ersten Endanschlag befindet.

18. Verfahren nach einem der zehn vorhergehenden Ansprüche, wobei bei einer Überschreitung einer Partikelzahl über einen definierten Grenzwert die Position der Sonde (122) auf dem Transportband (114) erfasst wird.

19. Verfahren nach einem der elf vorhergehenden Ansprüche, wobei die Sonde (122) eine Sondenöffnung (124) mit einem äußeren Durchmesser umfasst, wobei der Schritt a) derart durchgeführt wird, dass eine Schrittweite der Partikelzählvorrichtung (120) in die Transportrichtung (116) des Transportbands (114) mittels des Fahrgestells (184) kleiner ist als der äußere Durchmesser der Sondenöffnung (124).

**Claims**

1. A particle counting device (120) for counting particles in a sterilization tunnel (110) of a medicament filling system (112), wherein the sterilization tunnel (110) comprises at least one transport belt (114), wherein the particle counting device (120) comprises:

   • at least one probe (122), which can be connected to a particle counter (174), for receiving particles in the sterilization tunnel (110);
   • at least one scanner (176) having at least one probe holder (178) for fastening the probe (122), wherein the scanner (176) comprises:

   ○ at least one cross slide (180) with at least one linear guide (182), wherein the linear guide (182) is configured to guide the probe holder (178) transversely of a transport direction (116) of the transport belt (114) of the sterilization tunnel (110);
   ○ at least one carriage (184), wherein the cross slide (180) is fastened on the carriage (184), wherein the carriage (184) is configured to move the linear guide (182) in the transport direction (116) of the transport belt (114); and
   ○ at least one controller (186), wherein the controller (186) is configured to control a movement of the scanner (176);

   furthermore comprising at least one stationary user interface, wherein the user interface is connected to the scanner (176) and wherein a movement of the scanner (176) can be controlled by means of the user interface.

2. The particle counting device (120) according to the preceding claim, wherein the controller (186) comprises a programmable logic controller.

3. The particle counting device (120) according to either one of the preceding claims, wherein the particle counting device (120) furthermore comprises at least one particle counter (174) which can be connected to the probe (122).

4. The particle counting device (120) according to any one of the preceding claims, wherein at least one travel path and/or measurement positions for the particle counting are predefinable by means of the user interface.

5. The particle counting device (120) according to any one of the preceding claims, wherein the user interface is furthermore configured to move the probe (122) in a targeted manner to at least one predefinable probe position and to carry out a particle count there.

6. The particle counting device (120) according to any one of the preceding claims, wherein the user interface is configured to acquire and in particular to display particle counts depending on a probe position, wherein in particular the user interface is additionally connected to a particle counter (174).

7. A sterilization tunnel (110) of a medicament filling system (112), wherein the sterilization tunnel (110) comprises:

   • at least one transport belt (114), wherein the transport belt (114) is configured to guide at least one vessel along a transport direction (116) of the transport belt (114);
   • at least one suspended matter filter (118); and
   • at least one particle counting device (120) according to any one of the preceding claims arranged between the suspended matter filter (118) and the transport belt (114), wherein the probe (122) of the particle counting device (120) faces the suspended matter filter (118) with a probe opening (124).

8.  A method for counting particles in a sterilization tunnel (110) of a medicament filling system (112) by means of the particle counting device (120) according to any one of the preceding claims relating to a particle counting device (120), wherein the method comprises the following steps:

    a) moving the cross slide (180) in the transport direction (116) of the transport belt (114) by means of the carriage (184);
    b) guiding the probe holder (178) transversely of the transport direction (116) by means of the linear guide (182);

    wherein steps a) and b) are performed in succession and repeatedly.

9.  The method according to the preceding claim, wherein, before step a) is carried out, the probe holder (178) is arranged at a first end of the linear guide (182), wherein in step b) the probe holder (178) is guided from the first end to a second end of the linear guide (182).

10. The method according to either one of the two preceding claims, wherein furthermore, while the method is being carried out, a temperature in the sterilization tunnel (110) is acquired by means of at least one temperature sensor, wherein step a) is aborted when the temperature exceeds a defined limit value.

11. The method according to any one of the three preceding claims, wherein steps a) and/or b) are performed manually.

12. The method according to any one of the four preceding claims, wherein steps a) and/or b) are performed automatically.

13. The method according to any one of the five preceding claims, wherein the linear guide (182) has at least one drive (188), wherein the drive (188) has at least one first motor (191), wherein the particle counting device (120) furthermore has at least one second motor (192), wherein the second motor (192) is configured to drive the carriage (184), wherein the controller (186) for the first motor (191) and for the second motor (192) in each case has at least one digital output for a movement direction and at least one digital output for enabling the motor driver, wherein the following sequence of steps is performed when step a) and/or step b) is carried out:

    i. activation of the digital output for enabling the motor driver; and
    ii. generation of a pulse signal.

14. The method according to the preceding claim, wherein the steps i. and ii. are performed temporally offset from one another.

15. The method according to either one of the two preceding claims, wherein, once step i. has been carried out, the digital output for the movement direction is activated.

16. The method according to any one of the four preceding claims, wherein first pulses of the first motor (191) are counted by means of at least one first up-down counter, wherein second pulses of the second motor (192) are counted by means of at least one second up-down counter, wherein, by means of the first pulses of the first motor (191), a position of the probe (122) on the linear guide (182) and, by means of the second pulses of the second motor (192), a position of the carriage (184) on the transport belt (114) is determined.

17. The method according to the preceding claim, wherein the linear guide (182) has a first end stop and a second end stop, wherein, once step b) has been carried out, the first up-down counter of the first motor (191) is reset to zero when the probe (122) is located at the first end stop.

18. The method according to any one of the ten preceding claims, wherein, when a particle count exceeds a defined limit value, the position of the probe (122) on the transport belt (114) is acquired.

19. The method according to any one of the eleven preceding claims, wherein the probe (122) comprises a probe opening (124) with an outer diameter, wherein step a) is carried out such that an increment of the particle counting device (120) in the transport direction (116) of the transport belt (114) by means of the carriage (184) is smaller than the outer diameter of the probe opening (124).

**Revendications**

1. Dispositif de comptage de particules (120) permettant de compter les particules dans un tunnel de stérilisation (110) d'une installation de remplissage de médicaments (112), dans lequel le tunnel de stérilisation (110) comprend au moins une bande transporteuse (114), dans lequel le dispositif de comptage de particules (120) comprend :

   • au moins une sonde (122) pouvant être connectée à un compteur de particules (174) pour recevoir les particules dans le tunnel de stérilisation (110) ;
   • au moins un dispositif de balayage (176) comportant au moins un porte-sonde (178) pour fixer la sonde (122), dans lequel le dispositif de balayage (176) comprend :

      ◦ au moins un chariot transversal (180) comportant au moins un guidage linéaire (182), dans lequel le guidage linéaire (182) est conçu pour guider le porte-sonde (178) transversalement à une direction de transport (116) de la bande transporteuse (114) du tunnel de stérilisation (110) ;
      ◦ au moins un châssis (184), dans lequel le chariot transversal (180) est monté sur le châssis (184), dans lequel le châssis (184) est conçu pour déplacer le guidage linéaire (182) dans la direction de transport (116) de la bande transporteuse (114) ; et
      ◦ au moins un dispositif de commande (186), dans lequel le dispositif de commande (186) est conçu pour commander le déplacement du dispositif de balayage (176) ;

   en outre comprenant au moins une interface utilisateur stationnaire, dans lequel l'interface utilisateur est connectée au dispositif de balayage (176), et dans lequel, au moyen de l'interface utilisateur, le déplacement du dispositif de balayage (176) peut être commandé.

2. Dispositif de comptage de particules (120) selon la revendication précédente, dans lequel le dispositif de commande (186) comprend un automate programmable industrtiel.

3. Dispositif de comptage de particules (120) selon l'une quelconque des revendications précédentes, dans lequel le dispositif de comptage de particules (120) comprend en outre au moins un compteur de particules (174) pouvant être connecté à la sonde (122).

4. Dispositif de comptage de particules (120) selon l'une quelconque des revendications précédentes, dans lequel, au moyen de l'interface utilisateur, au moins un trajet de déplacement et/ou des positions de mesure pour le comptage de particules peuvent être prédéterminés.

5. Dispositif de comptage de particules (120) selon l'une quelconque des revendications précédentes, dans lequel l'interface utilisateur est en outre conçue pour déplacer de manière ciblée la sonde (122) vers au moins une position de sonde prédéterminée et pour effectuer un comptage de particules dans ladite position.

6. Dispositif de comptage de particules (120) selon l'une quelconque des revendications précédentes, dans lequel l'interface utilisateur est conçue pour détecter et, en particulier, représenter le nombre de particules en fonction de la position de la sonde, dans lequel l'interface utilisateur est en plus connectée à un compteur de particules (174).

7. Tunnel de stérilisation (110) d'une installation de remplissage de médicaments (112), dans lequel le tunnel de stérilisation (110) comprend :

   • au moins une bande transporteuse (114), dans lequel la bande transporteuse (114) est conçue pour guider au moins un récipient le long d'une direction de transport (116) de la bande transporteuse (114) ;
   • au moins un filtre à matières en suspension (118) ; et
   • au moins un dispositif de comptage de particules (120) selon l'une quelconque des revendications précédentes, disposé entre le filtre à matières en suspension (118) et la bande transporteuse (114), dans lequel, la sonde (122) du dispositif de comptage de particules (120) attribue une ouverture de sonde (124) au filtre à matières en suspension (118).

8. Procédé de comptage de particules dans un tunnel de stérilisation (110) d'une installation de remplissage de médicaments (112) au moyen d'un dispositif de comptage de particules (120) selon l'une quelconque des revendications précédentes portant sur un dispositif de comptage de particules (120), dans lequel le procédé comprend les étapes suivantes :

a) un déplacement du chariot transversal (180) dans la direction de transport (116) de la bande transporteuse (114) au moyen du châssis (184) ;

b) un guidage du porte-sonde (178) transversalement à la direction de transport (116) au moyen du guidage linéaire (182) ;

dans lequel les étapes a) et b) sont mises en œuvre successivement et de manière répétée.

9. Procédé selon la revendication précédente, dans lequel, avant la mise en œuvre de l'étape a), le porte-sonde (178) est disposé à une première extrémité du guidage linéaire (182), dans lequel dans l'étape b), le porte-sonde (178) est guidé de la première extrémité à une seconde extrémité du guidage linéaire (182).

10. Procédé selon l'une quelconque des deux revendications précédentes, dans lequel en outre, lors de la mise en œuvre du procédé, une température dans le tunnel de stérilisation (110) est détectée au moyen d'au moins un capteur de température ; dans lequel lorsque la température est supérieure à une valeur limite déterminée, l'étape a) est interrompue.

11. Procédé selon l'une quelconque des trois revendications précédentes, dans lequel les étapes a) et/ou b) sont mises en œuvre manuellement.

12. Procédé selon l'une quelconque des quatre revendications précédentes, dans lequel les étapes a) et/ou b) sont mises en œuvre automatiquement.

13. Procédé selon l'une quelconque des cinq revendications précédentes, dans lequel le guidage linéaire (182) comporte au moins un entraînement (188), dans lequel l'entraînement (188) comporte au moins un premier moteur (191), dans lequel le dispositif de comptage de particules (120) comporte en outre au moins un second moteur (192), dans lequel le second moteur (192) est conçu pour entraîner le châssis (184), dans lequel le dispositif de commande (186) comporte respectivement pour le premier moteur (191) et pour le second moteur (192) au moins une sortie numérique pour une direction de déplacement et au moins une sortie numérique pour une activation du pilote moteur, dans lequel lors d'une mise en œuvre de l'étape a) et/ou de l'étape b), la séquence d'étapes suivante est mise en œuvre :

i. l'activation de la sortie numérique pour l'activation du pilote moteur ; et
ii. la génération d'un signal d'impulsion.

14. Procédé selon la revendication précédente, dans lequel les étapes i. et ii. sont mises en œuvre de manière décalée dans le temps.

15. Procédé selon l'une quelconque des deux revendications précédentes, dans lequel, après la mise en œuvre de l'étape i., une activation de la sortie numérique pour la direction de déplacement est effectuée.

16. Procédé selon l'une quelconque des quatre revendications précédentes, dans lequel des premières impulsions du premier moteur (191) sont comptées au moyen d'au moins un premier compteur-décompteur, dans lequel des secondes impulsions du second moteur (192) sont comptées au moyen d'au moins un second compteur-décompteur, dans lequel une position d'une sonde (122) sur le guidage linéaire (182) est déterminée au moyen des premières impulsions du premier moteur (191) et une position du châssis (184) sur la bande transporteuse (114) est déterminée au moyen des secondes impulsions du second moteur (192).

17. Procédé selon la revendication précédente, dans lequel le guidage linéaire (182) comporte une première butée et une seconde butée, dans lequel, après la mise en œuvre de l'étape b), le premier compteur-décompteur du premier moteur (191) est remis à zéro lorsque la sonde (122) atteint la première butée.

18. Procédé selon l'une quelconque des dix revendications précédentes, dans lequel, lorsque le nombre de particules est supérieur à une limite déterminée, la position de la sonde (122) sur la bande transporteuse (114) est détectée.

19. Procédé selon l'une quelconque des onze revendications précédentes, dans lequel la sonde (122) comprend une ouverture de sonde (124) présentant un diamètre extérieur, dans lequel l'étape a) est mise en œuvre de telle sorte que le pas du dispositif de comptage de particules (120) dans la direction de transport (116) de la bande transporteuse (114) au moyen du châssis (184) est inférieur au diamètre extérieur de l'ouverture de sonde (124).

Fig. 1

Fig. 2

Fig. 3

180

200

182

194, 196, 198

202

## Fig. 4

206

204, 208

204, 210

204, 212

194, 196, 198    200

194, 196, 198    200

194, 196, 198    200

194, 196, 198    200

y

x

z

## Fig. 5

Fig. 6

Fig. 7

180

122

178

222

188, 191, 214, 215

230

232

218

174

228

216

200

182

202

194, 196, 198

Fig. 8

184

234

234

234

234

236

Fig. 9

Fig. 10

Fig. 11

252, 258

260, 262

268

266

264

256

Fig. 12

256

252, 258

184

266

268

Fig. 13

252, 258

184

252, 258

252, 254

238

252, 254

Fig. 14

120

116

184

122

270

270

180

114

Fig. 15

Fig. 16

Fig. 17 A

Fig. 17 B

Fig. 17 C

Fig. 18A

Fig. 18 B

Fig. 18 C

Fig. 18 D

530, 532, 588

590

+0    +0
+0    +0
+0    +0    602
+0    +0
+0    +0

+0    604

592

X

596    594

+0

598    +0

600

Y

Fig. 18 E

+30    +573
+89    +485
+0    +0
+0    +0
+0    +0

604    +2

Fig. 18 F

618

CTUD
Int                    610, 614

624 — CU
626 — CD
628 — R      QD — 636
630 — LD     CV — 634
632 — PV     QU — 636

620

652

>=1                  610, 612

654 —                CTUD
656 — ☼              Int

         638 — CU
         640 — CD
         642 — R

                     QD — 650
         644 — LD    CV — 648
         646 — PV    QU — 650

622

666              664

&                >=1                  610, 616

668 —o           674 —               CTUD
670 —o ☼              ☼              Int

         672 —           658 — CU
                         660 — CD
                         662 — R

                              QD — 682
                 676 — LD     CV — 680
                 678 — PV     QU — 682

Fig. 19

Fig. 20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 210198911 U